# EUROPEAN PATENT SPECIFICATION

(11) **EP 1 318 976 B1**
(45) Date of publication and mention of the grant of the patent: **24.11.2004**
(21) Application number: 01979230.8
(22) Date of filing: 06.09.2001
(51) Int. Cl.: C07C 235/34

(54) **SUBSTITUTED AMINOPROPOXYARYL DERIVATIVES USEFUL AS AGONISTS FOR LXR**
SUBSTITUIERTE AMINOPROPOXYARYLDERIVATE ALS LXR AGONISTEN
COMPOSANTS CHIMIQUES

(30) Priority: 18.09.2000 US 233144 P
(43) Date of publication of application: 18.06.2003
(73) Proprietor: GLAXO GROUP LIMITED, Greenford, Middlesex UB6 ONN (GB)
(72) Inventor: COLLINS, Jon Loren, GlaxoSmithKline, Research Triangle Park, NC 27709 (US); FIVUSH, Adam, M, Fishers, IN 46038 (US); MALONEY, Patrick Reed, GlaxoSmithKline, Research Triangle Park, NC 27709 (US); STEWART, Eugene L, GlaxoSmithKline, Research Triangle Park, NC 27709 (US); WILLSON, Timothy Mark, GlaxoSmithKline, Research Triangle Park, NC 27709 (US)
(74) Representative: Sewell, Richard Charles
(86) International application number: PCT/US2001/027622
(87) International publication number: WO 2002/024632

(56) References cited:
- EP-A- 0 558 062
- WO-A-00/54759
- WO-A-01/60818

## Description

### BACKGROUND OF THE INVENTION

The present invention relates to Liver X receptors (LXR). More particularly, the present invention relates to compounds useful as agonists for LXR , pharmaceutical formulations comprising such compounds, and therapeutic use of the same.

The orphan nuclear receptors, LXRα and LXRβ (collectively LXR) play a role in the maintenance of cholesterol balance. Peet *et al*., *Curr*. *Opin*. *Genet. Dev.* **8**:571-575 (1998). LXR is a transcription factor which regulates the expression of Cytochrome P450 7A (CYP7A). CYPP7A catalyses a key step in the conversion of cholesterol to bile acid, which process results in the removal of cholesterol from the liver.

In addition, LXR binds to the ATP Binding Cassette Transporter-1 (ABC1) (also known as ABCA 1) gene and increases expression of the gene to result in increased ABC1 protein. ABC1 is a membrane bound transport protein which is involved in the regulation of cholesterol efflux from extrahepatic cells onto nascent HDL particles. Mutations in the ABC1 gene are responsible for genetic diseases that result in the complete absence or low levels of HDL cholesterol and a concomitant highly increased risk of cardiovascular disease. See Brooks-Wilson *et al*., *Nat*. *Genet.* **22**:336-345 (1999); Bodzioch *et al*., *Nat*. *Genet.* **22**: 347-351 (1999); and Rust *et al*., *Nat.. Genet.* **22**:352-355 (1999). ABC1 knockout mice homozygous for the mutation in the ABC1 gene have virtually no plasma HDL, whereas the heterozygotes produce 50% of the HDL of wild type animals. See, Orso *et al*., *Nat*. *Genet.* **24**:192-196 (2000) and McNeish *et al*., *Proc. Natl*. *Acad*. *Sci*. *USA* **97**:4245-4250 (2000). ABC1 knockout mice also show increased cholesterol absorption. See, McNeish *et al*., *Proc. Natl*. *Acad*. *Sci*. *USA* **97**:4245-4250 (2000). Increased expression of ABC1 results in increased HDL cholesterol, decreased absorption of cholesterol, and increased removal of excess cholesterol from extrahepatic tissues, including macrophages.

EP 0 558 062 (published 1 September 1993) discloses phenoxyacetic acid derivatives as PGI2 receptor agonists.

WO 00/54759 (priority date 15 March 1999, published 21 September 2000) discloses arylcarbamoyl and arylsulfamoyl derivatives as LXR modulators.

WO01/60818 (priority date 14 February 2000, published 23 August 2001) discloses amido derivatives as LXR modulators.

Accordingly compounds which function as LXR agonists would be useful in methods of increasing ABC1 expression, increasing HDL cholesterol and treating LXR mediated diseases and conditions such as cardiovascular disease.

### SUMMARY OF THE INVENTION

According to a first aspect, the present invention provides compounds of formula (I): 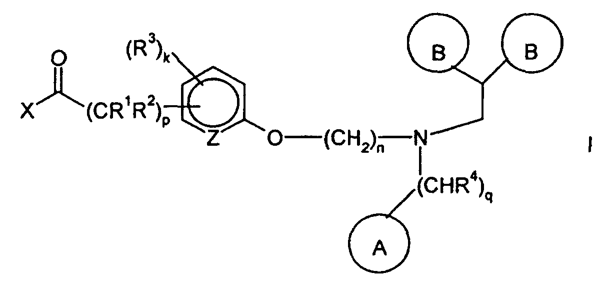 wherein:
X is OH or NH₂;
p is 0-6;
each R¹ and R² are the same or different and are each independently selected from the group consisting of H, C₁₋₈alkyl, C₁₋₈alkoxy and C₁₋₈thioalkyl;
Z is CH or N;
when Z is CH, k is 0-4;
when Z is N, k is 0-3;
each R³ is the same or different and is independently selected from the group consisting of halo and C₁₋₈alkoxy;
n is 3;
q is 0 or 1;
R⁴ is selected from the group consisting of H and C₁₋₈alkyl;
Ring A is selected from the group consisting of:
   i) C₃₋₈cycloalkyl,
   ii) aryl selected from the group consisting of: phenyl, 1-naphthyl or 2-naphthyl;
   iii) 4-8 membered heterocycle selected from the group consisting of: a monocyclic saturated or unsaturated non-aromatic carbocyclic ring or fused bicyclic non-aromatic carbocyclic ring each of which may contain 1, 2 or 3 heteroatoms selected from N, O and S; and
   iv) 5-6 membered heteroaryl selected from the group consisting of: an aromatic monocyclic heterocyclic ring or aromatic fused bicyclic ring each of which may contain 1, 2 or 3 heteroatoms selected from N, O and S;
wherein ring A may be independently substituted by one or more substituents selected from the group consisting of halo, -OH, C₁₋₈alkyl, C₂₋₈alkenyl, C₁₋ ₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano, wherein a is 0, 1 or 2; R⁶ is selected from the group consisting of H, C₁₋₈alkyl, C₁₋₈alkoxy and C₂₋₈alkenyl; each R⁷ and R⁸ is the same or different and is independently selected from the group consisting of H, C₁₋₈alkyl, C₂₋ ₈alkenyl and C₃₋₈alkynyl; R⁹ is selected from the group consisting of H, C₁₋ ₈alkyl and -NR⁷R⁸; and R¹⁰ is C₁₋₈alkyl;
each ring B is the same or different and is independently selected from the group consisting of cyclohexyl and phenyl; and
pharmaceutically acceptable salts and solvates thereof.

In another aspect, the present invention provides compounds which are LXR agonists.

In a third aspect, the present invention provides compounds which upregulate expression of ABC1.

In another aspect, the present invention provides a pharmaceutical composition comprising a compound of formula (I). The pharmaceutical composition may further comprise a pharmaceutically acceptable carrier or diluent.

In another aspect, the present invention provides a method for the prevention or treatment of an LXR mediated disease or condition. The method comprises administering a therapeutically effective amount of a compound of formula (I). The present invention also provides compounds of formula (I) for use in therapy and particularly for use in the prevention or treatment of an LXR mediated disease or condition. The present invention further provides the use of a compound of formula (I) for the preparation of a medicament for the prevention or treatment of an LXR mediated disease or condition.

In another aspect, the present invention provides a method for increasing reverse cholesterol transport. The method comprises administering a therapeutically effective amount of a compound of formula (I). The present invention also provides compounds of formula (I) for increasing reverse cholesterol transport. The present invention further provides the use of compounds of formula (I) for the preparation of a medicament for increasing reverse cholesterol transport

In another aspect, the present invention provides a method for inhibiting cholesterol absorption. The method comprises administering a therapeutically effective amount of a compound of formula (I). The present invention also provides compounds of formula (I) for inhibiting cholesterol absorption. The present invention further provides the use of compounds of formula (I) for the preparation of a medicament for inhibiting cholesterol absorption.

In another aspect, the present invention provides a method for increasing HDL-cholesterol. The method comprises administering a therapeutically effective amount of a compound of formula (I). The present invention also provides compounds of formula (I) for increasing HDL-cholesterol. The present invention further provides the use of compounds of formula (I) for the preparation of a medicament for increasing HDL-cholesterol.

In another aspect, the present invention provides a method for decreasing LDL-cholesterol. The method comprises administering a therapeutically effective amount of a compound of formula (I). The present invention also provides compounds of formula (I) for decreasing LDL-cholesterol. The present invention further provides the use of compounds of formula (I) for the preparation of medicaments for decreasing LDL-cholesterol.

In another aspect, the present invention provides a radiolabeled compound of formula (I). In one embodiment, the compound of formula (I) is tritiated. The present invention also provides a method for identifying compounds which interact with LXR. The method comprises the step of specifically binding a radiolabeled compound of formula (I) to the ligand binding domain of LXR. In another aspect, the present invention provides compounds identified using the assay methods described herein and methods for the prevention and treatment of an LXR-mediated disease or condition by administering a compound identified using the assay methods described herein. The assay methods are also useful for identifying compounds which are LXR agonists, compounds which are selective LXRβ agonists, compounds which upregulate ABC1, and compounds which are useful in methods for the treatment or prevention of LXR mediated diseases or conditions such as cardiovascular disease, including atherosclerosis.

In another aspect, the present invention provides a process for preparing compounds of formula (I). The process comprises reacting a solid phase-bound compound of formula (V): 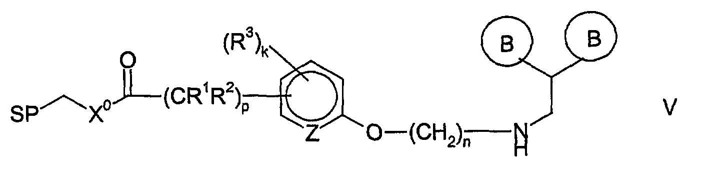 wherein SP is solid phase and X⁰ is -0- or -NH-, and all other variables are as defined above in connection with compounds of formula (I);
with a compound of formula (VIII): 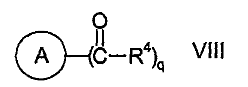 wherein all variables are as defined above in connection with compounds of formula (I).
The process may further comprise the additional step of cleaving the compound of formula (I) from the solid phase.

As another aspect, the present invention provides another process for preparing compounds of formula (I). The process comprises the steps of:
a) reacting a compound of formula (IV-A): 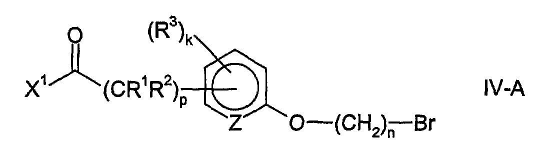 wherein X¹ is OR¹⁶ or NH₂, where R¹⁶ is a protecting group, and all other
   variables are as defined above in connection with compounds of formula (I); with a compound of formula (IX): 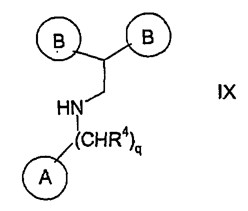 wherein all variables are as defined above in connection with compounds of formula (I)
   to prepare a compound of formula (I-A): 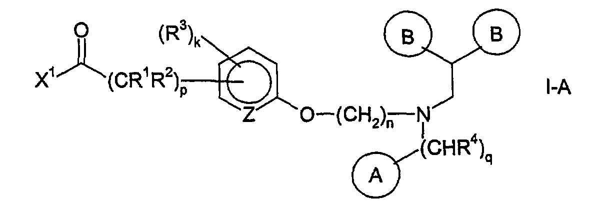 and
b) in the embodiment wherein X¹ is OR¹⁶, saponifying the compound of formula (I-A) to produce the compound of formula (I).

Either of the foregoing processes may comprise the additional step of converting a compound of formula (I) to a pharmaceutically acceptable salt or solvate thereof.

In another aspect, the present invention provides compounds of formula (I-A): 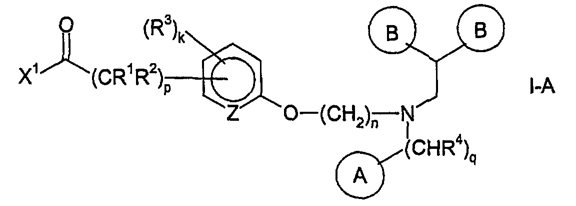 wherein
X¹ is OR¹⁶ or NH₂, where R¹⁶ is a protecting group;
p is 0-6;
each R¹ and R² are the same or different and are each independently selected from the group consisting of H, C₁₋₈alkyl, C₁₋₈alkoxy and C₁₋₈thioalkyl;
Z is CH or N;
when Z is CH, k is 0-4;
when Z is N, k is 0-3;
each R³ is the same or different and is independently selected from the group consisting of halo, -ON, C₁₋₈alkyl, C₂₋₈alkenyl, C₁₋₈alkoxy, C₂₋₈alkenyloxy, -S(O)ₐR⁶, -NR⁷R⁸, -COR⁶, COOR⁶, R¹⁰COOR⁶, OR¹⁰COOR⁶, CONR⁷R⁸, -OC(O)R⁹, -R¹⁰NR⁷R⁸, -OR¹⁰NR⁷R⁸, 5-6 membered heterocycle, nitro, and cyano; a is 0, 1 or 2;
R⁶ is selected from the group consisting of H, C₁₋₈alkyl, C₁₋₈alkoxy and C₂₋₈alkenyl;
each R⁷ and R⁸ are the same or different and are each independently selected from the group consisting of H, C₁₋₈alkyl, C₂₋₈alkenyl, C₃₋₈alkynyl;
R⁹ is selected from the group consisting of H, C₁₋₈alkyl and -NR⁷R⁸;
R¹⁰ is C₁₋₈alkyl;
n is 2-8;
q is 0 or 1;
R⁴ is selected from the group consisting of H, C₁₋₈alkyl, C₁₋₈alkenyl, and alkenyloxy; Ring A is selected from the group consisting of C₃₋₈cycloalkyl, aryl, 4-8 membered heterocycle, and 5-6 membered heteroaryl;
each ring B is the same or different and is independently selected from the group consisting of C₃₋₈cycloalkyl and aryl; and
pharmaceutically acceptable salts and solvates thereof.

Further aspects of the present invention are described in the description of preferred embodiments, examples, and claims which follow.

### DETAILED DESCRIPTION OF THE PREFERRED EMBODIMENTS

As used herein, the term "alkyl" refers to aliphatic straight or branched saturated hydrocarbon chains containing the specified number of carbon atoms. Examples of "alkyl" groups as used herein include but are not limited to methyl, ethyl, n-propyl, isopropyl, n-butyl, isobutyl, t-butyl, pentyl, hexyl, octyl and the like. The term "alkyl" also refers to substituted alkyl wherein the substituents are selected from the group consisting of halo, -OR⁷ and -SR⁷, where R⁷ is H or C₁₋₈alkyl. This definition of "alkyl" is also applicable to terms such as "thioalkyl" which incorporate the "alkyl" term. Thus, a "thioalkyl" as used herein refers to the group S-Ra where Ra is "alkyl" as defined.

As used herein, the term "halo" refers to any halogen atom ie., fluorine, chlorine, bromine or iodine.

As used herein, the term "alkenyl" refers to an aliphatic straight or branched unsaturated hydrocarbon chain containing at least one and up to three carbon-carbon double bonds. Examples of "alkenyl" groups as used herein include but are not limited to ethenyl and propenyl. The term "alkenyl" also refers to substituted alkenyl wherein the substituents are selected from the group consisting of halo, -OR⁷ and -SR⁷, where R⁷ is H or C₁₋₈alkyl.

As used herein, the term "alkoxy" refers to a group O-Ra where Ra is "alkyl" as defined above.

The term "alkenyloxy" as used herein refers to a group O-Rb where Rb is "alkenyl" as defined above.

As used herein, the term "cycloalkyl" refers to a non-aromatic carbocyclic ring having the specified number of carbon atoms and up to three carbon-carbon double bonds. "Cycloalkyl" includes by way of example cyclopropyl, cyclobutyl, cyclopentyl, cyclohexyl, cycloheptyl, cyclooctyl, cyclobutenyl, cyclopentenyl, cyclohexenyl and bicyclic cycloalkyl groups such as bicycloheptane and bicyclo(2.2.1)heptene. The term "cycloalkyl" also refers to substituted cycloalkyl wherein the ring bears one or more substituents selected from the group consisting of halo, -OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano, wherein a is 0, 1 or 2; R⁶ is selected from the group consisting of H, C₁₋₈alkyl, C₁₋₈alkoxy and C₂₋₈alkenyl; each R⁷ and R⁸ is the same or different and is independently selected from the group consisting of H, C₁₋₈alkyl, C₂₋₈alkenyl and C₃₋₈alkynyl; R⁹ is selected from the group consisting of H, C₁₋₈alkyl and -NR⁷R⁸; and R¹⁰ is C₁₋₈alkyl. As will be appreciated by those skilled in the art, the number of possible substituents on the cycloalkyl ring will depend upon the size of ring. In one preferred embodiment, the cycloalkyl is a cyclohexyl which may be substituted as described above.

The term "aryl" as used herein refers to aromatic groups selected from the group consisting of phenyl, 1-naphthyl and 2-naphthyl. The term "aryl" also refers to substituted aryl wherein the phenyl or naphthyl ring bears one or more substituents selected from the group consisting of halo, -OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano, wherein a is 0, 1 or 2; R⁶ is selected from the group consisting of H, C₁₋₈alkyl, C₁₋₈alkoxy and C₂₋₈alkenyl; each R⁷ and R⁸ is the same or different and is independently selected from the group consisting of H, C₁₋₈alkyl, C₂₋₈alkenyl and C₃₋₈alkynyl; R⁹ is selected from the group consisting of H, C₁₋ ₈alkyl and -NR⁷R⁸; and R¹⁰ is C₁₋₈alkyl. As will be appreciated by those skilled in the art, the number of possible substituents on the aryl ring will depend upon the size of ring. For example, when the aryl ring is phenyl, the aryl ring may have up to 5 substituents selected from the foregoing list. One skilled in the art will readily be able to determine the maximum number of possible substituents for a 1-naphthyl or 2-naphthyl ring. A preferred aryl ring according to the invention is phenyl, which may be substituted as described above.

The term "heterocycle" refers to a monocyclic saturated or unsaturated non-aromatic carbocyclic rings and fused bicyclic non-aromatic carbocyclic rings, having the specified number of members in the ring and containing 1, 2 or 3 heteroatoms selected from N, 0 and S. Examples of particular heterocyclic groups include but are not limited to tetrahydrofuran, dihydropyran, tetrahydropyran, pyran, oxetane, thietane, 1,4-dioxane, 1,3-dioxane, 1,3-dioxalane, piperidine, piperazine, tetrahydropyrimidine, pyrrolidine, morpholine, thiomorpholine, thiazolidine, oxazolidine, tetrahydrothiopyran, tetrahydrothiophene, and the like. The term "heterocycle" also refers to substituted heterocycles wherein the heterocyclic ring bears one or more substituents selected from the group consisting of halo, -OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano, wherein a is 0, 1 or 2; R⁶ is selected from the group consisting of H, C₁₋₈alkyl, C₁₋₈alkoxy and C₂₋₈alkenyl; each R⁷ and R⁸ is the same or different and is independently selected from the group consisting of H, C₁₋₈alkyl, C₂₋₈alkenyl and C₃₋₈alkynyl; and R⁹ is selected from the group consisting of H, C₁₋₈alkyl and -NR⁷R⁸; and R¹⁰ is C₁₋₈alkyl. As will be appreciated by those skilled in the art, the number of possible substituents on the heterocyclic ring will depend upon the size of ring. There are no restrictions on the positions of the optional substituents in the heterocycles. Thus, the term encompasses rings having a substituent attached to the ring through a heteroatom. One skilled in the art will readily be able to determine the maximum number and locations of possible substituents for any given heterocycle. A preferred heterocycle according to the invention is piperidine, which may be substituted as described above.

The term "heteroaryl" refers to aromatic monocyclic heterocyclic rings and aromatic fused bicyclic rings having the specified number of members in the ring, having at least one aromatic ring and containing 1, 2 or 3 heteroatoms selected from N, 0 and S. Examples of particular heteroaryl groups include but are not limited to furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinoline, isoquinoline, benzofuran, benzothiophene, indole, and indazole. The term "heteroaryl" also refers to substituted heteroaryls wherein the heteroaryl ring bears one or more substituents selected from the group consisting of halo, -OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano, wherein a is 0, 1 or 2; R⁶ is selected from the group consisting of H, C₁₋₈alkyl, C₁₋₈alkoxy and C₂₋₈alkenyl; each R⁷ and R⁸ is the same or different and is independently selected from the group consisting of H, C₁₋₈alkyl, C₂₋₈-salkenyl and C₃₋₈alkynyl; and R⁹ is selected from the group consisting of H, C₁₋₈alkyl and -NR⁷R⁸; and R¹⁰ is C₁₋₈alkyl. As will be appreciated by those skilled in the art, the number of possible substituents on the heteroaryl ring will depend upon the size of ring. There are no restrictions on the positions of the optional substituents in heteroaryls. Thus, the term encompasses rings having a substituent attached to the ring through a heteroatom. One skilled in the art will readily be able to determine the maximum number and locations of possible substituents for any given heteroaryl. A preferred heteroaryl according to the invention is pyridine, which may be substituted as described above.

As used herein, the term "protecting group" refers to suitable protecting groups useful for the synthesis of compounds of formula (I) wherein X is OH. Suitable protecting groups are known to those skilled in the art and are described in *Protecting Groups in Organic Synthesis,* 3^{rd} Edition, Greene, T. W.; Wuts, P. G. M. Eds.; John Wiley Et Sons: NY, 1999. Examples of preferred protecting groups include but are not limited to methyl, ethyl, benzyl, substituted benzyl, and tert-butyl. In one embodiment the protecting group is methyl.

Suitable pharmaceutically acceptable salts according to the present invention will be readily determined by one skilled in the art and will include, for example, acid addition salts prepared from inorganic acids such as hydrochloric, hydrobromic, phosphoric, metaphosphoric, nitric, sulphonic, and sulfuric acids, and organic acids such as acetic, benzenesulphonic, benzoic, citric, ethanesulphonic, fumaric, gluconic, glycollic, isothionic, lactic, lactobionic, maleic, malic, methanesulphonic, succinic, *p*-toluenesulfonic, salicylic, tartaric, and trifluoroacetic, formic, malonic, naphthalene-2-sulfonic, sulfamic, decanoic, orotic, 1-hydroxy-2-naphthoic, cholic, and pamoic. In one embodiment, the compounds of formula (I) are in the form of the hydrochloride salt.

When used in medicine, the salts of a compound of formula (I) should be pharmaceutically acceptable, but pharmaceutically unacceptable salts may conveniently be used to prepare the corresponding free base or pharmaceutically acceptable salts thereof.

As used herein, the term "solvate" is a crystal form containing the compound of formula (I) or a pharmaceutically acceptable salt thereof and either a stoichiometric or a non-stoichiometric amount of a solvent. Solvents, by way of example, include water, methanol, ethanol, or acetic acid. Hereinafter, reference to a compound of formula (I) is to any physical form of that compound, unless a particular form, salt or solvate thereof is specified.

The present invention provides compounds of formula I: 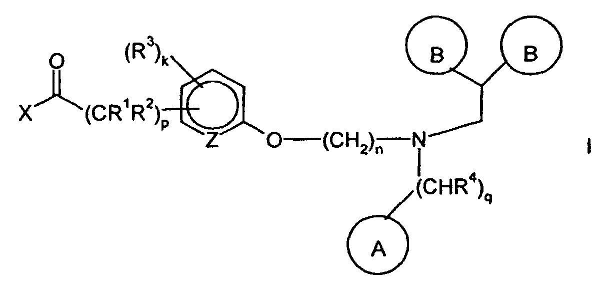 wherein:
X is OH or NH₂;
p is 0-6;
each R¹ and R² are the same or different and are each independently selected from the group consisting of H, C₁₋₈alkyl, C₁₋₈alkoxy and C₁₋₈thioalkyl;
Z is CH or N;
when Z is CH, k is 0-4;
when Z is N, k is 0-3;
each R³ is the same or different and is independently selected from the group consisting of halo and C₁₋₈alkoxy;
n is 3;
q is 0 or 1;
R⁴ is selected from the group consisting of H and C₁₋₈alkyl;
Ring A is selected from the group consisting of:
   i) C₃₋₈cycloalkyl,
   ii) aryl selected from the group consisting of: phenyl, 1-naphthyl or 2-naphthyl;
   iii) 4-8 membered heterocycle selected from the group consisting of: a monocyclic saturated or unsaturated non-aromatic carbocyclic ring or fused bicyclic non-aromatic carbocyclic ring each of which may contain 1, 2 or 3 heteroatoms selected from N, O and S; and
   iv) 5-6 membered heteroaryl selected from the group consisting of: an aromatic monocyclic heterocyclic ring or aromatic fused bicyclic ring each of which may contain 1, 2 or 3 heteroatoms selected from N, O and S;
wherein ring A may be independently substituted by one or more substituents selected from the group consisting of halo, -OH, C₁₋₈alkyl, C₂₋₈alkenyl, C₁₋ ₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano, wherein a is 0, 1 or 2; R⁶ is selected from the group consisting of H, C₁₋₈alkyl, C₁₋₈alkoxy and C₂₋₈alkenyl; each R⁷ and R⁸ is the same or different and is independently selected from the group consisting of H, C₁₋₈alkyl, C₂₋ ₈alkenyl and C₃₋₈alkynyl; R⁹ is selected from the group consisting of H, C₁₋ ₈alkyl and -NR⁷R⁸; and R¹⁰ is C₁₋₈alkyl;
each ring B is the same or different and is independently selected from the group consisting of cyclohexyl and phenyl; and
pharmaceutically acceptable salts and solvates thereof.

Certain compounds of formula (I) may exist in stereoisomeric forms (e.g. they may contain one or more asymmetric carbon atoms). The individual stereoisomers (enantiomers and diastereomers) and mixtures of these are included within the scope of the present invention. The present invention also covers the individual isomers of the compounds represented by formula (I) as mixtures with isomers thereof in which one or more chiral centers are inverted.

In one preferred embodiment, the compounds of formula (I) are defined where X is OH. In another preferred embodiment, X is NH₂.

In one embodiment, the compounds of formula (I) are defined wherein p is 0-3. In one preferred embodiment, p is 0 or 1. In one particular embodiment, p is 1.

Preferably, in the embodiments, where p is 1 or more, each R¹ and R² are the same or different and are each independently selected from the group consisting of H, C₁₋ ₈alkyl, and C₁₋₈alkoxy. In one preferred embodiment, each R¹ and R² are the same or different and are each independently selected from the group consisting of H and C₁₋ ₈alkyl. In another preferred embodiment, each R¹ and R² are the same or different and are each independently selected from the group consisting of H and C₁₋₃alkyl. In one particular embodiment, both R¹ and R² are H.

The group: 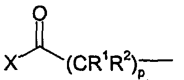 is preferably meta to the phenyl ether (when Z is CH) or pyridyl ether (when Z is N).

The group: 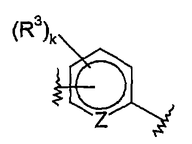 indicates a 6-membered aromatic ring which may contain up to 1 nitrogen atom (i.e., when Z is N) (i.e., the ring is phenyl or pyridine) and which may be substituted by one or more substituents R³. In one preferred embodiment, the compounds of formula (I) are defined where Z is CH. When Z is CH, k is 0-4, meaning that there can be up to 4 substituents R³ on the 6-membered aromatic ring. When Z is N, k is 0-3, meaning that there can be up to 3 substituents R³ on the 6-membered aromatic ring. In this embodiment, R³ is not attached to the N atom of the ring. Preferably, k is 0 or 1, more preferably 0.

In those embodiments wherein k is 1 or more, each R³ is preferably the same or different and is independently selected from the group consisting of halo and C₁₋ ₈alkoxy. More preferably, each R³ is the same or different and is independently selected from the group consisting of F, Cl and methoxy.

Preferably, q is 1.

According to one embodiment, when q is 1, R⁴ is H or C₁₋₈alkyl. Preferably, when q is 1, R⁴ is H.

Ring A is selected from the group consisting of C₃₋₈cycloalkyl, aryl, 4-8 membered heterocycle and 5-6 membered heteroaryl. By virtue of the definitions given above, for the terms "cycloalkyl," "aryl," "heterocycle," and "heteroaryl" this definition of Ring A also encompasses the foregoing rings optionally substituted with the substituents specified in the definitions above. In one embodiment, Ring A is selected from the group consisting of C₅₋₆cycloalkyl, aryl, 5-6 membered heterocycle and 5-6 membered heteroaryl (each optionally substituted). In one preferred embodiment, Ring A is a aryl optionally substituted from 1 to 5 times, more preferably, from 1 to 4 times. In one particular preferred embodiment, Ring A is phenyl optionally substituted from 1 to 5 times (more preferably from 1 to 4 times) with a substituent selected from the group consisting of halo, -OH, C₁₋₈alkyl, C₂₋₈alkenyl, C₁₋₈alkoxy, C₂₋ ₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano. In another preferred embodiment, Ring A is a 5-6 membered heterocycle optionally substituted from 1 to 8 times for a 5-membered heterocycle, and from 1 to 10 times for a 6-membered heterocycle with a substituent selected from the group consisting of halo, - OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano. In one particular preferred embodiment, Ring A is a 6-membered heterocycle optionally substituted from 1 to 10 times (preferably from 1 to 4 times).

Specific examples of Ring A according to the present invention include phenyl or piperidine optionally substituted from 1 to 5 times with a substituent selected from the group consisting of halo, C₁₋₈alkyl, C₁₋₈alkoxy, -COOR⁶ and S(O)ₐR⁶. More preferably, Ring A is phenyl or piperidine optionally substituted from 1 to 5 times with a substituent selected from the group consisting of F, Cl, -CF₃, -OCH₃, and -OCF₃. One specific preferred embodiment of compounds of formula (I) are defined wherein Ring A is phenyl optionally substituted from 1 to 4 times with a substituent selected from the group consisting of F, Cl, -CF₃, -OCH₃, and -OCF₃. Another specific embodiment of compounds of formula (I) is defined wherein Ring A is piperidine optionally substituted by -COOR⁶; and more preferably wherein the substituent -COOR⁶ is attached to the nitrogen of the piperidine ring and R⁶ is alkyl, e.g., methyl or ethyl.

Each Ring B is the same or different and is independently selected from the group consisting of C₃₋₈cycloalkyl and aryl. By virtue of the definitions given above, for the terms "cycloalkyl" and "aryl" this definition of Ring B also encompasses the foregoing rings optionally substituted with the substituents specified in the definitions above. In one embodiment, both Rings B are phenyl optionally substituted from 1 to 5 times (more preferably from 1 to 3 times) with a substituent selected from the group consisting of halo, -OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano. In another embodiment, Rings B are cyclohexyl optionally substituted from 1 to 10 times (more preferably from 1 to 4 times) with a substituent selected from the group consisting of halo, -OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano. In yet another embodiment, one Ring B is phenyl optionally substituted from 1 to 5 times (more preferably from 1 to 3 times) with a substituent selected from the group consisting of halo, -OH, C₁₋₈alkyl, C₂₋ ₈alkenyl, C₁₋₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano and the other Ring B is cyclohexyl optionally substituted from 1 to 10 times (more preferably from 1 to 4 times) with a substituent selected from the group consisting of halo, -OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano. In one particular embodiment, both Rings B are unsubstituted phenyl or unsubstituted cyclohexyl; more preferably unsubstituted phenyl.

The present invention contemplates and includes all combinations of the preferred groups defined above.

Preferred compounds of formula (I) include compounds selected the group consisting of:
2-(3-{3-[[2-chloro-3-(trifluoromethyl)benzyl](2,2-diphenylethyl)amino]propoxy} phenyl) acetamide,
2-(3-{3-[[2-chloro-3-(trifluoromethyl)benzyl](2,2-diphenylethyl)amino]propoxy}phenyl)acetic acid,
(3-{2-[(2,2-diphenylethyl)-(4-methoxybenzyl)amino]propoxy}phenyl)acetamide,
(3-{2-[(2,2-diphenylethyl)-(4-methoxybenzyl)amino]propoxy}phenyl)acetic acid,
2-(3-{3-[(2,2-diphenylethyl)(2-fluoro-4-methoxybenzyl)amino]propoxy}phenyl) acetamide,
2-(3-{3-[(2,4-dimethoxybenzyl)(2,2-diphenylethyl)amino]propoxy}phenyl) acetamide,
2-[3-(3-{(2,2-diphenylethyl)[4-fluoro-2-(trifluoromethyl)benzyl]amino}propoxy) phenyl] acetamide,
2-(3-{3-[(2,3-dichlorobenzyl)(2,2-diphenylethyl)amino]propoxy}phenyl) acetamide,
2-[3-(3-{(2,2-diphenylethyl)[3-(trifluoromethoxy)benzyl]amino}propoxy)phenyl] acetamide,
2-(3-{3-[(2,2-diphenylethyl)(3-fluoro-4-methoxybenzyl)amino]propoxy}phenyl) acetamide,
2-(3-{3-[(2,5-dimethoxybenzyl)(2,2-diphenylethyl)amino]propoxy}phenyl) acetamide,
2-[3-(3-{(2,2-diphenylethyl)[3-(trifluoromethyl)benzyl]amino}propoxy)phenyl] acetamide,
2-[3-(3-{(2,2-diphenylethyl)[2-fluoro-3-(trifluoromethyl)benzyl]amino}propoxy) phenyl] acetamide;
Ethyl 4-[{3-[3-(aminocarbonyl)phenoxy]propyl}(2,2-diphenylethyl)amino]-1-piperidinecarboxylate;
3-{3-[(1-Benzoyl-4-piperidinyl)-(2,2-diphenylethyl)amino]propoxy}benzamide;
3-{3-[(1-Acetyl-4-piperidinyl)(2,2-diphenylethyl)amino]propoxy}benzamide;
Benzyl 4-[{3-[3-(aminocarbonyl)phenoxy]propyl}(2,2-diphenylethyl)amino]-1-piperidinecarboxylate;
3-(3-{(2,2-Diphenylethyl)[1-(2-phenylethyl)-4-piperidinyl]amino}propoxy)benzamide;
Ethyl 4-[{3-[3-(aminocarbonyl)phenoxy]propyl}(2-cyclohexyl-2-phenylethyl)amino]-1-piperidinecarboxylate;
3-{3-[(1-Benzoyl-4-piperidinyl)(2-cyclohexyl-2-phenylethyl)amino]propoxy}benzamide;
3-{3-[(1-Acetyl-4-piperidinyl)(2-cyclohexyl-2-phenylethyl)amino]propoxy}benzamide;
*tert*-Butyl 4-[{3-[3-(aminocarbonyl)phenoxy]propyl}(2-cyclohexyl-2-phenylethyl)amino]-1-piperidinecarboxylate;
Benzyl 4-[{3-[3-(aminocarbonyl)phenoxy]propyl}(2-cyclohexyl-2-phenylethyl)amino]-1-piperidinecarboxylate;
3-{3-[(1-Benzyl-4-piperidinyl)(2-cyclohexyl-2-phenylethyl)amino]propoxy}benzamide;
Ethyl 4-[{3-[3-(2-amino-2-oxoethyl)phenoxy]propyl}(2,2-diphenylethyl)amino]-1-piperidinecarboxylate;
2-(3-{3-[(1-Benzoyl-4-piperidinyl)(2,2-diphenylethyl)amino]propoxy}phenyl)-acetamide;
2-(3-{3-[(1-Acetyl-4-piperidinyl)(2,2-diphenylethyl)amino]propoxy}phenyl)-acetamide;
*tert*-Butyl 4-[{3-[3-(2-amino-2-oxoethyl)phenoxy]propyl}(2,2-diphenylethyl)amino]-1-piperidinecarboxylate;
Benzyl 4-[{3-[3-(2-amino-2-oxoethyl)phenoxy]propyl}(2,2-diphenylethyl)amino]-1-piperidinecarboxylate;
2-[3-(3-{(2,2-Diphenylethyl)[1-(2-phenylethyl)-4-piperidinyl]amino}propoxy)phenyl]-acetamide;
2-(3-{3-[(1-Benzoyl-4-piperidinyl)(2-cyclohexyl-2-phenylethyl)amino]propoxy}phenyl)acetamide;
2-(3-{3-[(1-Acetyl-4-piperidinyl)(2-cyclohexyl-2-phenylethyl)amino]propoxy}phenyl)acetamide;
Benzyl-4-[{3-[3-(2-amino-2-oxoethyl)phenoxy]propyl}(2-cyclohexyl-2-phenylethyl)amino]-1-piperidinecarboxylate;
3-{3-[(3-Cyanobenzyl)(2,2-diphenylethyl)amino]propoxy}benzamide;
3-{3-[Cyclohexyl(2,2-diphenylethyl)amino]propoxy}benzamide;
4-[{3-[3-(Aminocarbonyl)phenoxy]propyl}(2,2-diphenylethyl)amino]-1-piperidinecarboxamide;
3-{3-[(1,3-Benzodioxol-4-ylmethyl)(2,2-diphenylethyl)amino]propoxy}benzamide;
3-{3-[(3,4-Dimethoxybenzyl)(2,2-diphenylethyl)amino]propoxy}benzamide;
3-{3-[(4-Cyanobenzyl)(2-cyclohexyl-2-phenylethyl)amino]propoxy}benzamide;
3-{3-[(4-Cyanobenzyl)(2-cyclohexyl-2-phenylethyl)amino]propoxy}benzamide;
2-(3-{3-[Cyclohexyl(2,2-diphenylethyl)amino]propoxy}phenyl)acetamide;
2-(3-{3-[(3,4-Dimethoxybenzyl)(2,2-diphenylethyl)amino]propoxy}phenyl)acetamide;
3-{3-[(2-Cyclohexyl-2-phenylethyl)(3,4-dimethoxybenzyl)amino]propoxy}benzamide;
3-{3-[(2,6-Dichlorobenzyl)(2,2-diphenylethyl)amino]propoxy}benzamide;
3-{[{3-[3-(Aminocarbonyl)phenoxy]propyl}(2,2-diphenylethyl)amino]methyl}benzoic acid;
4-{[{3-[3-(Aminocarbonyl)phenoxy]propyl}(2,2-diphenylethyl)amino]methyl}benzoic acid;
3-(3-{(2,2-Diphenylethyl)[(5-methoxy-1*H*-indol-3-yl)methyl]amino}propoxy)-benzamide;
3-{3-[(2,2-Diphenylethyl)(4-methoxybenzyl)amino]propoxy}benzamide;
3-{3-[[(1-Acetyl-1*H*-indol-3-yl)methyl](2,2-diphenylethyl)amino]propoxy}benzamide;
Methyl 4-{[{3-[3-(aminocarbonyl)phenoxy]propyl}(2,2-diphenylethyl)amino]methyl}benzoate;
3-{3-[(2,3-Dihydro-1,4-benzodioxin-6-ylmethyl)(2,2-diphenylethyl)amino]propoxy}benzamide;
3-{3-[(2,2-Diphenylethyl)(4-pyridinylmethyl)amino]propoxy}benzamide;
2-(3-{3-[(2-Cyclohexyl-2-phenylethyl)(3,4-difluorobenzyl)amino]propoxy}phenyl) acetamide;
2-(3-{3-(2,2-Diphenylethyl)[[(6-chloro-1,3-benzodioxol-5-yl)methyl]amino]propoxy}phenyl)acetamide;
2-(3-{3-[(2,2-Diphenylethyl)(cyclohexylmethyl)amino]propoxy}phenyl) acetamide;
2-(3-{3-[(2,2-Diphenylethyl)(bicyclo[2.2.1]hept-5-en-2-ylmethyl)amino]propoxy}phenyl) acetamide;
2-(3-{3-[(2,2-diphenylethyl)(2,4-dimethoxy-5-pyrimidinyl) methyl)amino]propoxy} phenyl) acetamide;
2-(3-{3-[(2,2-Diphenylethyl(5-isopropyl-3-methyl-4-isoxazolyl)methyl)amino]-propoxy}phenyl) acetamide;
2-(3-{3-[(2,2-Diphenylethyl)( 3,4-dihydro-2*H*-pyran-2-ylmethyl)amino]propoxy}phenyl) acetamide;
2-(3-{3-[(2,2-Diphenylethyl)( 4-chloro-1*H*-pyrazol-3-yl)methyl)amino]propoxy}phenyl) acetamide;
2-(3-{3-[(2,2-Diphenylethyl)()[(7-methoxy-1,3-benzodioxol-5-yl)methyl)amino]-propoxy}phenyl) acetamide;
2-(3-{3-[(2,2-Diphenylethyl-(2,6,6-trimethyl-1-cyclohexen-1-yl)ethyl)amino]-propoxy}phenyl) acetamide;
2-(3-{3-[(2,2-Diphenylethyl)( 3-cyclohexen-1-ylmethyl)amino]propoxy}phenyl) acetamide;
2-[3-(3-{(2,2-Diphenylethyl)[(2*E*)-3-phenyl-2-propenyl]amino}propoxy) phenyl]acetamide;
Ethyl 2-{[{3-[3-(2-amino-2-oxoethyl)phenoxy]propyl}(2,2-diphenylethyl)amino]-methyl}cyclopropanecarboxylate;
2-(3-{3-[(2,2-diphenylethyl)( 1-cyclohexen-1-ylmethyl)amino]propoxy} phenyl) acetamide;
2-(3-{3-[(2,2-Diphenylethyl)( 1*H*-benzimidazol-2-ylmethyl)amino]propoxy}phenyl) acetamide;
2-(3-{3-[(2,2-Diphenylethyl)[(1,3-dimethyl-2-oxo-2,3-dihydro-1*H*-benzimidazol-5-yl)methyl]amino]propoxy}phenyl) acetamide; and
2-(3-{3-[(2,2-Diphenylethyl)(2-pyrrolidinylmethyl)amino]propoxy}phenyl) acetamide;
and pharmaceutically acceptable salts and solvates thereof.

More preferred compounds of formula (I) include:
2-(3-{3-[[2-chloro-3-(trifluoromethyl)benzyl](2,2-diphenylethyl)amino]propoxy} phenyl) acetamide;
2-(3-{3-[[2-chloro-3-(trifluoromethyl)benzyl](2,2-diphenylethyl)amino]propoxy}phenyl)acetic acid;
(3-{2-[(2,2-diphenylethyl)-(4-methoxybenzyl)amino]-propoxy}phenyl)acetamide;
(3-{2-[(2,2-diphenylethyl)-(4-methoxybenzyl)amino]-propoxy}phenyl)acetic acid;
2-(3-{3-[(2,2-diphenylethyl)(2-fluoro-4-methoxybenzyl)amino]propoxy}phenyl) acetamide;
2-(3-{3-[(2,4-dimethoxybenzl)(2,2-diphenylethyl)amino]propoxy}phenyl) acetamide;
2-[3-(3-{(2,2-diphenylethyl)[4-fluoro-2-(trifluoromethyl)benzyl]amino}propoxy) phenyl] acetamide;
2-(3-{3-[(2,3-dichlorobenzyl)(2,2-diphenylethyl)amino]propoxy}phenyl) acetamide;
2-[3-(3-{(2,2-diphenylethyl)[3-(trifluoromethoxy)benzyl]amino}propoxy)phenyl] acetamide;
2-(3-{3-[(2,2-diphenylethyl)(3-fluoro-4-methoxybenzyl)amino]propoxy}phenyl) acetamide;
2-(3-{3-[(2,5-dimethoxybenzyl)(2,2-diphenylethyl)amino]propoxy}phenyl) acetamide;
2-[3-(3-{(2,2-diphenylethyl)[3-(trifluoromethyl)benzyl]amino}propoxy)phenyl] acetamide; and
2-[3-(3-{(2,2-diphenylethyl)[2-fluoro-3-(trifluoromethyl)benzyl]amino}propoxy) phenyl] acetamide;
and pharmaceutically acceptable salts and solvates thereof.

Particularly preferred compounds of fomula (I) include:
2-(3-{3-[[2-chloro-3-(trifluoromethyl)benzyl](2,2-diphenylethyl)amino]propoxy} phenyl) acetamide;
2-(3-{3-[[2-chloro-3-(trifluoromethyl)benzyl](2,2-diphenylethyl)amino]propoxy}phenyl)acetic acid;
(3-{2-[(2,2-diphenylethyl)-(4-methoxybenzyl)amino]-propoxy}phenyl)acetamide; and
(3-{2-[(2,2-diphenylethyl)-(4-methoxybenzyl)amino]-propoxy}phenyl)acetic acid;
and pharmaceutically acceptable salts and solvates thereof.

One particularly preferred compound is 2-(3-{3-[[2-chloro-3-(trifluoromethyl)benzyl] (2,2-diphenylethyl)amino]propoxy}-phenyl)acetic acid and pharmaceutically acceptable salts and solvates thereof.

Another particularly preferred compound is 2-(3-{3-[[2-chloro-3-(trifluoromethyl)benzyl](2,2-diphenylethyl)amino]propoxy} phenyl) acetamide and pharmaceutically acceptable salts and solvates thereof.

Hereinafter all references to "compounds of formula (I)" refer to compounds of formula (I) as described above together with their pharmaceutically acceptable salts and solvates.

Preferably, the compounds of formula (I) are LXR agonists. As used herein, the term "LXR agonist" refers to compounds which achieve at least 50% activation of LXR relative to 24(S),25-epoxycholesterol, the appropriate positive control in the HTRF assay described below in Example 1. More preferably, the compounds of this invention achieve 100% activation of LXR in the HTRF assay.

More preferably, the compounds of formula (I) are selective LXRβ agonists. As used herein, "selective LXRβ agonist" refers to a LXR agonist whose EC₅₀ for LXRβ is at least 2-3 fold, preferably, 5 fold and more preferably greater than 10 fold lower than its EC₅₀ for LXRα. EC₅₀ is the concentration at which a compound achieves 50% of its maximum activity.

In addition, preferably compounds of formula (I) will upregulate expression of ABC1. By upregulating expression of ABC1, is meant that the induction of ABC1 upon treatment of cells with compounds of formula (I) at a concentration less than or equal to 10 micromolar is greater than 2 fold greater than in the absence of compounds of formula (I) in the assay described below in Example 3. Thus the compounds of formula (I) are useful in methods for upregulating expression of ABC1.

The compounds of the formula (I) are useful for a variety of medicinal purposes. The compounds of formula (I) may be used in methods for the prevention or treatment of LXR mediated diseases and conditions. LXR mediated diseases or conditions include cardiovascular disease including atherosclerosis, arteriosclerosis, hypercholesteremia, and hyperlipidemia. In particular, the compounds of formula (I) are useful in the treatment and prevention of cardiovascular disease including artherosclerosis and hypercholesteremia.

The present invention also provides a method for increasing reverse cholesterol transport. Lipoprotein metabolism is a dynamic process comprised of production of triglyceride rich particles from the liver (as VLDL), modification of these lipoprotein particles within the plasma (VLDL to IDL to LDL) and clearance of the particles from the plasma, again by the liver. This process provides the transport of triglycerides and free cholesterol to cells of the body. Reverse cholesterol transport is the proposed mechanism by which peripheral cholesterol is returned to the liver from extra-hepatic tissue. The process is carried out by HDL cholesterol. The combination of lipoprotein production (VLDL, HDL) from the liver, modification of particles (all) within the plasma and subsequent clearance back to the liver, accounts for the steady state cholesterol concentration of the plasma. Without wishing to be bound by any particular theory, it is currently believed that the compounds of formula (I) increase reverse cholesterol transport by raising the plasma level of HDL cholesterol and/or by increasing cholesterol efflux from the arteries.

The compounds of formula (I) are also useful for inhibiting cholesterol absorption, increasing HDL-cholesterol, and decreasing LDL-cholesterol.

The methods of the present invention are useful for the treatment of animals including mammals generally and particularly humans.

The methods of the present invention comprise the step of administering a therapeutically effective amount of the compound of formula (I). As used herein, the term "therapeutically effective amount" refers to an amount of the compound of formula (I) which is sufficient to achieve the stated effect. Accordingly, a therapeutically effective amount of a compound of formula (I) used in the method for the prevention or treatment of LXR mediated diseases or conditions will be an amount sufficient to prevent or treat the LXR mediated disease or condition. Similarly, a therapeutically effective amount of a compound of formula (I) for use in the method of increasing reverse cholesterol transport will be an amount sufficient to increase reverse cholesterol transport.

The amount of a compound of formula (I) or pharmaceutically acceptable salt or solvate thereof, which is required to achieve the desired biological effect will depend on a number of factors such as the use for which it is intended, the means of administration, and the recipient, and will be ultimately at the discretion of the attendant physician or veterinarian. In general, a typical daily dose for the treatment of LXR mediated diseases and conditions in a human, for instance, may be expected to lie in the range of from about 0.01 mg/kg to about 100 mg/kg. This dose may be administered as a single unit dose or as several separate unit doses or as a continuous infusion. Similar dosages would be applicable for the treatment of other diseases, conditions and therapies including upregulating expression of ABC1, increasing reverse cholesterol transport, inhibiting cholesterol absorption, increasing HDL-cholesterol and decreasing LDL-cholesterol.

Thus in a further aspect the present invention provides pharmaceutical compositions comprising, as active ingredient, a compound of formula (I) or a pharmaceutically acceptable salt or solvate thereof. The composition may further comprise at least one pharmaceutical carrier or diluent. These pharmaceutical compositions may be used in the prophylaxis and treatment of the foregoing diseases or conditions and in cardiovascular therapies as mentioned above.

The carrier must be pharmaceutically acceptable and must be compatible with, i.e. not have a deleterious effect upon, the other ingredients in the composition. The carrier may be a solid or liquid and is preferably formulated as a unit dose formulation, for example, a tablet which may contain from 0.05 to 95% by weight of the active ingredient. If desired other physiologically active ingredients may also be incorporated in the pharmaceutical compositions of the invention.

Possible formulations include those suitable for oral, sublingual, buccal, parenteral (for example subcutaneous, intramuscular, or intravenous), rectal, topical including transdermal, intranasal and inhalation administration. Most suitable means of administration for a particular patient will depend on the nature and severity of the disease or condition being treated or the nature of the therapy being used and on the nature of the active compound, but where possible, oral administration is preferred for the prevention and treatment of LXR mediated diseases and conditions.

Formulations suitable for oral administration may be provided as discrete units, such as tablets, capsules, cachets, lozenges, each containing a predetermined amount of the active compound; as powders or granules; as solutions or suspensions in aqueous or non-aqueous liquids; or as oil-in-water or water-in-oil emulsions.

Formulations suitable for sublingual or buccal administration include lozenges comprising the active compound and, typically a flavored base, such as sugar and acacia or tragacanth and pastilles comprising the active compound in an inert base, such as gelatin and glycerine or sucrose acacia.

Formulations suitable for parenteral administration typically comprise sterile aqueous solutions containing a predetermined concentration of the active compound; the solution is preferably isotonic with the blood of the intended recipient. Additional formulations suitable for parenteral administration include formulations containing physiologically suitable co-solvents and/or complexing agents such as surfactants and cyclodextrins. Oil-in-water emulsions are also suitable formulations for parenteral formulations. Although such solutions are preferably administered intravenously, they may also be administered by subcutaneous or intramuscular injection.

Formulations suitable for rectal administration are preferably provided as unit-dose suppositories comprising the active ingredient in one or more solid carriers forming the suppository base, for example, cocoa butter.

Formulations suitable for topical or intranasal application include ointments, creams, lotions, pastes, gels, sprays, aerosols and oils. Suitable carriers for such formulations include petroleum jelly, lanolin, polyethyleneglycols, alcohols, and combinations thereof.

Formulations of the invention may be prepared by any suitable method, typically by uniformly and intimately admixing the active compound with liquids or finely divided solid carriers or both, in the required proportions and then, if necessary, shaping the resulting mixture into the desired shape.

For example a tablet may be prepared by compressing an intimate mixture comprising a powder or granules of the active ingredient and one or more optional ingredients, such as a binder, lubricant, inert diluent, or surface active dispersing agent, or by moulding an intimate mixture of powdered active ingredient and inert liquid diluent.

Suitable formulations for administration by inhalation include fine particle dusts or mists which may be generated by means of various types of metered dose pressurized aerosols, nebulisers, or insufflators.

For pulmonary administration via the mouth, the particle size of the powder or droplets is typically in the range 0.5 -10µm, preferably 1-5µm, to ensure delivery into the bronchial tree. For nasal administration, a particle size in the range 10-500µm is preferred to ensure retention in the nasal cavity.

Metered dose inhalers are pressurized aerosol dispensers, typically containing a suspension or solution formulation of the active ingredient in a liquefied propellant. During use, these devices discharge the formulation through a valve adapted to deliver a metered volume, typically from 10 to 150 µl, to produce a fine particle spray containing the active ingredient. Suitable propellants include certain chlorofluorocarbon compounds, for example, dichlorodifluoromethane, trichlorofluoromethane, dichlorotetrafluoroethane and mixtures thereof. The formulation may additionally contain one or more co-solvents, for example, ethanol surfactants, such as oleic acid or sorbitan trioleate, anti-oxidants and suitable flavouring agents.

Nebulisers are commercially available devices that transform solutions or suspensions of the active ingredient into a therapeutic aerosol mist either by means of acceleration of a compressed gas typically air or oxygen, through a narrow venturi orifice, or by means of ultrasonic agitation. Suitable formulations for use in nebulisers consist of the active ingredient in a liquid carrier and comprising up to 40% w/w of the formulation, preferably less than 20%w/w. The carrier is typically water or a dilute aqueous alcoholic solution, preferably made isotonic with body fluids by the addition of, for example, sodium chloride. Optional additives include preservatives if the formulation is not prepared sterile, for example, methyl hydroxy-benzoate, anti-oxidants, flavoring agents, volatile oils, buffering agents and surfactants.

Suitable formulations for administration by insufflation include finely comminuted powders which may be delivered by means of an insufflator or taken into the nasal cavity in the manner of a snuff. In the insufflator, the powder is contained in capsules or cartridges, typically made of gelatin or plastic, which are either pierced or opened *in situ* and the powder delivered by air drawn through the device upon inhalation or by means of a manually-operated pump. The powder employed in the insufflator consists either solely of the active ingredient or of a powder blend comprising the active ingredient, a suitable powder diluent, such as lactose, and an optional surfactant. The active ingredient typically comprises from 0.1 to 100 w/w of the formulation.

In addition to the ingredients specifically mentioned above, the formulations of the present invention may include other agents known to those skilled in the art of pharmacy, having regard for the type of formulation in issue. For example, formulations suitable for oral administration may include flavouring agents and formulations suitable for intranasal administration may include perfumes.

Compounds of the invention can be made according to any suitable method of organic chemistry. According to one method, compounds of formula (I) are prepared using a solid phase synthesis process as depicted in Scheme 1: 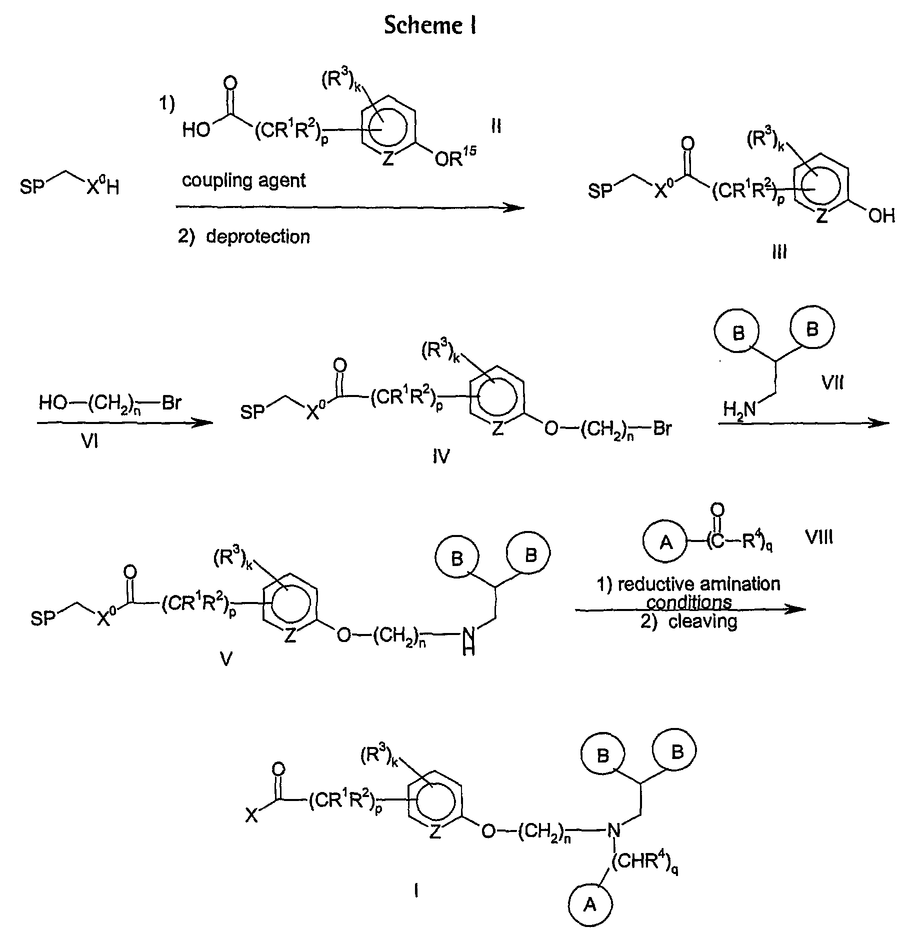 wherein X⁰ is -0- or -NH-, SP is solid phase, R¹⁵ is H or a protecting group, and all other variables are as defined above in connection with the description of compounds of formula (I).

In general, the reaction proceeds by a) reacting a solid phase-bound amine (where X in the compound of formula (I) is NH₂) or alcohol (where X in the compound of formula (I) is OH) with a compound of formula (II) and a coupling agent to produce a solid phase-bound compound of formula (III); b) in the embodiment wherein R¹⁵ is a protecting group, deprotecting the solid phase bound compound to prepare the compound of formula (III); c) alkylating the solid phase-bound compound of formula (III) with an alcohol of formula (VI) to produce a solid phase-bound compound of formula (IV); d) reacting the solid-phase-bound compound of formula (IV) with a compound of formula (VII) to produce the solid-phase bound compound of formula (V); and e) reacting the solid phase-bound compound of formula (V) with a compound of formula (VIII) under reductive amination conditions to produce the solid phase-bound compound of formula (I). The process may optionally further comprise the step of cleaving the solid phase-bound compound of formula (I) from the solid phase using conventional techniques such as treatment with mild acid.

Compounds of formula (II) are commercially available or can be prepared using conventional techniques such as those described in European Patent No. 303,742 published 22 February 1989.

Suitable solid phase materials and coupling agents for use in the foregoing method are commercially available and will be readily apparent to those skilled in the art. Examples of suitable solid phase materials include polymer resins such as Rink Resin SS from Advanced Chemtech, and Argogel-MB-OH from Argonaut Technologies.

The alcohols of formula (VI), the compounds of formula (VII) and the compounds of formula (VIII) are all commercially available or can be prepared using conventional techniques.

Compounds of formula (I) may also be prepared by an alternative method involving solution phase synthesis. The solution phase synthesis is depicted in Scheme (II) below. 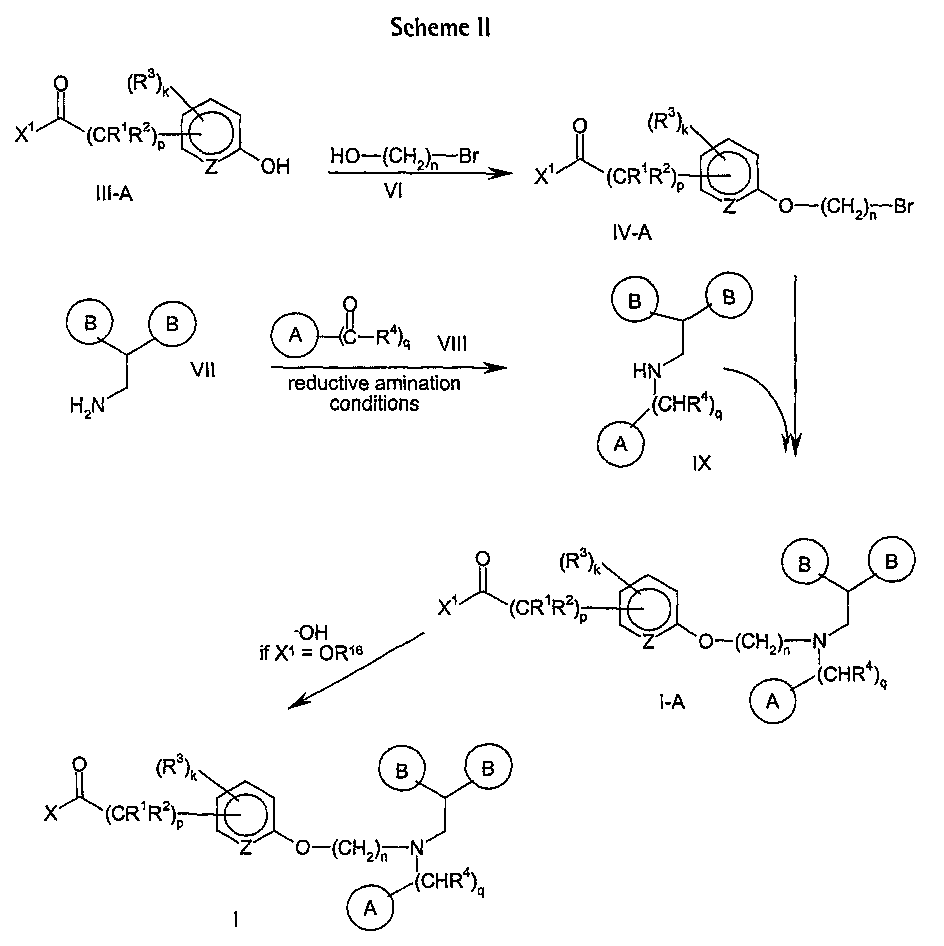 wherein X¹ is OR¹⁶ or NH₂, where R¹⁶ is a protecting group, and all other variables are as defined above in connection with the description of compounds of formula (I).

In general, the process comprises the steps of: a) alkylating a compound of formula (III-A) with an alcohol of formula (VI) to produce the compound of formula (IV-A), b) reacting a compound of formula (VII) with a compound of formula (VIII) under reductive amination conditions to produce a compound of formula (IX), c) reacting the compound of formula (IV-A) with the compound of formula (IX) to produce the compound of formula (I-A), and d) in the embodiment wherein X¹ is OR¹⁶, saponifying the ester to produce compounds of formula (I) wherein X is OH.

The compounds of formula (III-A) are prepared by conventional esterification procedures, such as those described in A. Kreimeyer, *et al*., *J. Med. Chem*. **1999**, *42*, 4394-4404.

The compounds of formula (VII) are commercially available or can be prepared using conventional techniques.

As another aspect, the present invention further provides compounds of formula (I-A) 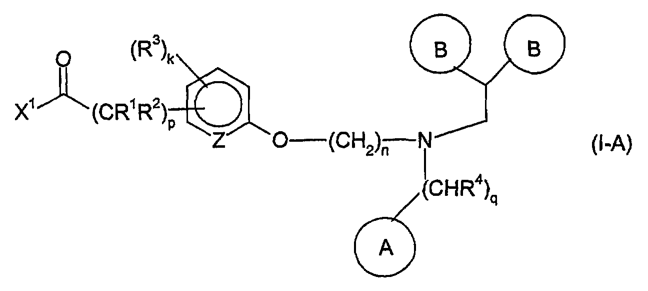 wherein
X¹ is OR¹⁶ or NH₂, where R¹⁶ is a protecting group;
p is 0-6;
each R¹ and R² are the same or different and are each independently selected from the group consisting of H, C₁₋₈alkyl, C₁₋₈alkoxy and C₁₋₈thioalkyl;
Z is CH or N;
when Z is CH, k is 0-4;
when Z is N, k is 0-3;
each R³ is the same or different and is independently selected from the group consisting of halo, -OH, C₁₋₈alkyl, C₂₋₈alkenyl, C₁₋₈alkoxy, C₂₋₈alkenyloxy, -S(O)ₐR⁶, -NR⁷R⁸, -COR⁶, COOR⁶, R¹⁰COOR⁶, OR¹⁰COOR⁶, CONR⁷R⁸, -OC(O)R⁹, -R¹⁰NR⁷R⁸, -OR¹⁰NR⁷R⁸, 5-6 membered heterocycle, nitro, and cyano;
a is 0, 1 or 2;
R⁶ is selected from the group consisting of H, C₁₋₈alkyl, C₁₋₈alkoxy and C₂₋₈alkenyl;
each R⁷ and R⁸ are the same or different and are each independently selected from the group consisting of H, C₁₋₈alkyl, C₂₋₈alkenyl, C₃₋₈alkynyl;
R⁹ is selected from the group consisting of H, C₁₋₈alkyl and -NR⁷R⁸;
R¹⁰ is C₁₋₈alkyl;
n is 2-8;
q is 0 or 1;
R⁴ is selected from the group consisting of H, C₁₋₈alkyl, C₁₋₈alkenyl, and alkenyloxy;
Ring A is selected from the group consisting of C₃₋₈cycloalkyl, aryl, 4-8 membered heterocycle, and 5-6 membered heteroaryl;
each ring B is the same or different and is independently selected from the group consisting of C₃₋₈cycloalkyl and aryl; and
pharmaceutically acceptable salts and solvates thereof;
which are useful as intermediates for the preparation of compounds of formula (I). Preferred compounds of formula (I-A) are defined according to the preferred definitions of variables as described for compounds of formula (I).

The present invention also provides radiolabeled compounds of formula (I). Radiolabeled compounds of formula (I) can be prepared using conventional techniques. For example, radiolabeled compounds of formula (I) can be prepared by reacting the intermediate of formula (I-A) with tritium gas in the presence of an appropriate catalyst to produce radiolabeled compounds of formula (I).

In the embodiment wherein X¹ is -NH₂, the radiolabled compounds of formula (I) are directly achieved using the foregoing method. In the embodiment wherein X¹ is OR¹⁶, the radiolabeled compound of formula (I-A) is saponified to produce the radiolabeled compounds of formula (I) wherein X is OH. In one preferred embodiment, the compounds of formula (I) are tritiated.

The radiolabeled compounds of formula (I) are useful in assays for the identification of compounds which interact with LXR, and particularly for the identification of compounds which bind to LXR. Accordingly, the present invention provides an assay method for identifying compounds which interact with LXR, which method comprises the step of specifically binding the radiolabeled compound of formula (I) to the ligand binding domain of LXR. The method may further comprise the step of adding a test compound and measuring any decrease in the specific binding of the radiolabeled compound of formula (I) to the ligand binding domain of LXR (i.e., either LXRα or LXRβ). Thus, suitable assay methods will include conventional competition binding assays. The radiolabeled compounds of formula (I) can be employed in LXRα and LXRβ binding assays according to the methods described in Moore, L B.; Parks, D. J.; Jones, S. A.; Bledsoe, R. K.; Consler, T. G.; Stimmel, J. B.; Goodwin, B.; Liddle, C.; Blanchard, S. G.; Willson, T. M.; Collins, J. L.; Kliewer, S. A. *J*. *Biol*. *Chem.* 2000, *275* (20), 15122-15127; Jones, S. A.; Moore, L B.; Shenk, J. L.; Wisely, B. G.; Hamilton, G. A.; McKee, D. D.; Tomkinson, N. C. 0.; LeCluyse, E. L; Lambert, M. H.; Willson, T. M.; *et al*. *Mol*. *Endrocrinol*. 2000, *14*, 27-39; and Janowski, Bethany A.; Grogan, Michael J.; Jones, Stacey A.; Wisely, G. Bruce; Kliewer, Steven A.; Corey, Elias J.; Mangelsdorf, David J.. Structural requirements of ligands for the oxysterol liver X receptors LXRa and LXRb Proc. Natl. Acad. Sci. U. S. A. (1999), 96(1), 266-271, the subject matter of which is incoporated herein in their entirety. The same assay procedures using the radiolabeled compounds of formula (I) may also be used to identify compounds which are LXR agonists, compounds which are selective LXRβ agonists and compounds which upregulate ABC1.

The present invention further comprises compounds identified using the foregoing assay method and methods of treating the various conditions and diseases described hereinabove, with a compound identified using the foregoing assay method. The following examples are intended for illustration only and are not intended to limit the scope of the invention in any way, the present invention being defined by the claims.

In the examples, the following terms have the designated meaning: "pRSETa" is a known expression vector available from Invitrogen; "IPTG" means isopropyl β-D-thiogalactopyranoside; "PO₄" means phosphate; "PBS" means phosphate buffered saline; "TBS" means tris-buffered saline; EDTA means ethylenediamine tetraacetic acid; "DTT" means dithiothreitol; "FAF-BSA" means fatty-acid free bovine serum albumin; "SRC-1" means steroid receptor coactivator 1; "CS" means charcoal stripped; "nM" means nanomolar; "µM" means micromolar; "mM" means millimolar; "pM" means picomolar; "mmol" means millimoles; "g" means grams; "ng" means nanograms; "mg/ml" means milligram per milliliter; "µL" means microliters; and "mL" means milliliter.

### Example 1: Assay for LXRβ Activity

A modified polyhistidine tag (MKKGHHHHHHG) (SEQ ID No. 1) was fused in frame to the human LXRβ ligand binding domain (amino acids 185-461 of Genbank accession number U07132) and subcloned into the expression vector pRSETa (Invitrogen) under the control of an IPTG inducible T7 promoter. The human LXRβ ligand binding domain was expressed in E. coli strain BL21(DE3). Ten-liter fermentation batches were grown in Rich PO₄ media with 0.1 mg/mL Ampicillin at 25°C for 12 hours, cooled to 9°C and held at that temperature for 36 hours to a density of OD600=14. At this cell density, 0.25 mM IPTG was added and induction proceeded for 24 hours at 9°C, to a final OD600 = 16. Cells were harvested by centrifugation (20 minutes, 3500g, 4°C), and concentrated cell slurries were stored in PBS at -80°C.

Typically 25-50 g of cell paste is resuspended in 250-500 mL TBS, pH 8.0 (25mM Tris, 150 mM NaCl). Cells are lysed by passing 3 times through an APV Rannie MINI-lab homogenizer and cell debris is removed by centrifugation (30 minutes, 20,000g, 4°C). The cleared supernatant is filtered through coarse pre-filters, and TBS, pH 8.0, containing 500 mM imidazole is added to obtain a final imidazole concentration of 50mM. This lysate is loaded onto a column (XK-26, 10 cm) packed with Sepharose [Ni++ charged] Chelation resin (available from Pharmacia) and pre-equilibrated with TBS pH 8.0/ 50mM imidazole. After washing to baseline absorbance with equilibration buffer, the column is washed with approximately one column volume of TBS pH -8.0 containing 95mM imidazole. LXRβLBD(185-461) is eluted with a gradient from 50 to 500 mM imidazole. Column peak fractions are pooled immediately and diluted 5 fold with 25 mM Tris pH 8.0, containing 5% 1,2-propanediol, .5mM EDTA and 5mM DTT. The diluted protein sample is then loaded onto a column (XK-16, 10cm) packed with Poros HQ resin (anion exchange). After washing to baseline absorbance with the dilution buffer the protein is eluted with a gradient from 50 -500 mM NaCl. Peak fractions are pooled and concentrated using Centri-prep 10K (Amicon) filter devices and subjected to size exclusion, using a column (XK-26, 90 cm) packed with Superdex-75 resin (Pharmacia) pre-equilibrated with TBS, pH 8.0, containing 5 % 1,2-propanediol, 0.5mM EDTA and 5mM DTT.

LXRβ protein was diluted to approximately 10µM in PBS and five-fold molar excess of NHS-LC-Biotin (Pierce) was added in a minimal volume of PBS. This solution was incubated with gentle mixing for 30 minutes at ambient room temperature. The biotinylation modification reaction was stopped by the addition of 2000x molar excess of Tris-HCl, pH 8. The modified LXRβ protein was dialyzed against 4 buffer changes, each of at least 50 volumes, PBS containing 5mM DTT, 2mM EDTA and 2% sucrose. The biotinylated LXRβ protein was subjected to mass spectrometric analysis to reveal the extent of modification by the biotinylation reagent. In general, approximately 95% of the protein had at least a single site of biotinylation; and the overall extent of biotinylation followed a normal distribution of multiple sites, ranging from one to nine.

The biotinylated protein was incubated for 20-25 minutes at a concentration of 25nM in assay buffer (50mM KCI, 50mM Tris-pH8, 0.1mg/ml FAF-BSA, 10mM DTT) with equimolar amounts of streptavidin-AlloPhycoCyanin (APC, Molecular Probes). At the same time, the biotinylated peptide comprising amino acids 675-699 of SRC-1 (CPSSHSSLTERHKILHRLLOEGSPS-CONH2) (SEQ ID No. 2) at a concentration of 25nM was incubated in assay buffer with a ½ molar amount of streptavidin-labelled Europium (Wallac) for 20-25 minutes. After the initial incubations are completed, a 10 molar excess (250nM) of cold biotin was added to each of the solutions to block the unattached streptavidin reagents. After 20 min at room temp, the solutions were mixed yielding a concentration of 12.5nM for the dye-labelled LXRβ protein and SRC-1 peptide.

80µL of the protein/peptide mixture was added to each well of an assay plate containing 20µL of test compound. The final volume in each well was 0.1 mL, and the concentration in the well for the dye-labelled protein and peptide was 10nM. The final test compound concentrations were between 56pM and 10µM. The plates were incubated at room temp in the dark for 4-12 hours and then counted on a Wallac Victor fluorescent plate reader.

In this assay 1µM 24(S),25-epoxycholesterol gave a reading of 20000 fluorescence units over a background reading of 10000 fluorescence units.

### Example 2: Assay for LXRα Activity

The assay for LXRα was run according to the procedures of **Example 1**, above using his-tagged LXRα ligand binding domain (amino acids 183-447 of Genbank accession number U22662 , with the 14^{th} amino acid corrected to A from R).

In this assay 1µM 24(S),25-epoxycholesterol gave a reading of 20000 fluorescence units over a background reading of 10000 fluorescence units.

### Example 3: Assay for ABC1 expression in macrophages

RAW 264.7 cells, obtained from ATCC, were grown in Dulbecco's Modified Eagle Media (DMEM, GIBCO) supplemented with 10% fetal bovine serum (FBS, Irvine Scientific), 2 mM glutamine (Irvine Scientific), 100 U penicillin/ml and 100 mg streptomycin/ml (Irvine Scientific). Cells were passaged routinely at 3-4 day intervals at a plating density of 1:3.

To assess the effects of test compounds on ABC1 expression, the cells were passaged into CS media (DMEM/F12 media without phenol red supplemented with 10% charcoal/dextran-treated FBS, 2 mM glutamine, 100 U penicillin/ml and 100 mg streptomycin/ml and 100 mM mevalonic acid lactone). Two days later, the media was replaced with fresh CS media containing 10 µM of the test compound. After 24 hours, the media was removed and replaced with fresh CS media containing fresh drug. After 24 more hours, the media was aspirated and the cells lysed in Trizol reagent (GIBCO). RNA was then extracted according to manufacturer's instructions. The RNA was quantitated following RNAse-free DNAse treatment by using the Ribogreen System (Molecular Probes), and then diluted to 10ng/microL.

ABC1 expression was determined by quantitative PCR. TaqMan reactions were performed using the standard conditions on the AB17700; 5.5mM MgCl2, 1X TaqMan Buffer A, 300microM each dNTP, 20U RNAse inhibitor, 12.5U MuLV RT;ase, 300nM of each primer, 200nM TaqMan probe, 1.25U AmpliTaq Gold, and 50ng RNA in a 50uL volume. The reaction conditions were 48°C for 30 minutes, 95°C for 10 minutes, and 40 cycles of 94°C for 15 seconds/60°C for 1 minute. The sequence of the primers and probe for mouse ABC1 (X75926) were: forward primer: AAGGGTTTCTTTGCTCAGATTGTC (SEQ ID No. 3); reverse primer: TGCCAAAGGGTGGCACA (SEQ ID No. 4); probe oligo: CCAGCTGTCTTTGTTTGCATTGCCC (SEQ ID No. 5). Results were analyzed on the ABI7700 using Sequence Detector v1.6 software provided with the machine. ABC1 expression was calculated as fold induction in test compound-treated cells relative to vehicle-treated cells.

### Example 4: 2-(3-{3-[[2-Chloro-3-(trifluoromethyl)benzyl](2,2-diphenylethyl)amino] propoxy}phenyl)acetamide

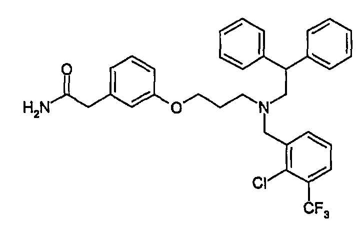

Rink Resin SS (1.0 g, 0.70 mmol, 0.70 mmol/g loading, Advanced ChemTech) was treated with 10 mL of 20% piperidine in dimethylformamide and rotated for 30 minutes at room temperature. The resin was filtered, treated with 10 mL of 20% piperidine in dimethylformamide, and rotated for 1 hour. The resin was filtered, washed with dimethylformamide (2 x 15 mL) and dichloromethane (2 x 15 mL), and dried under house vacuum to give deprotected resin. Separately, a slurry of 3-hydroxyphenylacetic acid (0.53 g, 3.5 mmol) and [O-(7-azabenzotriazol-1-yl)-1,1,3,3-tetramethyluronium hexafluorophosphate] (1.33 g, 3.5 mmol) in 10 mL of anhydrous 1-methyl-2-pyrrolidinone was treated with 2,6-lutidine (0.82 mL, 7.0 mmol) and stirred until the solid dissolved. The resulting solution was added to the deprotected resin, and the reaction was rotated for 15 hours. The resin was filtered, washed sequentially with dichloromethane (3 x 10 mL), dimethylformamide (3 x 10 mL), dichloromethane (2 x 10 mL), methanol (3 x 10 mL) and dichloromethane (3 x 10 mL), and dried under house vacuum overnight at 40°C. The dry resin was treated with 18 mL of anhydrous toluene followed by triphenylphosphine (4.59 g, 17.5 mmol) and 3-bromo-1-propanol (2.43 g, 17.5 mmol). The resulting mixture was cooled to 0°C and treated in a dropwise fashion with a solution of diisopropyl azodicarboxylate (3.54 g, 17.5 mmol) in 9 mL of anhydrous toluene. The reaction was allowed to slowly rise to room temperature and stirred for 15 hours. The resin was filtered, washed sequentially with dichloromethane (2 x 25 mL), dimethylformamide (2 x 25 mL), dichloromethane (3 x 25 mL), methanol (3 x 25 mL) and dichloromethane (3 x 25 mL), and dried under house vacuum overnight at 40°C. The bromide functionalized resin was treated with a solution of diphenethylamine (5.52g, 28.0 mmol) (or 2-cyclohexyl-2-phenylethanamine (28.0 mmol, PCT Publication No. WO97/41846) for the cyclohexyl examples) in 20 mL of anhydrous dimethylsulfoxide and rotated for 15 hours. The resin was filtered, washed sequentially with dichloromethane (2 x 25 mL), dimethylformamide (2 x 25 mL), dichloromethane (3 x 25 mL), methanol (3 x 25 mL) and dichloromethane (3 x 25mL), and dried under house vacuum overnight at 40°C. A small portion of the secondary amine resin (0.20 g, 0.165 mmol) was treated with a solution of 2-chloro-3-trifluoromethylbenzaldehyde (1.03 g, 4.90 mmol) in 9 mL of dimethylformamide. Solid sodium triacetoxyborohydride (1.05 g, 4.90 mmol) was added followed by 1 mL of glacial acetic acid, and the reaction was rotated for 15 hours. The resin was filtered, washed sequentially with dichloromethane (2 x 25 mL), dimethylformamide (2 x 25 mL), dichloromethane (3 x 25 mL), methanol (3 x 25 mL) and dichloromethane (3 x 25 mL), and dried under house vacuum overnight at 40°C. The resin-bound product was treated with 5 mL of trifluoroacetic acid/dichloromethane (5/95) for 15 minutes, and the filtrate was collected. The cleavage procedure was repeated three times, and the filtrates were combined and concentrated under reduced pressure. The crude product was purified by preparative thin layer chromatography (silica gel, 1 mm plates, Merck 20 x 20 cm silica gel 60 F₂₅₄) eluting with ethyl acetate:hexane:triethyl-amine (74:25:1) to give 28 mg (29% yield based on theoretical loading of secondary amine resin) of title compound as a viscous oil: ¹H NMR (CDCl₃, 400 MHz) δ 7.45 (d, 1 H, J = 7.6), 7.25- 7.11 (m, 12 H), 6.91 (t, 1 H, J = 7.7), 6.66 (s, 1 H), 6.64 (s, 1 H), 5.35 (bs, 1 H), 5.48 (bs, 1 H), 4.11 (t, 1 H, J = 7.7), 3.77 (s, 2 H), 2.68 (t, 2 H, J = 5.9), 3.53 (s, 2 H), 2.12 (d, 2 H, J = 7.7), 2.70 (t, 2 H, J = 6.6), 1.83 (t, 2 H, J = 6.2); MS (ESP+) *m*/*e* 582 (MH⁺); TLC (methanol:methylene chloride/3:97) R_{*f*} = 0.53.

### Example 5: (3-{2-[(2,2-Diphenylethyl)-(4-methoxybenzyl)amino]-propoxy}phenyl)-acetamide

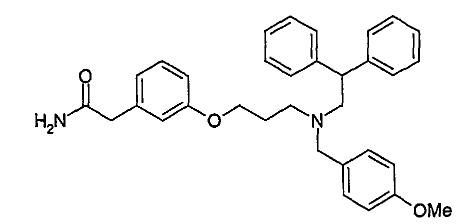

The title compound was prepared according to the procedures of **Example 4** to give 143 mg (43% yield based on theoretical loading of bromide functionalized resin) of a viscous oil: ¹H NMR (CDCl₃, 400MHz) δ 7.16-7.05 (m, 11 H), 6.93 (d, 2 H, J = 8.5), 6.81 (d, 1 H, J = 7.4), 6.63 (d, 2 H, J = 8.5), 6.53 (d, 1 H, J = 6.1), 6.63 (s, 1 H), 4.12 (t, 1 H, J = 7.8), 3.63 (s, 3 H), 3.49 (t, 2 H, J = 6.2), 3.44 (s, 2 H), 3.43 (s, 2 H), 2.95 (d, 2 H, J = 7.8), 2.50 (t, 2 H, J = 6.3), 1.68 (tt, 2 H, J = 6.2); MS (ESP+) *m*/*e* 509 (MH⁺); TLC (methanol:methylene chloride/3:97) R_{*f*} = 0.50.

### Example 6: 2-(3-{3-[(2,2-Diphenylethyl)(2-fluoro-4-methoxybenzyl)amino]propoxy} phenyl)acetamide

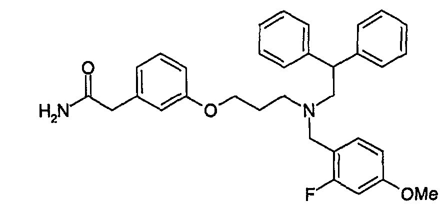

The title compound was prepared according to the methods of **Example 4**: HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R}= 1.85 min; MS (ESP+) *m*/*e* 527 (MH⁺).

### Example 7: 2-(3-{3-[(2,4-Dimethoxybenzyl)(2,2-diphenylethyl)amino]propoxy} phenyl)acetamide

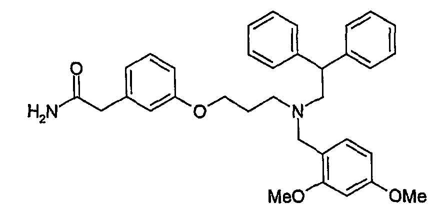

The title compound was prepared according to the procedures of **Example 4**: HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R} = 2.45 min; MS (ESP+) *m*/*e* 539 (MH⁺).

### Example 8: 2-[3-(3-{(2,2-Diphenylethyl)[4-fluoro-2-(trifluoromethyl)benzyl]amino} propoxy)phenyl]acetamide

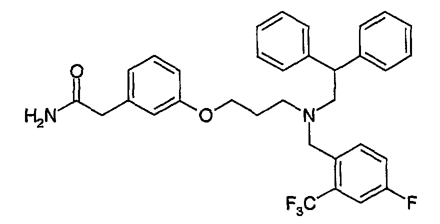

The title compound was prepared according to the procedure of **Example** 4: HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R}= 2.41 min; MS (ESP+) *m*/*e* 565 (MH⁺).

### Example 9: 2-(3-{3-[(2,3-Dichlorobenzyl)(2,2-diphenylethyl)amino]propoxy}phenyl) acetamide

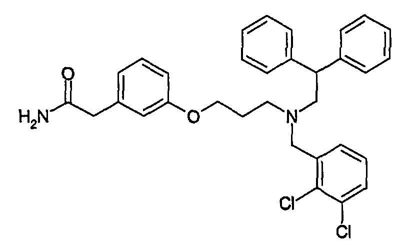

The title compound was prepared according to the procedure of **Example 4**: HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R}= 2.09 min; MS (ESP+) *m*/*e* 569 (MH⁺).

### Example 10: 2-[3-(3-{(2,2-Diphenylethyl)[3-(trifluoromethoxy)benzyl]aminol} propoxy)phenyl]acetamide

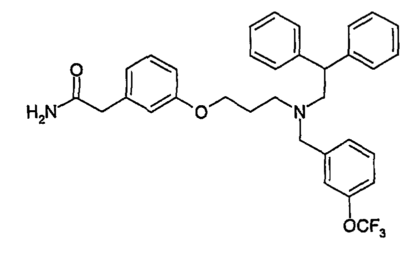

The title compound was prepared according to the procedure of **Example 4**: HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R}= 2.69 min; MS (ESP+) *m*/*e* 563 (MH⁺).

### Example 11: 2-(3-{3-[(2,2-Diphenylethyl)(3-fluoro-4-methoxybenzyl)amino]propoxy}phenyl)acetamide

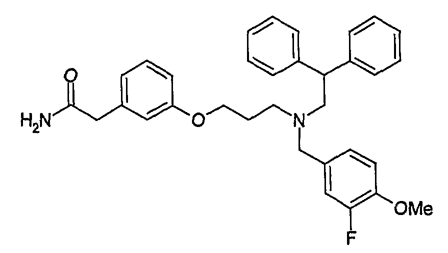

The title compound was prepared according to the procedure of **Example 4**: HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R}= 1.99 min; MS (ESP+) *m*/*e* 527 (MH⁺).

### Example 12: 2-(3-{3-[(2,5-Dimethoxybenzyl)(2,2-diphenylethyl)amino]propoxy} phenyl)acetamide

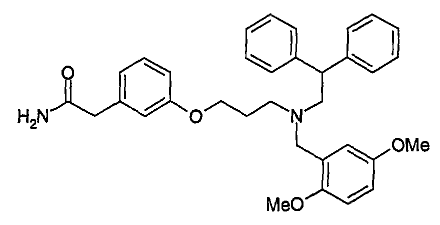

The title compound was prepared according to **Example 4**: ¹H NMR (CDCl₃, 400 MHz) δ 7.28-7.09 (m, 12 H), 6.91 (d, 1 H, J = 8.3), 6.86 (d, 1 H, J = 7.4), 6.4 (m, 2 H), 6.37 (s, 1 H), 6.25 (d, 1 H, J = 8.1), 4.15 (t, 1 H, J = 7.0), 3.79 (s, 3 H), 3.75-3.56 (m, 8 H), 3.01 (d, 2 H, J = 7.6), 2.61 (t, 2 H, J = 5.7), 1.80 (t, 2 H, J = 6); HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R}=2.27 min; MS (ESP+) *m*/*e* 539 (MH⁺).

### Example 13: 2-[3-(3-{(2,2-Diphenylethyl)[3-(trifluoromethyl)benzyl]amino}propoxy) phenyl]acetamide

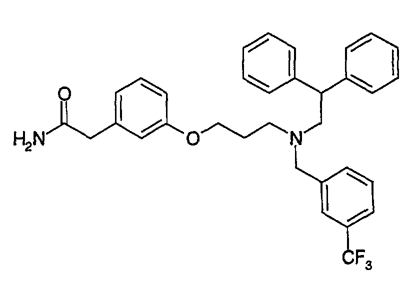

The title compound was prepared according to the procedure of **Example 4**: HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R}= 2.41 min; MS (ESP+) *m*/*e* 546 (MH⁺).

### Example 14: 2-[3-(3-{(2,2-Diphenylethyl)[2-fluoro-3-(trifluoromethyl)benzyl]amino} propoxy)phenyl]acetamide

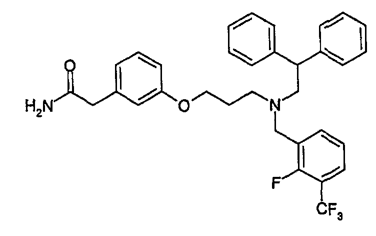

The title compound was prepared according to the procedure of **Example 4**: ¹H NMR (CDCl₃, 400 MHz) δ 7.37 (t, 1 H, J = 6.9), 7.23-7.11 (m, 12 H), 6.88 (t, 1 H, J = 6.9), 6.82 (d, 1 H, J = 7.5), 6.64-6.61 (m, 2 H), 4.12 (t, 1 H, 7.8), 3.76-3.62 (m, 4 H), 3.52 (s, 2 H), 3.09 (d, 2 H, J = 6), 2.67 (s, 2 H), 1.82 (s, 2 H); HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R}=2.44 min; MS (ESP+) *m*/*e* 565 (MH⁺).

### Example 15: (3-{2-[(2,2-Diphenylethyl)-(4-methoxybenzyl)amino]-propoxy}phenyl) acetic acid

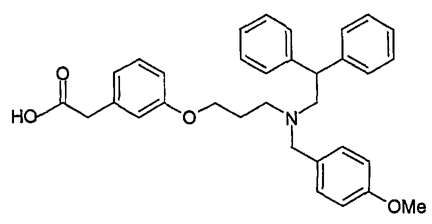

### Solid-phase synthesis:

Argogel-MB-OH (6.0g, 2.40mmol, Argonaut Technologies) was treated with a solution of (3-{[*tert*-butyl(dimethyl)silyl]oxy}phenyl)acetic acid (5.40g, 19.2 mmol, Eur. Pat. Appl. (1987) Application: EP 87-303742 19870428) in 50 mL of anhydrous dichloromethane followed by dicyclohexylcarbodiimide (4.16g, 19.2 mmol) and 4-dimethylaminopyridine (2.50 g, 19.2 mmol). After rotating at room temperature for 15 hours, the resin was filtered, washed sequentially with dichloromethane (2 x 25 mL), dimethylformamide (2 x 25mL), dichloromethane (3 x 25 mL), methanol (3 x 25 mL), dichloromethane (3 x 25 mL) and diethyl ether (2 x 25 mL). After drying under house vacuum overnight at 40°C, the resin was treated with 1.0 M tetrabutylammonium fluoride (24 mL, 23.4 mmol) in tetrahydrofuran, and the mixture was rotated for 4 hours. The resin was filtered, washed sequentially with dichloromethane (2 x 25 mL), dimethylformamide (2 x 25 mL), dichloromethane (3 x 25 mL), methanol (3 x 25 mL), and dichloromethane (3 x 25 mL) to give the deprotected phenol. The dry resin was treated with 90 mL of anhydrous toluene followed by triphenylphosphine (15.8 g, 60.0 mmol) and 3-bromo-1-propanol (8.4 g, 60.0 mmol). Upon cooling to 0°C, diisopropyl azodicarboxylate (12.1 g, 60.0 mmol) in 20 mL of anhydrous toluene was added in a dropwise fashion. The reaction was allowed to warm to room temperature and stirred for 15 hours. The resin was filtered, washed sequentially with dichloromethane (2 x 50 mL), dimethylformamide (2 x 50 mL), dichloromethane (3 x 50 mL), methanol (2 x 50 mL) and dichloromethane (3 x 50 mL), and dried under house vacuum. The bromide functionalized resin was treated with a solution of diphenethylamine (25.0 g, 127 mmol) in 60 mL of anhydrous dimethylsulfoxide, and the reaction was rotated for 15 hours. The resin was filtered, washed sequentially with dichloromethane (2 x 50 mL), dimethylformamide (2 x 50 mL), dichloromethane (3 x 50 mL), methanol (3 x 50 mL) and dichloromethane (3 x 50 mL), and dried under house vacuum at 40°C. The secondary amine resin (5.75 g, 2.0 mmol) was treated with a solution of 4-methoxybenzaldehyde (5.44 g, 40.0 mmol) in 80 mL of 8% acetic acid in dimethylformamide. Solid sodium triacetoxyborohydride (8.5 g, 40.0 mmol) was added, and the reaction was rotated for 15 hours. The resin was filtered, washed sequentially with dichloromethane (2 x 50 mL), dimethylformamide (2 x 50 mL), dichloromethane (3 x 50 mL), methanol (3 x 50 mL) and dichloromethane (3 x 50mL), and dried under house vacuum overnight at 50°C. The resin-bound product was treated with 30 mL of trifluoroacetic acid/dichloromethane (15/85) for 15 minutes, and the filtrate was collected. The cleavage procedure was repeated again, and the combined filtrates were concentrated under reduced pressure. The crude product was purified by preparative thin layer chromatography (silica gel, 1 mm plates, Merck 20 x 20 cm silica gel 60 F₂₅₄) eluting with methanol:dichloromethane (3:97) to afford 57 mg (6% yield based on theoretical loading of secondary amine resin) of the title compound as a viscous oil: ¹H NMR (CDCl₃, 400MHz) δ 7.18-7.03 (m, 10 H), 6.93 (d, 2 H, J = 8.6), 6.72 (d, 2 H, J = 7.6), 6.65 (d, 2 H, J = 8.6), 6.58 (s, 1 H), 6.49 (d, 1 H, J = 6.4), 4.71 (bs, 2 H), 4.11 (t, 1 H, J = 7.8), 3.65 (s, 3 H), 3.51 (t, 2 H, J = 6.2), 3.49 (s, 2 H), 3.40 (s, 2 H), 2.97 (d, 2 H, J = 7.8), 2.53 (t, 2 H, J = 6.5), 1.70 (tt, 2 H, J = 6.2); MS (ESP+) *m*/*e* 510 (MH⁺); TLC (CH₂Cl₂:MeOH/97:3) R_{*f*}= 0.13.

### Example 16: 2-(3-{3-[[2-Chloro-3-(trifluoromethyl)benzyl](2,2-diphenylethyl)amino] propoxy}-phenyl)acetic acid

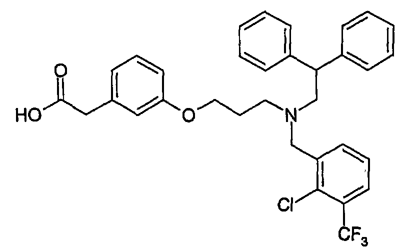

The title compound was prepared according to the procedure of **Example 15** to give 7.0 mg (5% yield based on theoretical loading of secondary amine resin) of a viscous oil: ¹H NMR (CDCl₃, 400MHz) δ 7.42 (d, 1 H, J = 7.6), 7.23-7.10 (m, 12 H), 6.85 (t, 2 H, J = 8.1), 6.63 (s, 1 H), 6.61 (s, 1 H), 4.11 (t, 1 H, J = 7.8), 3.75 (s, 2 H), 3.63 (t, 2 H, J = 6.0), 3.59 (s, 2 H), 2.12 (d, 2 H, J = 7.8), 2.67 (t, 2 H, J - 6.6), 1.81 (tt, 2 H, J = 6.2); MS (ESP+) *m*/*e* 582 (MH⁺); TLC (EtOAc:hexanes/1:1) R_{*f*}= 0.58.

### Example 17: (3-{2-[(2,2-Diphenylethyl)-(4-methoxybenzyl)amino]-propoxy}phenyl) acetic acid hydrochloride salt

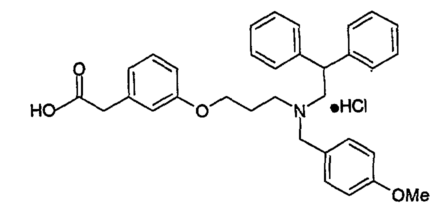

### Solution-phase synthesis:

A solution of methyl (3-{3-[(2,2-diphenylethyl)(4-methoxybenzyl)amino]propoxy}phenyl)acetate (100 mg, 0.19 mmol) in 1.5 mL of tetrahydrofuran and 1 mL of water was treated with 1N aqueous LiOH (0.29 mL, 0.29 mmol). After stirring at room temperature for 2 hours, additional 1N aqueous LiOH (0.29 mL, 0.29 mmol) was added and stirring was continued for 2 hours. The reaction was neutralized with AcOH (66 µL, 0.58 mmol) and poured into H₂O/EtOAc. The layers were separated and the aqueous layer was extracted with EtOAc (3x). The combined organic layers were washed with brine (1x), dried over magnesium sulfate, filtered, and concentrated in vacuo. The crude material was purified by preparative thin layer chromatography (silica gel, 1 mm plates, Merck 20 x 20 cm silica gel 60 F₂₅₄) eluting with CH₂Cl₂:MeOH (95:5) to afford an oil. The oil was dissolved in Et₂O and acidified with excess HCl/Et₂O. The reaction was concentrated in vacuo and dried under reduced pressure to give 155 mg (75% yield) of the title compound as a white solid: ¹H NMR (C₅D₅N, 400MHz) δ 7.40-7.00 (m, 15 H), 6.89 (d, 2 H, J = 8.6), 6.82 (dd, 1 H, J = 8.1, 2.2), 4.41 (t, 1 H, J = 7.6), 3.89 (s, 2 H), 3.67 (t, 2 H, J = 6.4) 3.64 (s, 3 H), 3.59 (s, 2 H), 3.13 (d, 2 H, J = 7.6), 2.64 (t, 2 H, J = 6.7), 1.90-1.80 (m, 2 H).

### Example 18: 2-(3-{3-[[2-Chloro-3-(trifluoromethyl)benzyl](2,2-diphenylethyl)amino] propoxy}-phenyl)acetic acid hydrochloride salt

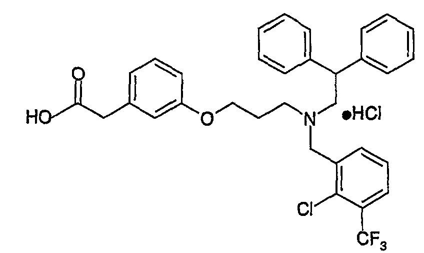

The title compound was prepared in 56% yield from methyl (3-{3-[[2-chloro-3-(trifluoromethyl)benzyl](2,2-diphenylethyl)amino]propoxy}phenyl)acetate according to the procedures of **Example 17**: ¹H NMR (C₅D₅N, 400MHz) δ 7.60-7.05 (m, 15 H), 7.01 (t, 1 H, J = 7.6), 6.84 (dd, 1 H, J = 8.4, 2.4), 4.32 (t, 1 H, J = 7.6), 3.89 (s, 2 H), 3.77 (s, 2 H), 3.71 (t, 2 H, J = 5.6), 3.16 (d, 2 H, J = 7.6), 2.65 (t, 2 H, J = 6.4), 1.88-1.78 (m, 2 H).

### Example 19: Methyl (3-{3-[(2,2-diphenylethyl)(4-methoxybenzyl)amino] propoxy}phenyl)acetate

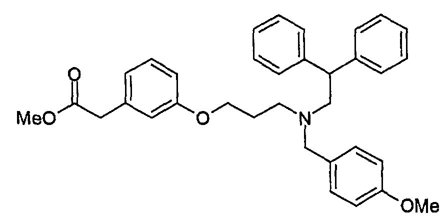

A solution of methyl-[3-(3-bromopropoxy)phenyl]acetate (100 mg, 0.35 mmol) and *N*-(2,2-diphenylethyl)-*N*-(4-methoxybenzyl)amine (110 mg, 0.35 mmol) in 0.70 mL of acetonitrile was treated with solid K₂CO₃ (48 mg, 0.35 mmol). The reaction was heated to reflux and stirred 15 hours. Upon cooling to room temperature, the reaction was filtered through a pad of silica gel washing with EtOAc, and the filtrate was concentrated in vacuo. The crude product was purified by preparative thin layer chromatography (silica gel, 1 mm plates, Merck 20 x 20 cm silica gel 60 F₂₅₄) eluting with hexanes:EtOAc (6:1) to afford 100 mg (55% yield) of title compound as a viscous oil: ¹H NMR (CDCl₃, 400MHz) δ 7.25-7.10 (m, 10 H), 6.98 (d, 2 H, J = 8.6), 6.84 (d, 1 H, J = 7.6), 6.70 (d, 2 H, J = 8.6), 6.65 (br s, 1 H), 6.61 (dd, 1 H, J = 8.4, 2.4), 4.17 (t, 1 H, J = 7.6), 3.76 (s, 3 H), 3.68 (s, 3 H), 3.60 (t, 2 H, J = 6.4), 3.59 (s, 2 H), 3.54 (s, 2 H), 3.03 (d, 2 H, J = 7.6), 2.60 (t, 2 H, J = 6.4), 1.79 (m, 2H); HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R}=2.49 min; MS (ESP+) *m*/*e* 524 (MH⁺); TLC (EtOAc:hexanes/1:1) R_{*f*}= 0.20.

### Example 20: Methyl (3-{3-[[2-chloro-3-(trifluoromethyl)benzyl](2,2-diphenylethyl) amino]propoxy}phenyl)acetate

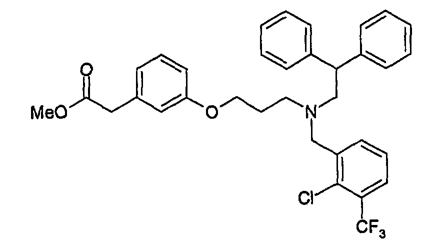

A solution of methyl [3-(3-bromopropoxy)phenyl]acetate (1.0 g, 3.48 mmole) and *N*-[2-chloro-3-(trifluoromethyl)benzyl]-2,2-diphenylethanamine (1.63 g, 4.18 mmole) in 20 mL of acetonitrile was treated with potassium carbonate (0.72 g, 5.2 mmol). The reaction mixture was heated to reflux and stirred for 4 days. The reaction mixture was filtered, and the filtrate was concentrated in vacuo. The crude product was purified by flash chromatography (silica gel cartridge, Biotage 32-63um, 60A) with 10% EtOAc:hexanes as the eluent to afford 1.69 g (81% yield) of the title compound as a viscous oil: ¹H NMR (CDCl₃, 400 MHz) δ 7.46-7.44 (d, 1 H, J = 7.7), 7.25-7.14 (m, 12 H), 6.91-6.84 (m, 2 H), 6.66-6.62 (m, 2 H), 4.15-4.09 (t, 1 H, J= 7.6), 3.78 (s, 1 H), 3.69-3.66 (m, 5 H), 3.59(S, 2 H), 3.15-3.13 (d, 2 H, J= 7.7), 2.72-2.68 (t, 2 H, J= 6.6), 1.87-1.80 (m, 2 H); MS (ESP+) *m*/*e* 597 (MH⁺); TLC (hexanes:EtOAc/9:1) R_{f} = 0.36.

### Example 21: Methyl [3-(3-bromopropoxy)phenyl]acetate

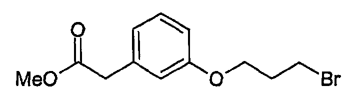

A solution of methyl 3-hydroxyphenylacetate (11.3 g, 0.068 mole) in 300 mL of anhydrous toluene was treated with 3-bromopropanol (12.2 g, 0.088 mol). Polymer bound triphenylphosphine (36.0 g, 0.108 mole, 3 mmol/g, Fluka Chemie) was then added, and the mixture reacted for 15 minutes. The reaction mixture was then cooled to 0°C and diisopropylazodicarboxylate (16.9 g, 0.084 mol) was added in a dropwise fashion. After stirring at room temperature overnight, the crude reaction mixture was filtered and the solid washed with 100 mL toluene. After concentration of the filtrate in vacuo, the crude product was purified by column chromatography over silica gel (silica gel 60, EM Science) using 15% EtOAc:hexane as eluent to afford 15.8 g (81% yield) of the title compound as an oil: ¹H NMR (CDCl₃, 400 MHz) δ 7.23-7.19 (m, 1 H), 6.85-6.7 (m, 3), 4.09-4.06 (t, 2 H, J = 5.8), 3.67 (s, 3 H), 3.67-3.56 (m, 4 H), 2.32-2.26 (p, 2 H, J= 6.0) ; MS (ESP+) *m*/*e* 288 (MH⁺); TLC (hexanes:EtOAc/3:1) R_{*f*} = 0.68. Anal. (C₁₂H₁₅O₃Br) C, H, N.

### Example 22: N-(2,2-Diphenylethyl)-N-(4-methoxybenzyl)amine

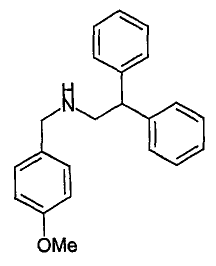

A solution of 2,2-diphenethylamine (10.0 g, 50.7 mmol) and 98% p-anisaldehyde (6.17 mL , 50.7 mmol) in 80 mL of methanol and 40 mL of trimethylorthoformate was stirred at room temperature for 15 hours whereupon polymer-supported borohydride resin (20.3 g, 55.8 mmol, 2.5 mmol/g, Aldrich) was added in one portion. After stirring at room temperature for 24 h, the reaction was filtered and the filtrate was concentrated in vacuo. The crude product was purified by column chromatography over silica gel (silica gel 60, EM Science) using EtOAc:hexane/40:60 with 1% NH₄OH as the eluent to give 13.0 g (81% yield) of the title compound as an oil: ¹H NMR (CDCl₃, 400 MHz) δ7.32-7.12 (m, 12 H), 4.22 (t, 1 H, J = 7.6), 3.78 (s, 3 H), 3.75 (s, 2 H), 3.21 (d, 2 H, J = 7.6); HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R}=1.67 min; MS (ESP+) *m*/*e* 318 (MH⁺); TLC (hexanes:EtOAc/4:1) R_{*f*}= 0.48.

### Example 23: N-[2-Chloro-3-(trifluoromethyl)benzyl]-N-(2,2-diphenylethyl)amine

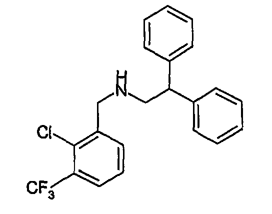

The title compound was prepared in 57% yield from 2,2-diphenethylamine and 2-chloro-3-trifluoromethylbenzaldehyde as in Example 22: ¹H NMR (CDCl₃, 400 MHz) δ 7.57 (d, 1 H, J = 8.0), 7.52 (d, 1 H, J = 7.6), 7.32-7.15 (m, 11 H), 4.20 (t, 1 H, J = 7.6), 3.94 (s, 2 H), 3.22 (d, 2 H, J = 7.6); HPLC (Waters symmetry shield, RPq 3.5 micron, 2.1 x 30 mm, 85:15/H₂O:CH₃CN with 0.1% HCOOH to 100% CH₃CN after 4 min, flow rate = 0.8 mL/min) t_{R}=2.39 min; MS (ESP+) *m*/*e* 390 (MH⁺); TLC (hexanes:EtOAc/4:1) R_{*f*} = 0.42.

### Example 24: Ethyl 4-[{3-[3-(aminocarbonyl)phenoxy]propyl}(2,2-diphenylethyl)-amino]-1-piperidinecarboxylate

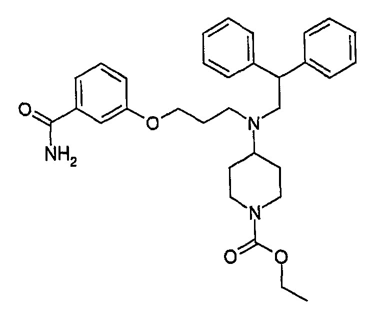

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 1.98 min; MS (ESP+) *m*/*e* 530 (MH⁺).

### Example 25: 3-{3-[(1-Benzoyl-4-piperidinyl)-(2,2-diphenylethyl)amino]propoxy}benzamide

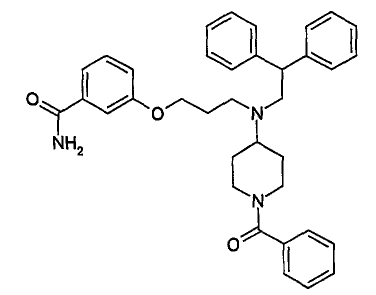

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 1.99 min; MS (ESP+) *m*/*e* 562 (MH⁺).

### Example 26: 3-{3-[(1-Acetyl-4-piperidinyl)(2,2-diphenylethyl)amino]propoxy} benzamide

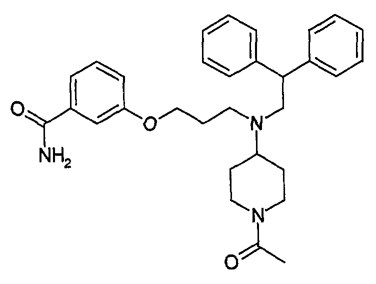

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 1.70 min; MS (ESP+) *m*/*e* 500 (MH⁺).

### Example 27: Benzyl 4-[{3-[3-(aminocarbonyl)phenoxy]propyl}(2,2-diphenylethyl)-amino]-1-piperidinecarboxylate

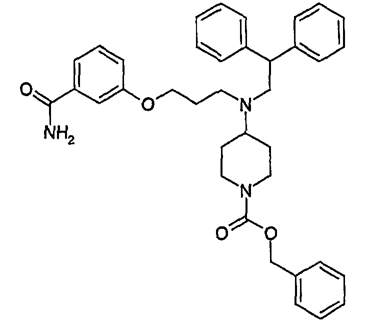

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.26 min; MS (ESP+) *m*/*e* 592 (MH⁺).

### Example 28: 3-(3-{(2,2-Diphenylethyl)[1-(2-phenylethyl)-4-piperidinyl]amino} propoxy)benzamide

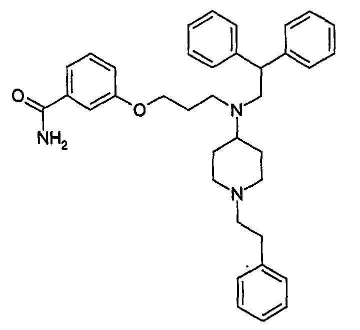

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 1.90 min; MS (ESP+) *m*/*e* 562 (MH⁺).

### Example 29: Ethyl 4-[{3-[3-(aminocarbonyl)phenoxy]propyl}(2-cyclohexyl-2-phenylethyl)amino]-1-piperidinecarboxylate

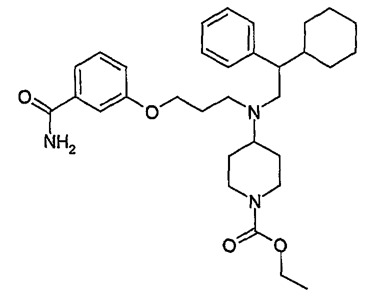

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.19 min; MS (ESP+) *m*/*e* 536 (MH⁺).

### Example 30: 3-{3-[(1-Benzoyl-4-piperidinyl)(2-cyclohexyl-2-phenylethyl)amino] propoxy}benzamide

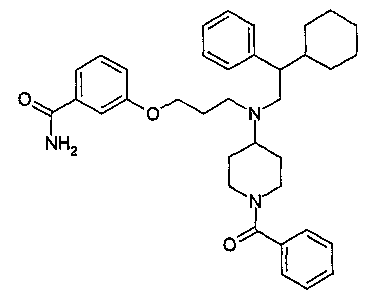

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.17 min; MS (ESP+) *m*/*e* 568 (MH⁺).

### Example 31: 3-{3-[(1-Acetyl-4-piperidinyl)(2-cyclohexyl-2-phenyethyl)amino] propoxy}benzamide

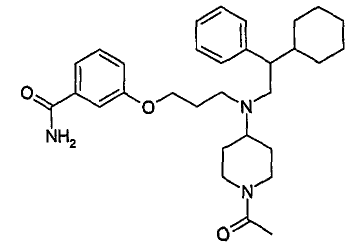

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 1.95 min; MS (ESP+) *m*/*e* 506 (MH⁺).

### Example 32: tert-Butyl 4-[{3-[3-(aminocarbonyl)phenoxy]propyl}(2-cyclohexyl-2-phenylethyl)amino]-1-piperidinecarboxylate

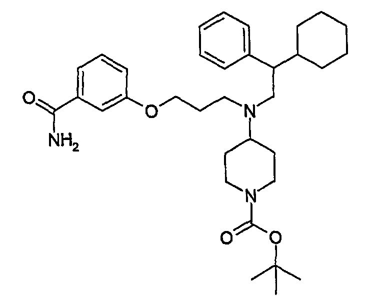

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.41 min; MS (ESP+) *m*/*e* 564 (MH⁺).

### Example 33: Benzyl 4-[{3-[3-(aminocarbonyl)phenoxy]propyl}(2-cyclohexyl-2-phenylethyl)amino]-1-piperidinecarboxylate

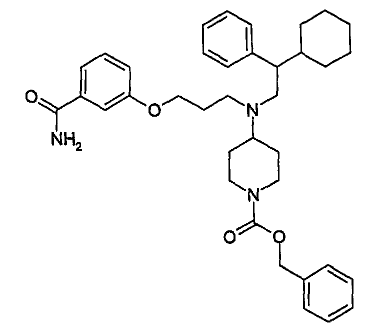

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.41 min; MS (ESP+) *m*/*e* 598 (MH⁺).

### Example 34: 3-{3-[(1-Benzyl-4-piperidinyl)(2-cyclohexyl-2-phenylethyl)amino] propoxy}benzamide

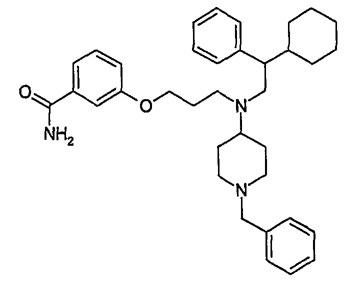

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 1.70 min; MS (ESP+) m/e 554 (MH⁺).

### Example 35: Ethyl 4-[{3-[3-(2-amino-2-oxoethyl)phenoxy]propyl}(2,2-diphenylethyl)amino]-1-piperidinecarboxylate

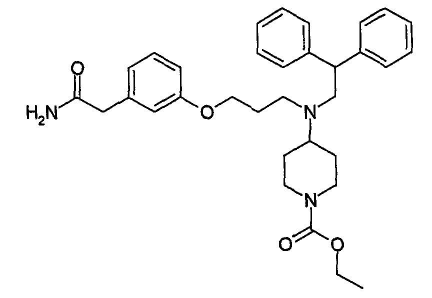

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.01 min; MS (ESP+) *m*/*e* 544 (MH⁺).

### Example 36: 2-(3-{3-[(1-Benzoyl-4-piperidinyl)(2,2-diphenylethyl)amino] propoxy}phenyl)acetamide

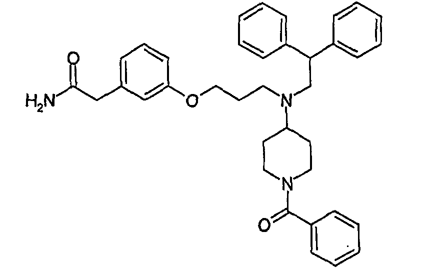

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.04 min; MS (ESP+) *m*/*e* 576 (MH⁺).

### Example 37: 2-(3-{3-[(1-Acetyl-4-piperidinyl)(2,2-diphenylethyl)amino]propoxy} phenyl)acetamide

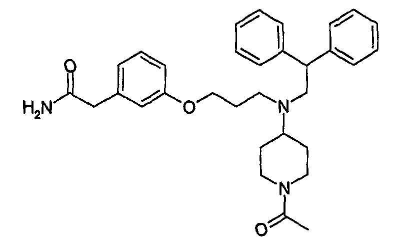

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 1.74 min; MS (ESP+) *m*/*e* 514 (MH⁺).

### Example 38: tert-Butyl 4-[{3-[3-(2-amino-2-oxoethyl)phenoxy]propyl}(2,2-diphenylethyl)amino]-1-piperidinecarboxylate

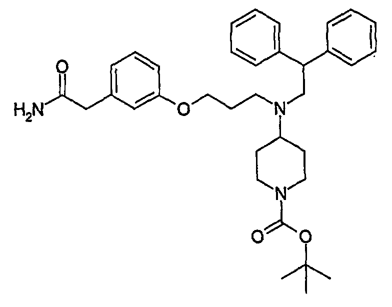

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.25 min; MS (ESP+) *m*/*e* 572 (MH⁺).

### Example 39: Benzyl 4-[{3-[3-(2-amino-2-oxoethyl)phenoxy]propyl}(2,2-diphenylethyl)amino]-1-piperidinecarboxylate

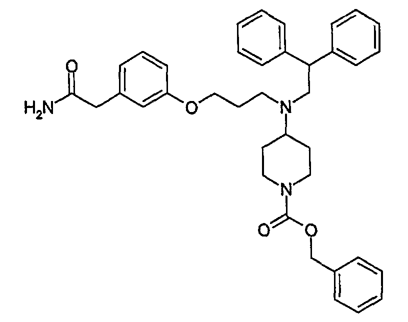

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.30 min; MS (ESP+) *m*/*e* 606 (MH⁺).

### Example 40: 2-[3-(3-{(2,2-Diphenylethyl)[1-(2-phenylethyl)-4-piperidinyl]-amino}propoxy)phenyl]acetamide

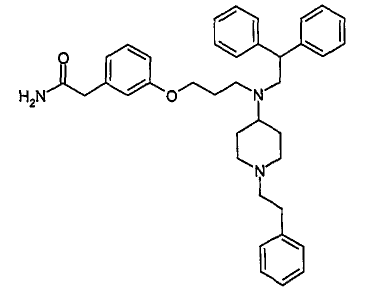

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 1.67 min; MS (ESP+) *m*/*e* 576 (MH⁺).

### Example 41: 2-(3-{3-[(1-Benzoyl-4-piperidinyl)(2-cyclohexyl-2-phenylethyl) amino]propoxy}phenyl)acetamide

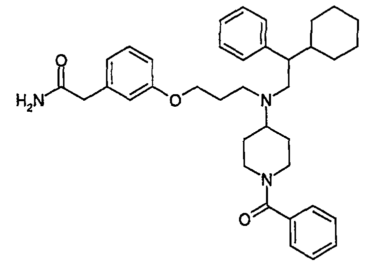

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.23 min; MS (ESP+) *m*/*e* 582 (MH⁺).

### Example 42: 2-(3-{3-[(1-Acetyl-4-piperidinyl)(2-cyclohexyl-2-phenylethyl) amino]propoxy}phenyl)acetamide

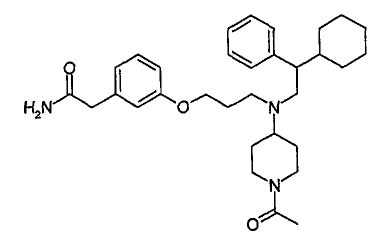

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 1.95 min; MS (ESP+) *m*/*e* 520 (MH⁺).

### Example 43: Benzyl-4-[{3-[3-(2-amino-2-oxoethyl)phenoxy]propyl}(2-cyclohexyl-2-phenylethyl)amino]-1-piperidinecarboxylate

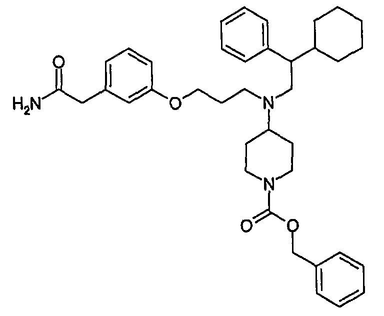

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.44 min; MS (ESP+) *m*/*e* 612 (MH⁺).

### Example 44: 3-{3-[(3-Cyanobenzyl)(2,2-diphenylethyl)amino]propoxy}benzamide

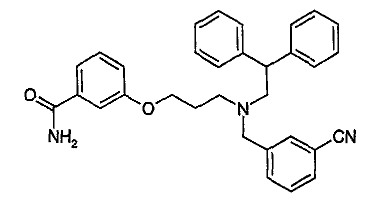

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.40 min; MS (ESP+) *m*/*e* 490 (MH⁺).

### Example 45: 3-{3-[Cyclohexyl(2,2-diphenylethyl)amino]propoxy}benzamide

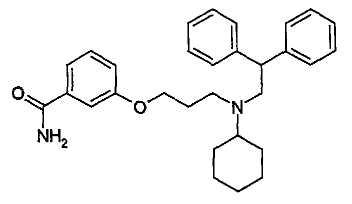

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 1.99 min; MS (ESP+) *m*/*e* 457 (MH⁺).

### Example 46: 4-[{3-[3-(Aminocarbonyl)phenoxy]propyl}(2,2-diphenylethyl)amino]-1 piperidinecarboxamide

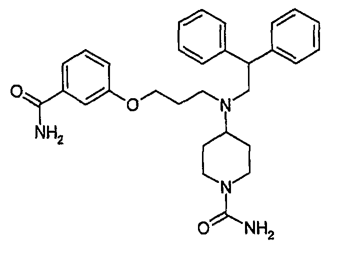

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 1.60 min; MS (ESP+) *m*/*e* 501 (MH⁺).

### Example 47: 3-{3-[(1,3-Benzodioxol-4-ylmethyl)(2,2-diphenylethyl)amino] propoxy}benzamide

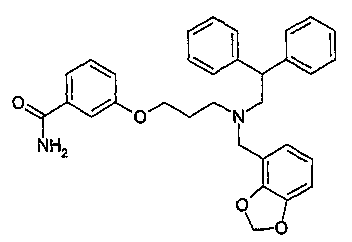

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.12 min; MS (ESP+) *m*/*e* 509 (MH⁺).

### Example 48: 3-{3-[(3,4-Dimethoxybenzyl)(2,2-diphenylethyl)amino]propoxy} benzamide

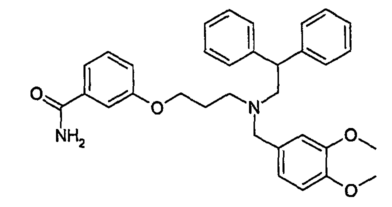

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.00 min; MS (ESP+) *m*/*e* 525 (MH⁺).

### Example 49: 3-{3-[(4-Cyanobenzyl)(2-cyclohexyl-2-phenylethyl)amino] propoxy}benzamide

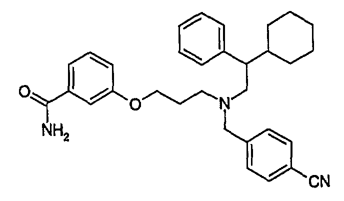

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.45 min; MS (ESP+) m/e 496 (MH⁺).

### Example 50: 3-{3-[(4-Cyanobenzyl)(2-cyclohexyl-2-phenylethyl)amino] propoxy}benzamide

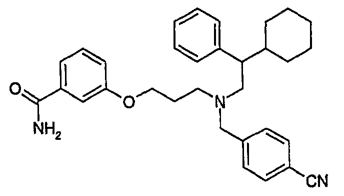

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.45 min; MS (ESP+) *m*/*e* 496 (MH⁺).

### Example 51: 2-(3-{3-[Cyclohexyl(2,2-diphenylethyl)amino]propoxy} phenyl)acetamide

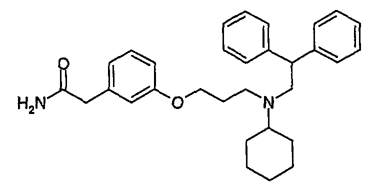

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.07 min; MS (ESP+) *m*/*e* 471 (MH⁺).

### Example 52: 2-(3-{3-[(3,4-Dimethoxybenzyl)(2,2-diphenylethyl)amino]propoxy} phenyl)acetamide

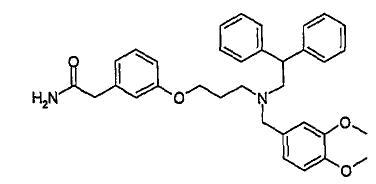

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.06 min; MS (ESP+) m/e 539 (MH⁺).

### Example 53: 3-{3-[(2-Cyclohexyl-2-phenylethyl)(3,4-dimethoxybenzyl)amino] propoxy}benzamide

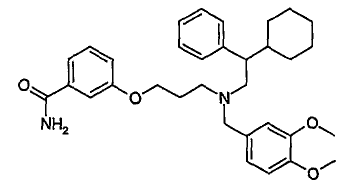

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.62 min; MS (ESP+) *m*/*e* 531 (MH⁺).

### Example 54: 3-{3-[(2,6-Dichlorobenzyl)(2,2-diphenylethyl)amino]propoxy}benzamide

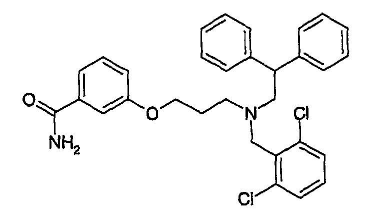

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 3.04 min; MS (ESP+) *m*/*e* 534 (MH⁺).

### Example 55: 3-{[{3-[3-(Aminocarbonyl)phenoxy]propyl}(2,2-diphenylethyl) amino]methyl}benzoic acid

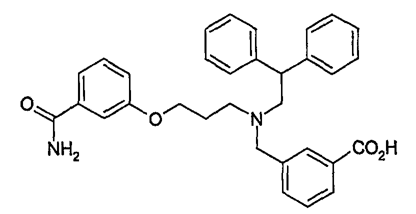

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.00 min; MS (ESP+) *m*/*e* 509 (MH⁺).

### Example 56: 4-{[{3-[3-(Aminocarbonyl)phenoxy]propyl}(2,2-diphenylethyl) amino]methyl}benzoic acid

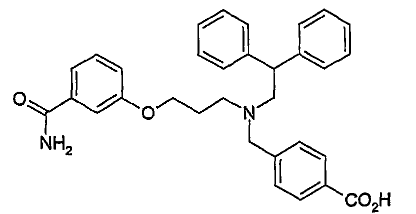

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.05 min; MS (ESP+) *m*/*e* 509 (MH⁺).

### Example 57: 3-(3-{(2,2-Diphenylethyl)[(5-methoxy-1H-indol-3-yl)methyl]amino} propoxy)benzamide

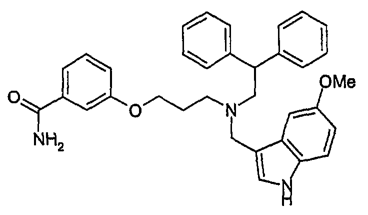

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.12 min; MS (ESP+) *m*/*e* 534 (MH⁺).

### Example 58: 3-{3-[(2,2-Diphenylethyl)(4-methoxybenzyl)amino]propoxy}benzamide

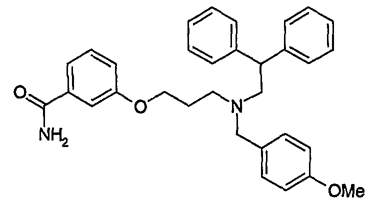

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.10 min; MS (ESP+) *m*/*e* 495 (MH⁺).

### Example 59: 3-{3-[[(1-Acetyl-1H-indol-3-yl)methyl](2,2-diphenylethyl)amino] propoxy}benzamide

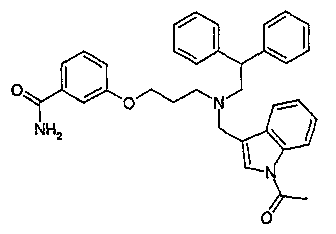

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.27 min; MS (ESP+) *m*/*e* 546 (MH⁺).

### Example 60: Methyl 4-{[{3-[3-(aminocarbonyl)phenoxy]propyl}(2,2-diphenylethyl) amino]methyl}benzoate

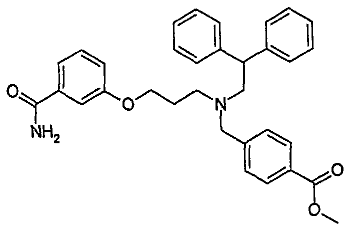

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.36 min; MS (ESP+) *m*/*e* 523 (MH⁺).

### Example 61: 3-{3-[(2,3-Dihydro-1,4-benzodioxin-6-ylmethyl)(2,2-diphenylethyl) amino]propoxy}benzamide

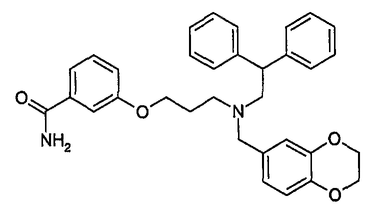

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.06 min; MS (ESP+) *m*/*e* 523 (MH⁺).

### Example 62: 3-{3-[(2,2-Diphenylethyl)(4-pyridinylmethyl)amino]propoxy}benzamide

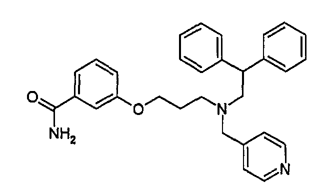

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.12 min; MS (ESP+) m/e466 (MH⁺).

### Example 63: 2-(3-{3-[(2-Cyclohexyl-2-phenylethyl)(3,4-difluorobenzyl)amino]-propoxy}phenyl)acetamide

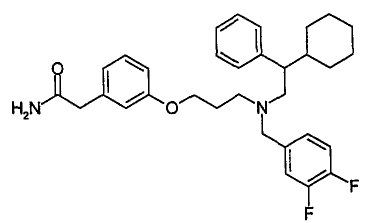

The title compound was prepared according to the procedures of **Example 4**: HPLC (Waters symmetry shield, C8, 3.5 micron, 2.1 x 50 mm, 85:15/H₂O:MeOH to 100% MeOH after 4 min, flow rate = 0.8 mL/min) t_{R}= 2.12 min; MS (ESP+) *m*/*e* 521 (MH⁺).

### Example 64: 2-(3-{3-(2,2-Diphenylethyl)[[(6-chloro-1,3-benzodioxol-5-yl)methyl] amino]propoxy}phenyl)acetamide

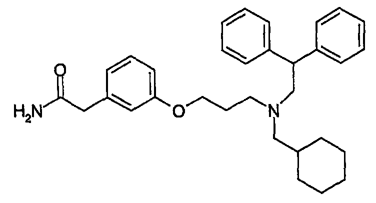

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 4.04 min; MS (ESP+) *m*/*e* 557 (MH⁺).

### Example 65: 2-(3-{3-[(2,2-Diphenylethyl)(cyclohexylmethyl)amino]propoxy}phenyl) acetamide

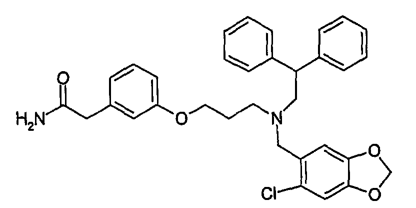

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.56 min; MS (ESP+) *m*/*e* 485 (MH⁺).

### Example 66: 2-(3-{3-[(2,2-Diphenylethyl)(bicyclo[2.2.1]hept-5-en-2-ylmethyl)amino]propoxy}phenyl) acetamide

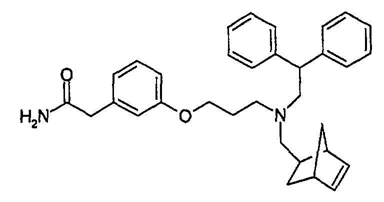

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.49 min; MS (ESP+) m/e 495 (MH⁺).

### Example 67: 2-(3-{3-[(2,2-diphenylethyl)(2,4-dimethoxy-5-pyrimidinyl) methyl)amino]propoxy} phenyl) acetamide

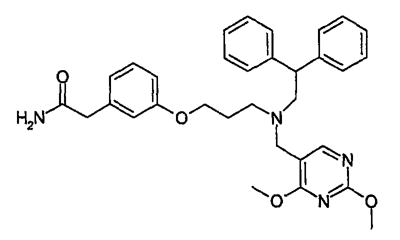

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.45 min; MS (ESP+) *m*/*e* 541 (MH⁺).

### Example 68: 2-(3-{3-[(2,2-Diphenylethyl(5-isopropyl-3-methyl-4-isoxazolyl)-methyl)amino]propoxy}phenyl) acetamide

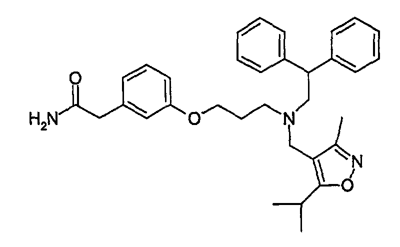

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 4.45 min; MS (ESP+) *m*/*e* 526 (MH⁺).

### Example 69: 2-(3-{3-[(2,2-Diphenylethyl)(3,4-dihydro-2H-pyran-2-ylmethyl)amino]-propoxy}phenyl)acetamide

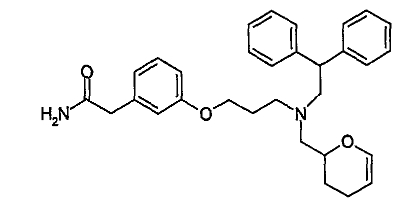

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.53 min; MS (ESP+) *m*/*e* 485 (MH⁺).

### Example 70: 2-(3-{3-[(2,2-Diphenylethyl)( 4-chloro-1H-pyrazol-3-yl)methyl)amino]propoxy}phenyl)acetamide

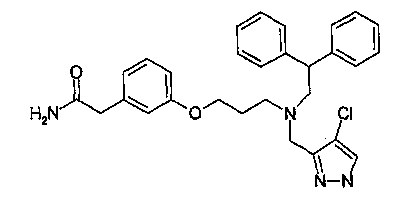

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.43 min; MS (ESP+) *m*/*e* 503 (MH⁺).

### Example 71: 2-(3-{3-[(2,2-Diphenylethyl)()[(7-methoxy-1,3-benzodioxol-5-yl)methyl)amino]propoxy}phenyl)acetamide

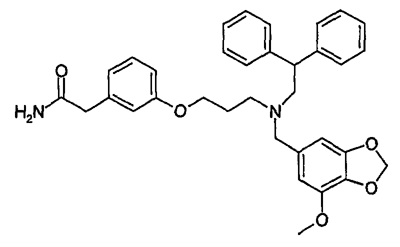

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.51 min; MS (ESP+) *m*/*e* 553 (MH⁺).

### Example 72: 2-(3-{3-[(2,2-Diphenylethyl-(2,6,6-trimethyl-1-cyclohexen-1-yl)ethyl)amino]propoxy}phenyl)acetamide

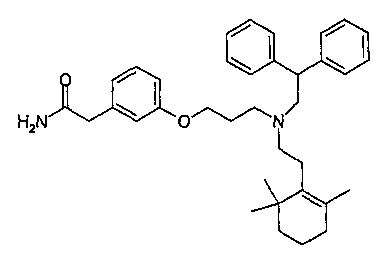

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.97 min; MS (ESP+) *m*/*e* 540 (MH⁺).

### Example 73: 2-(3-{3-[(2,2-Diphenylethyl)(3-cyclohexen-1-ylmethyl)amino]-propoxy}phenyl)acetamide

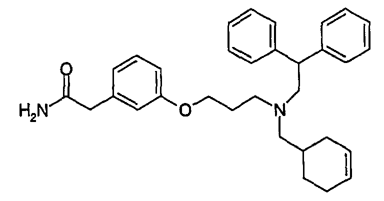

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.51 min; MS (ESP+) *m*/*e* 483 (MH⁺).

### Example 74: 2-[3-(3-{(2,2-Diphenylethyl)[(2E)-3-phenyl-2-propenyl]amino}propoxy) phenyl]acetamide

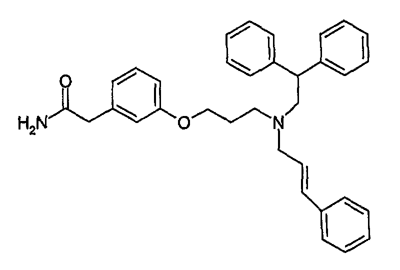

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.59 min; MS (ESP+) *m*/*e* 505 (MH⁺).

### Example 75: Ethyl 2-{[{3-[3-(2-amino-2-oxoethyl)phenoxy]propyl}(2,2-diphenylethyl)amino]methyl}cyclopropanecarboxylate

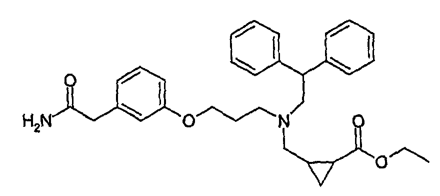

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.35 min; MS (ESP+) *m*/*e* 515 (MH⁺).

### Example 76: 2-(3-{3-[(2,2-diphenylethyl)(1-cyclohexen-1-ylmethyl)amino]propoxy} phenyl)acetamide

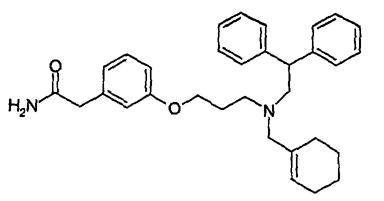

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.55 min; MS (ESP+) m/e483 (MH⁺).

### Example 77: 2-(3-{3-[(2,2-Diphenylethyl)(1H-benzimidazol-2-ylmethyl)amino] propoxy}phenyl)acetamide

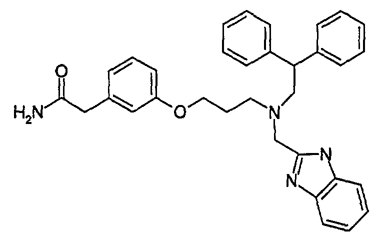

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.77 min; MS (ESP+) *m*/*e* 519 (MH⁺).

### Example 78: 2-(3-{3-[(2,2-Diphenylethyl)[(1,3-dimethyl-2-oxo-2,3-dihydro-1H-benzimidazol-5-yl)methyl]amino]propoxy}phenyl)acetamide

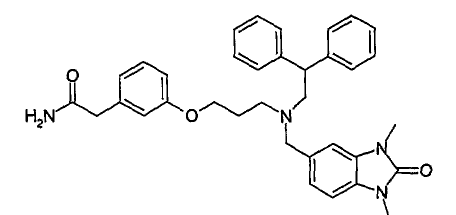

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.41 min; MS (ESP+) *m*/*e* 563 (MH⁺).

### Example 79: 2-(3-{3-[(2,2-Diphenylethyl)(2-pyrrolidinylmethyl)amino]propoxy} phenyl)acetamide

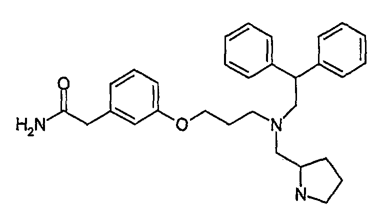

The title compound was prepared according to the methods of **Example 4**: HPLC (Waters symmetry shield, C8 3.0 micron, 2 x 50 mm, 85:15/H₂O:CH₃OH to 100% CH₃OH after 3 min, flow rate = 0.8 mL/min) t_{R}= 3.48 min; MS (ESP+) *m*/*e* 472 (MH⁺).

### Examples 80-283

The following compounds were synthesized according to the methods of Example 4.

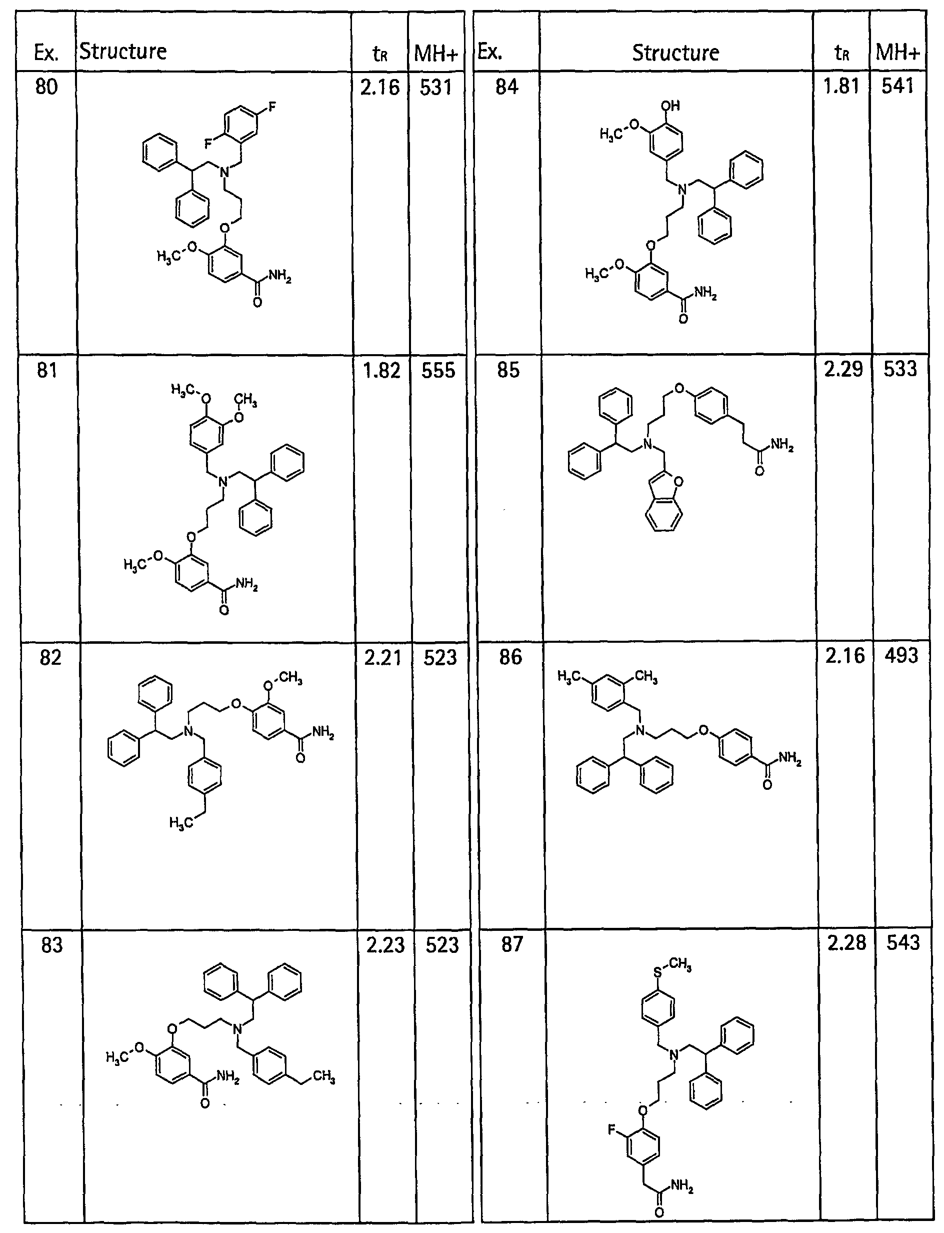

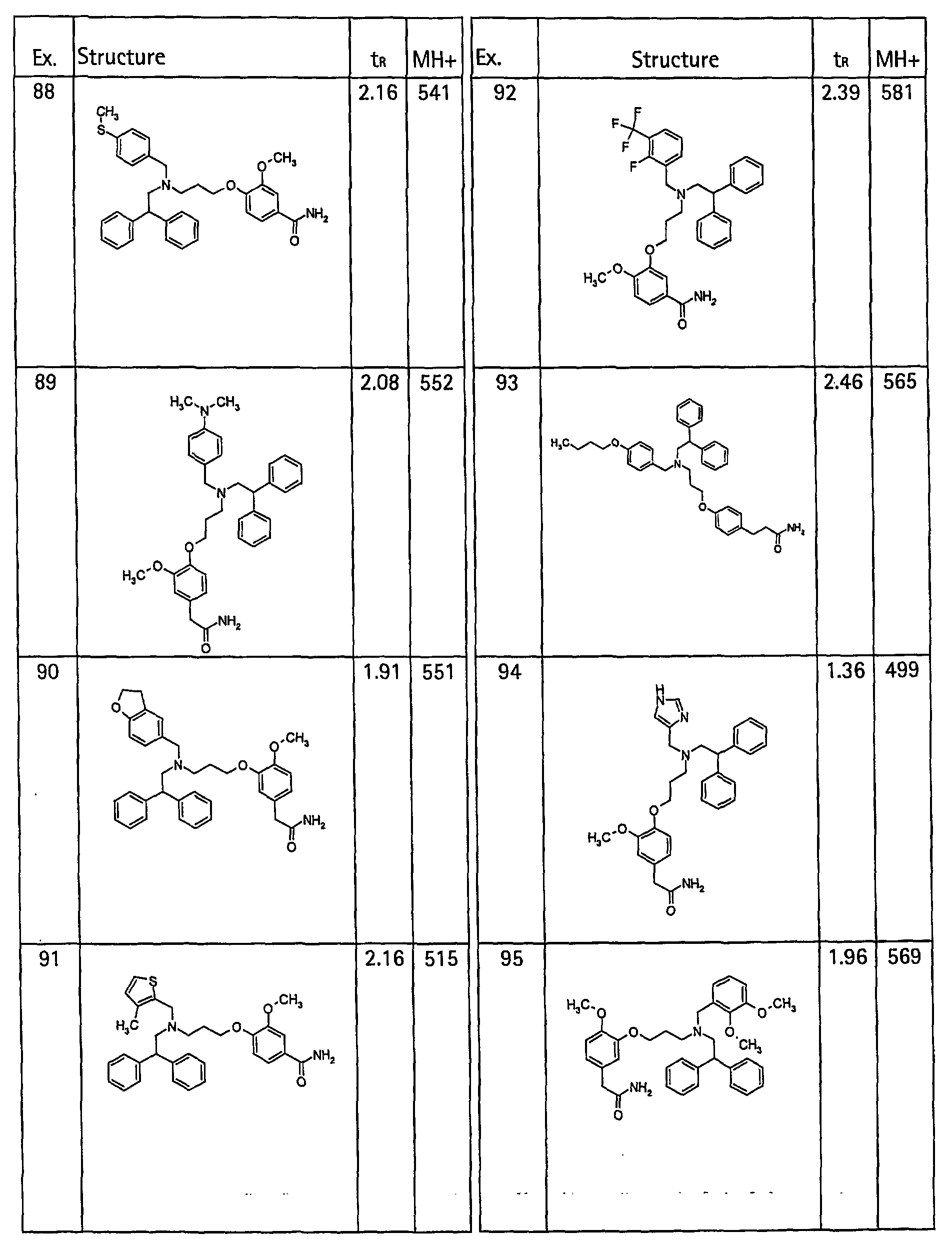

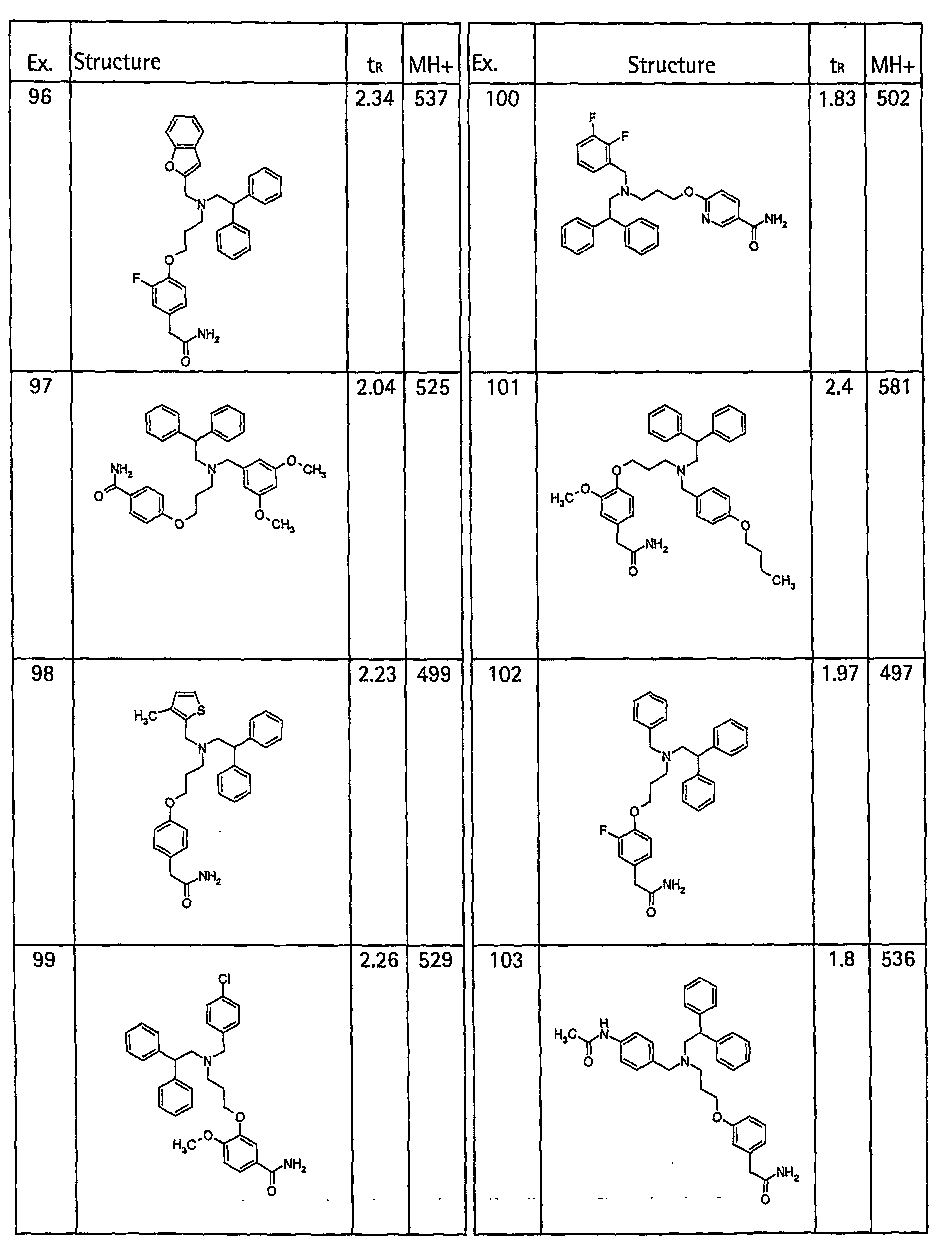

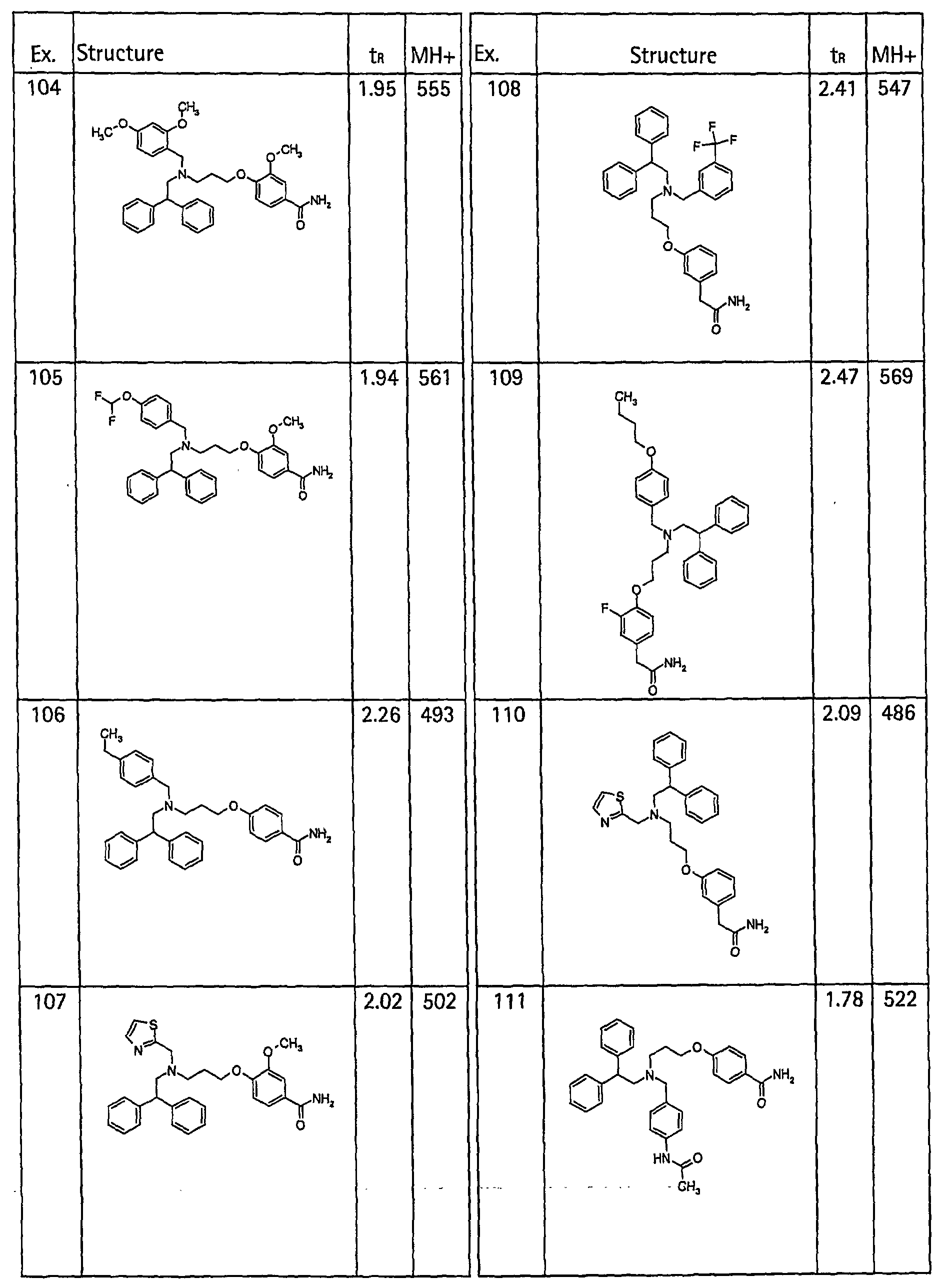

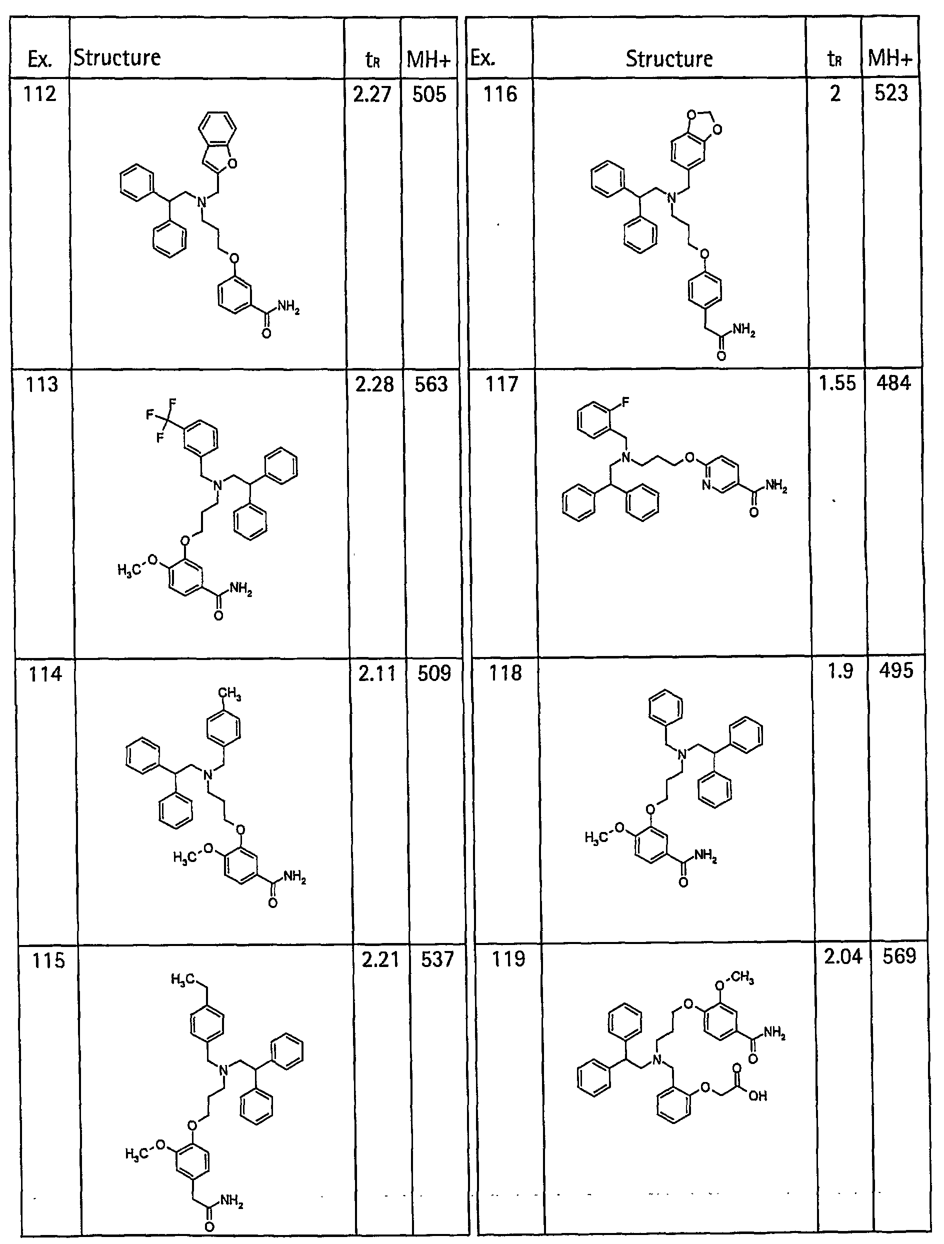

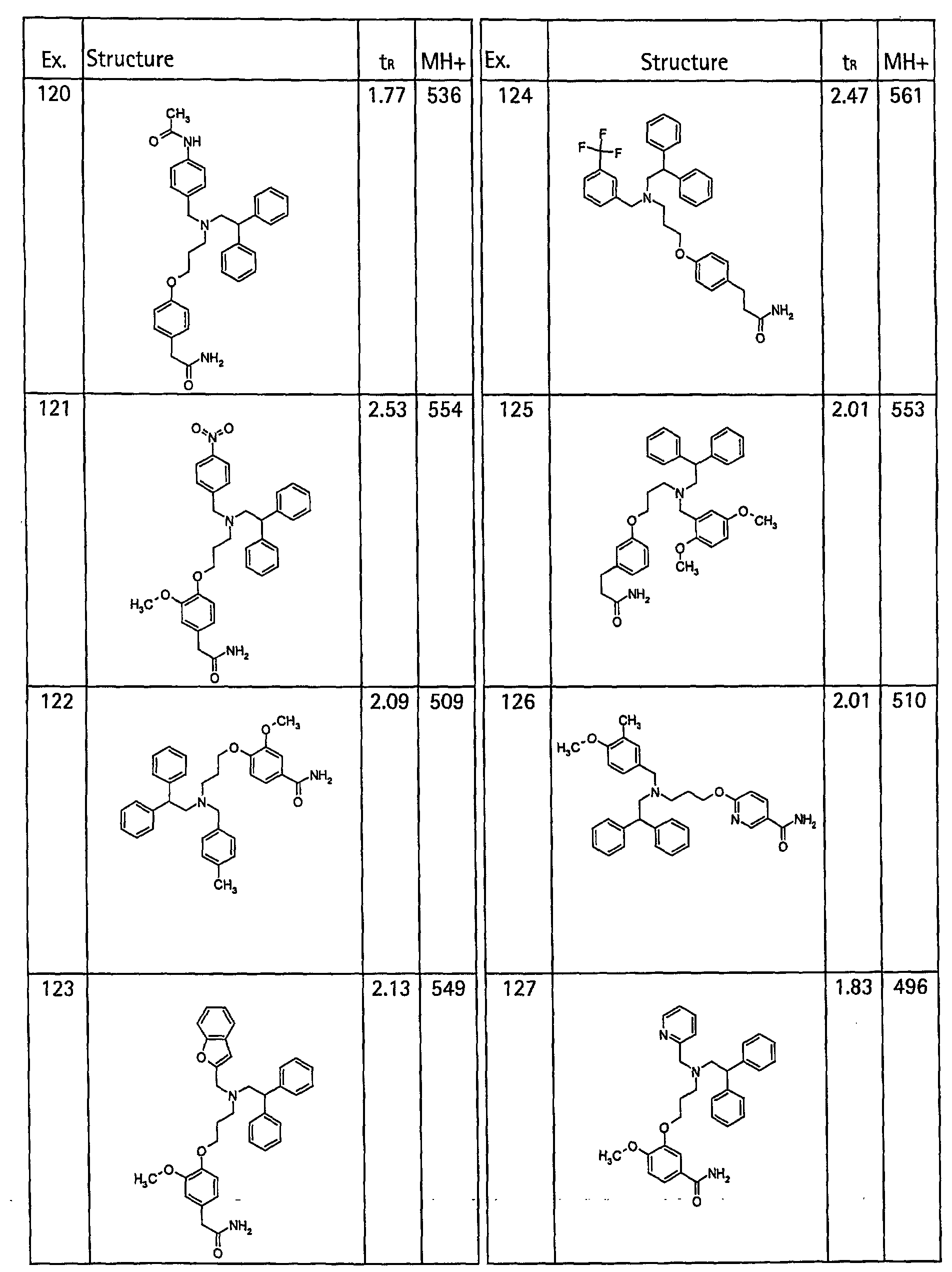

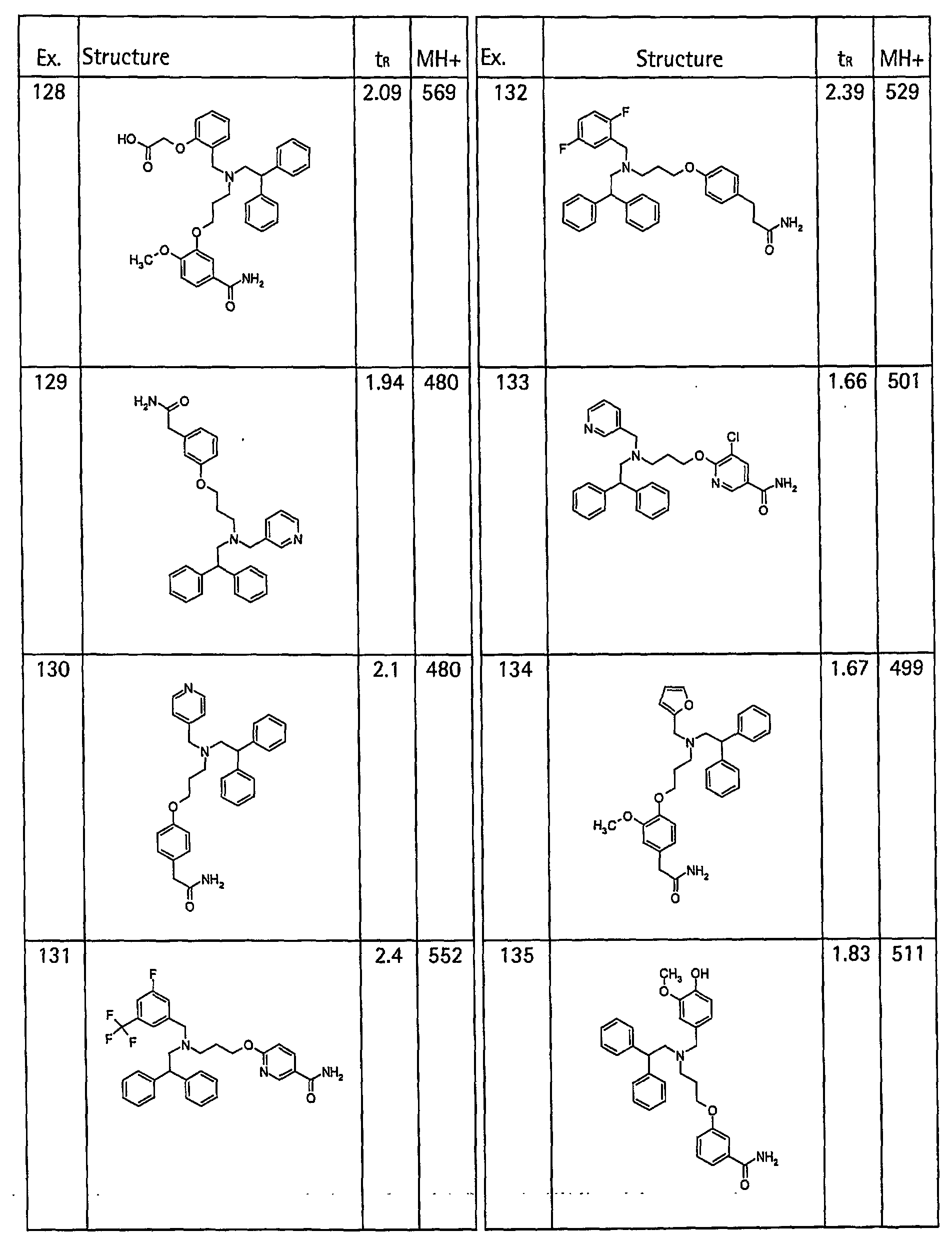

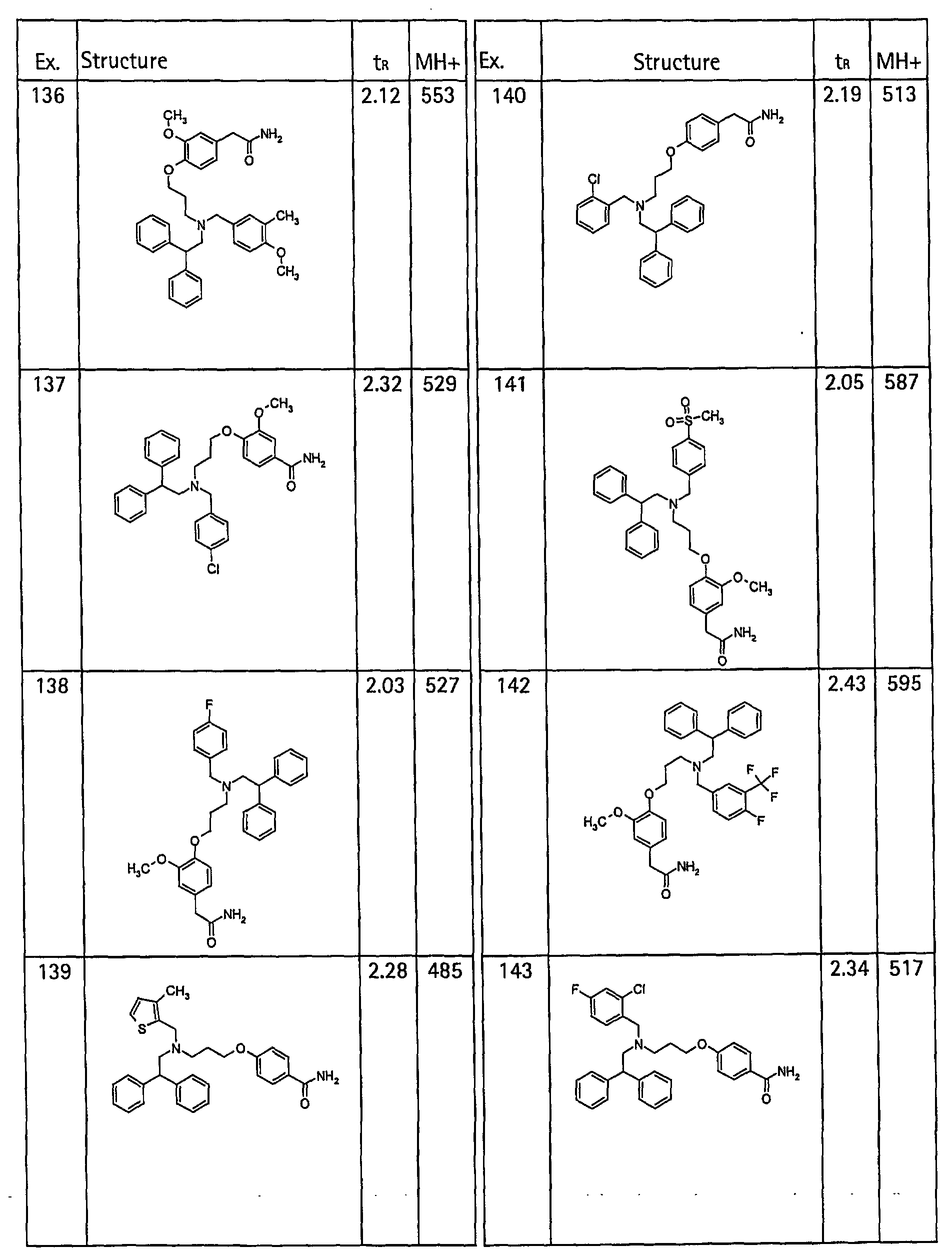

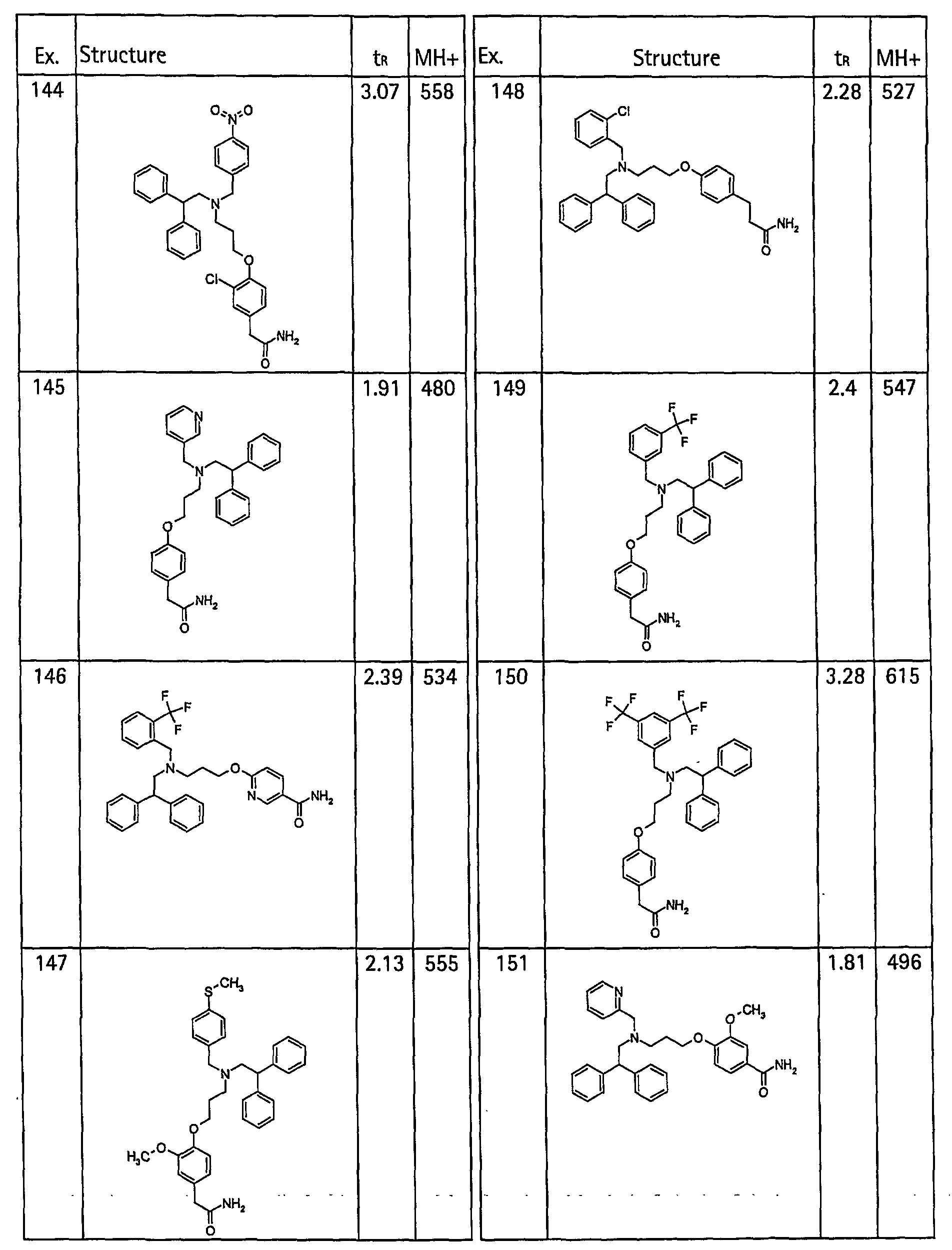

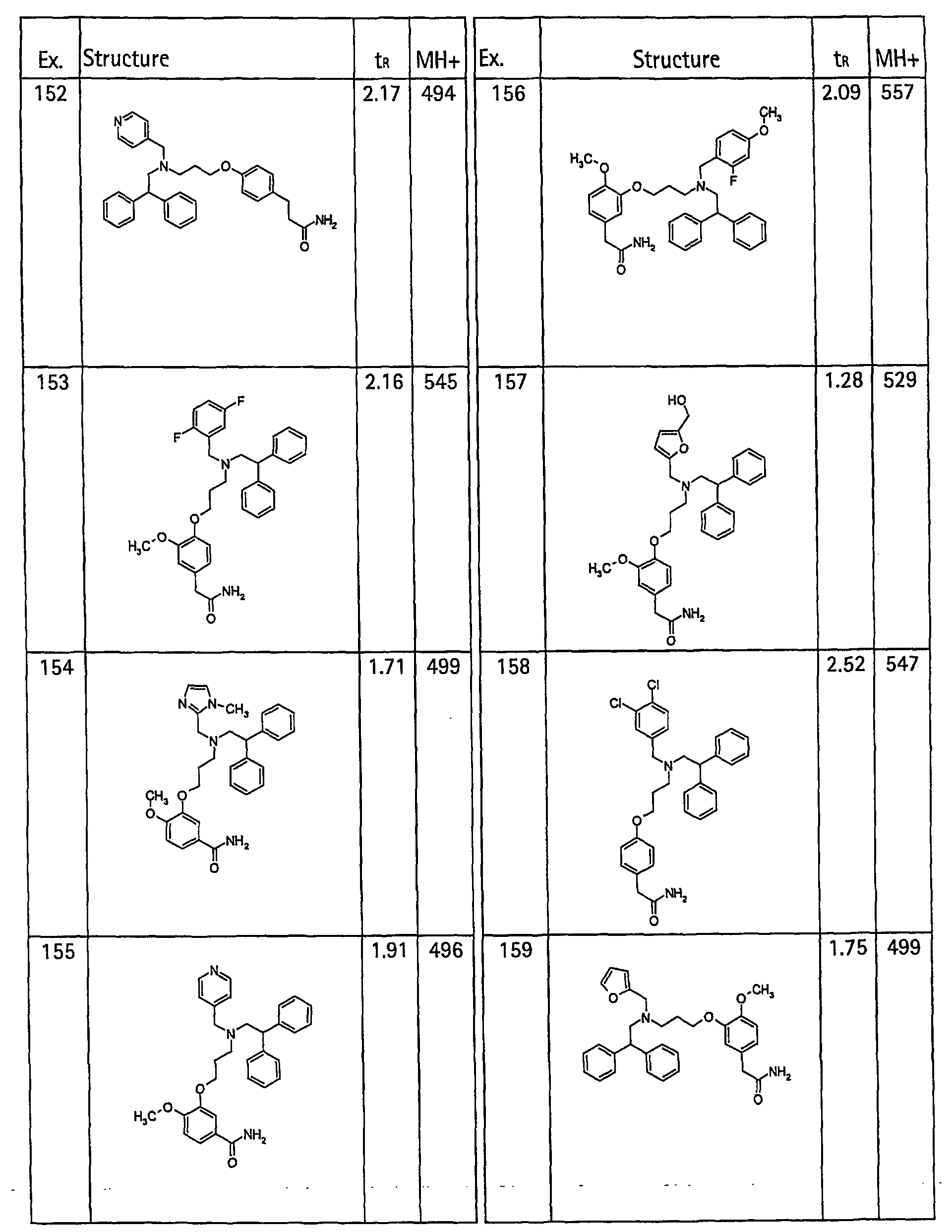

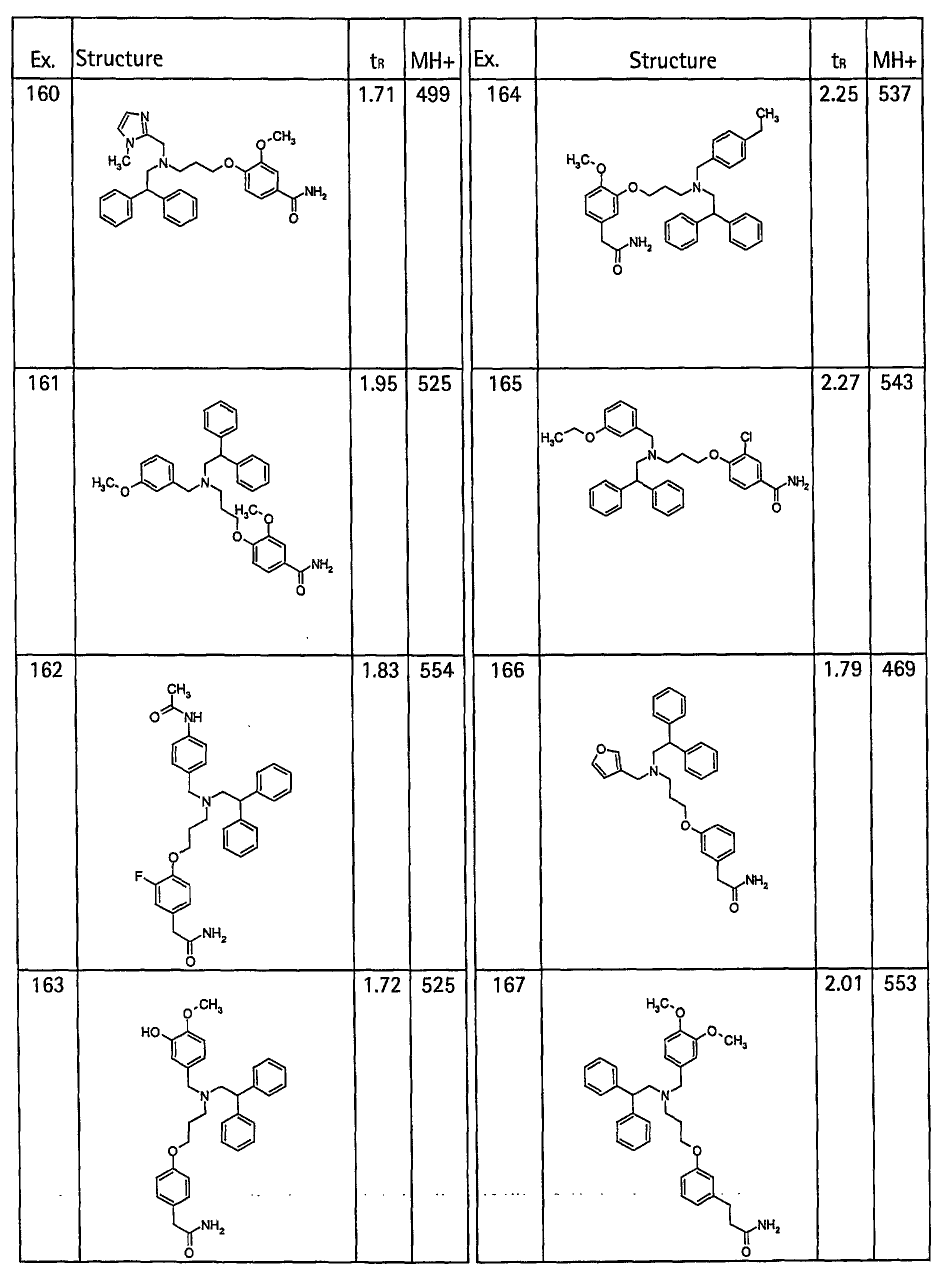

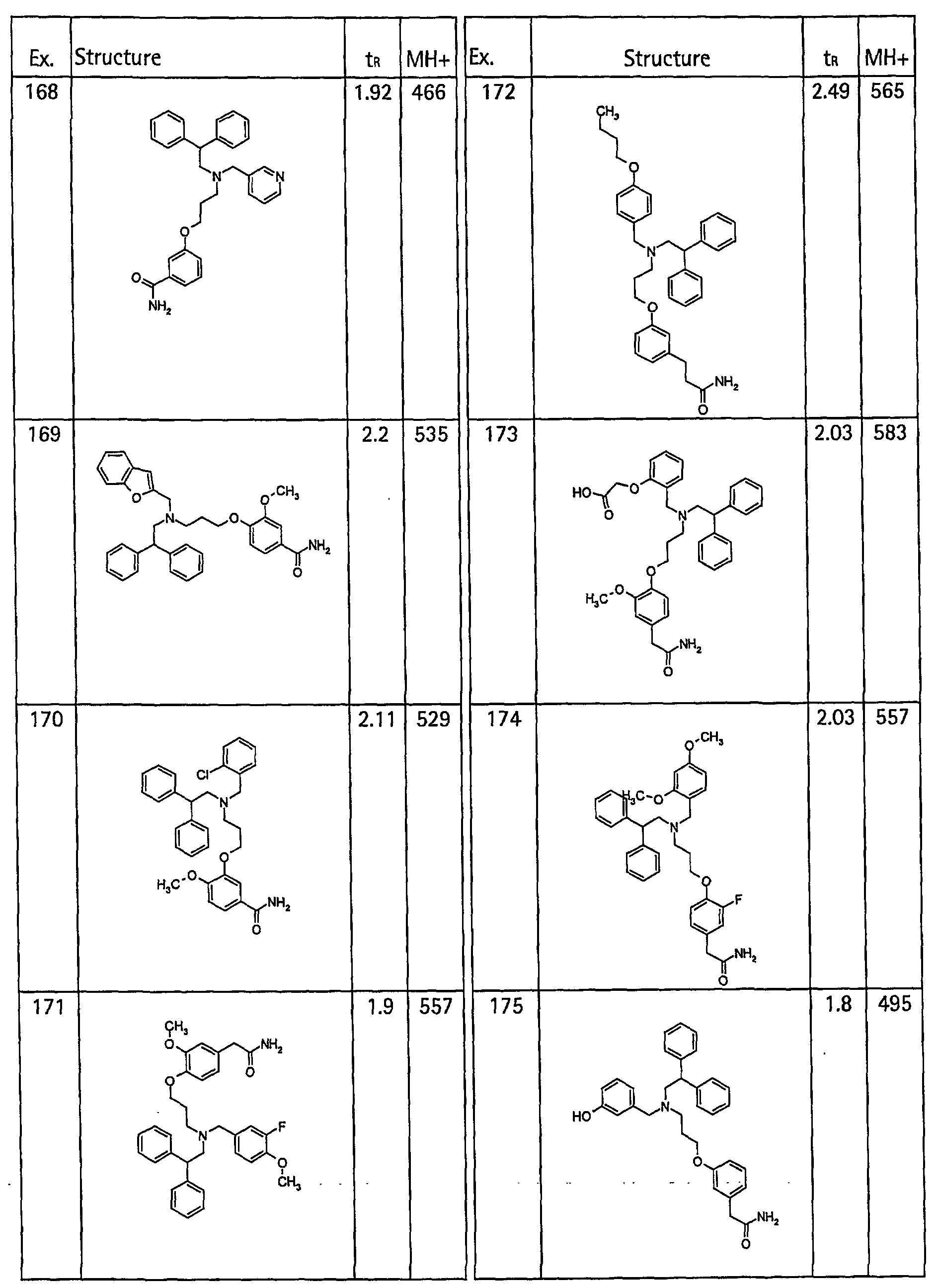

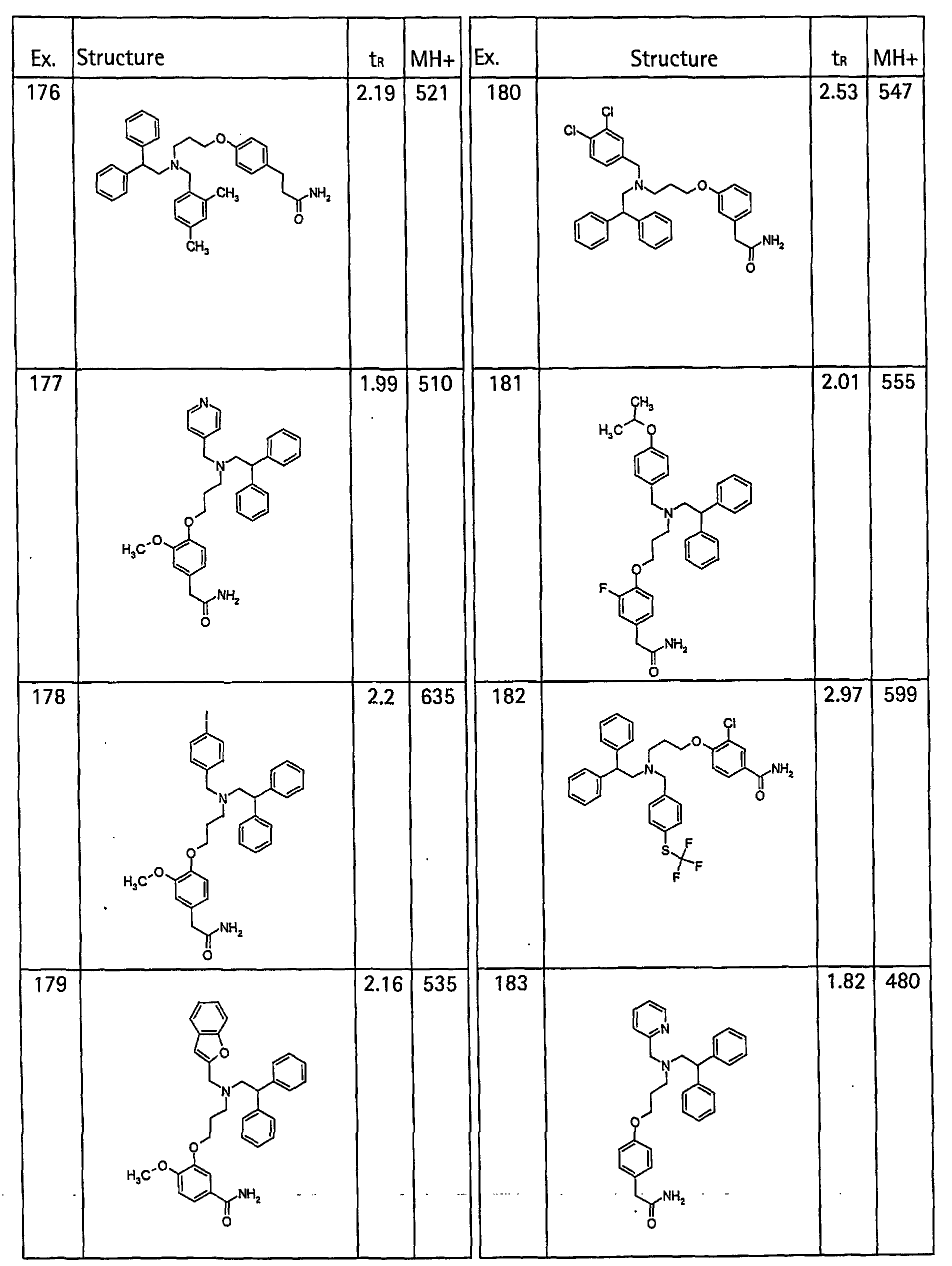

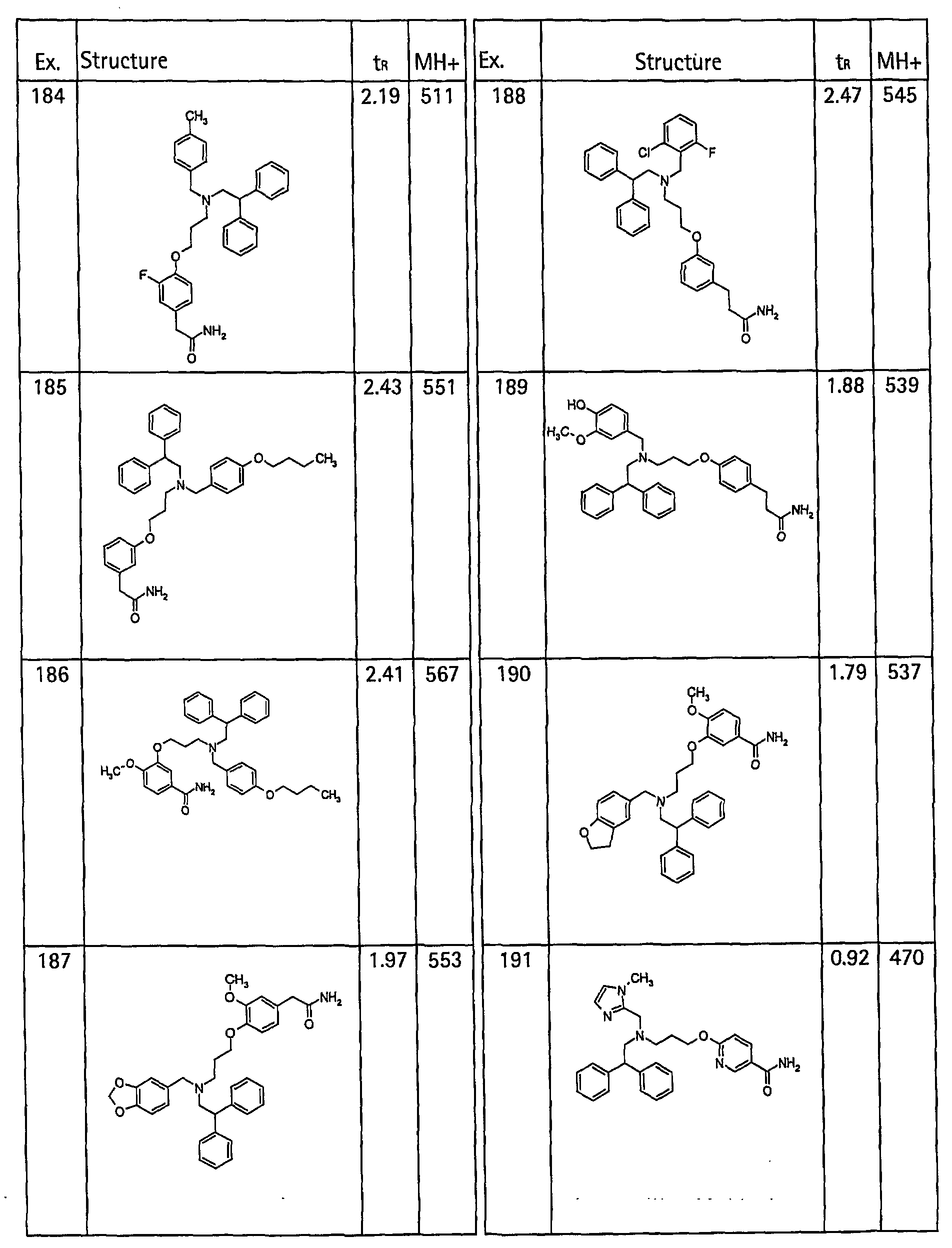

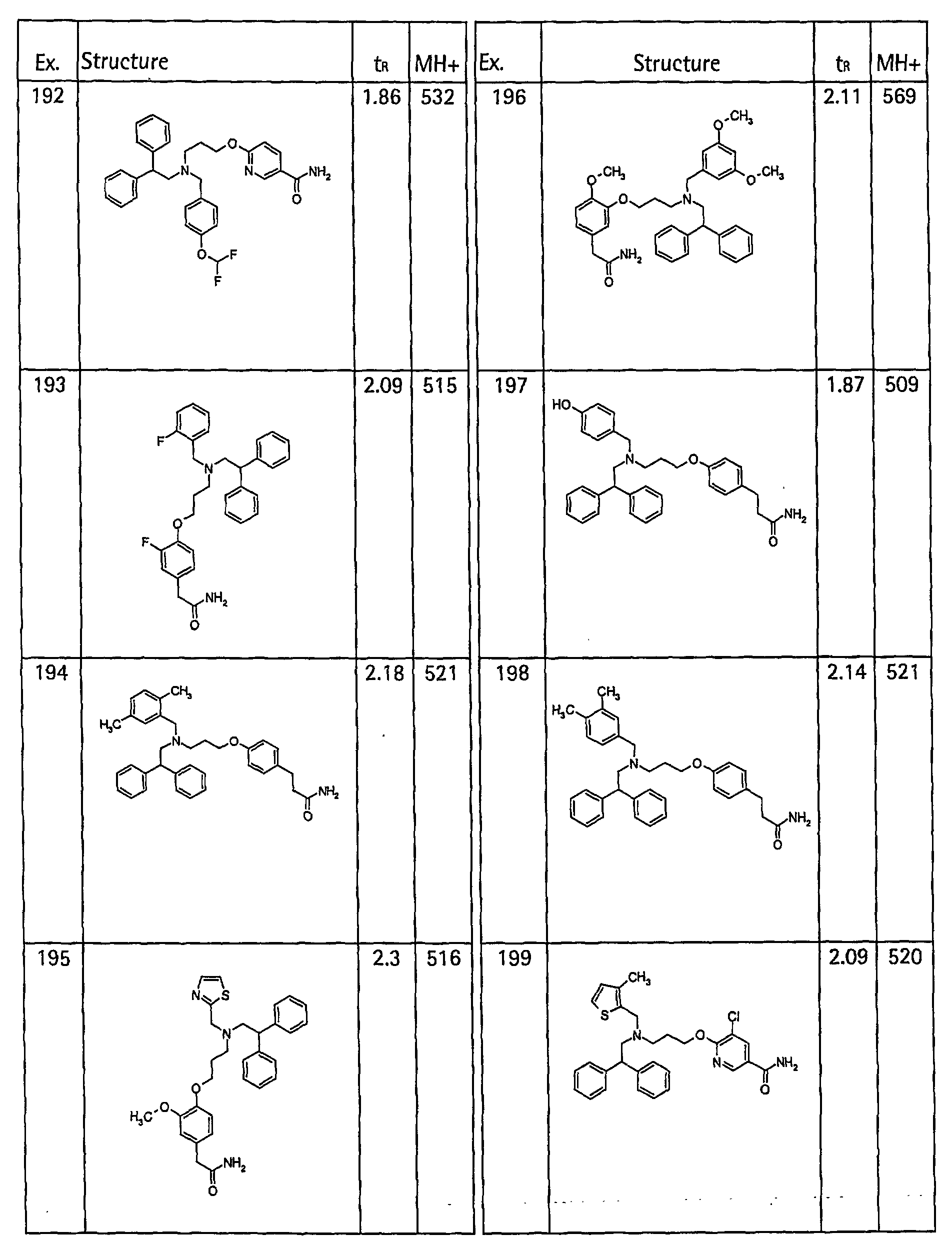

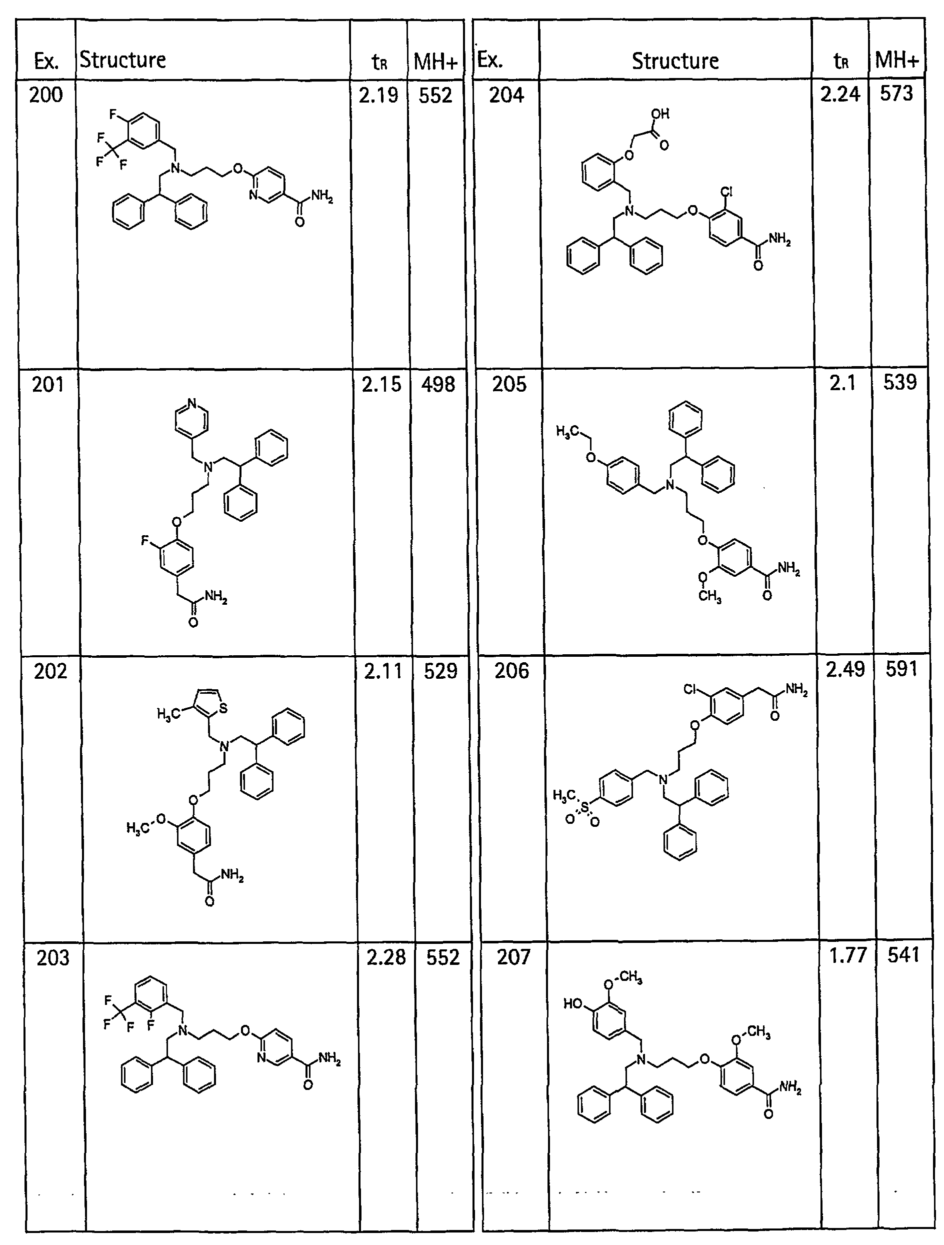

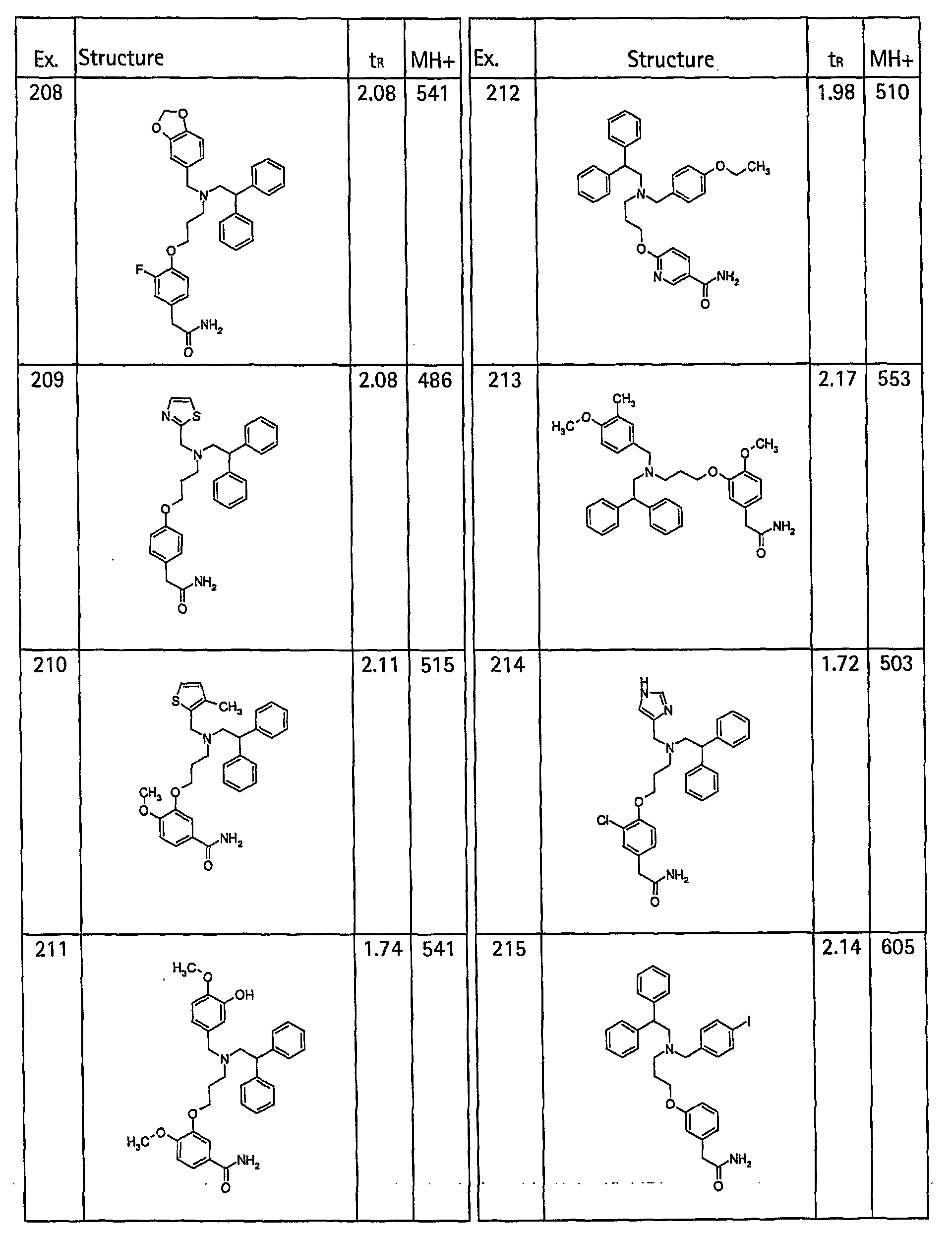

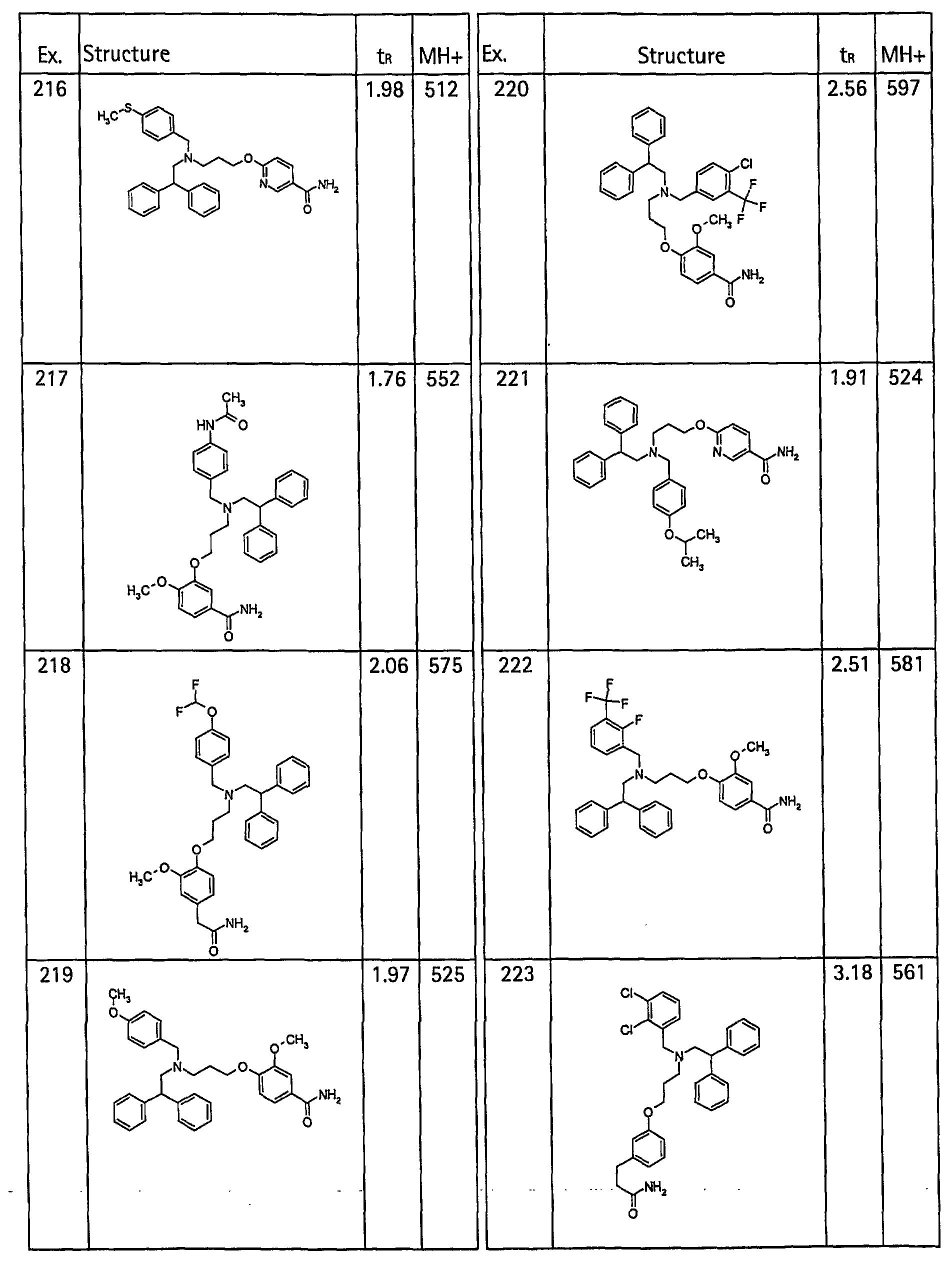

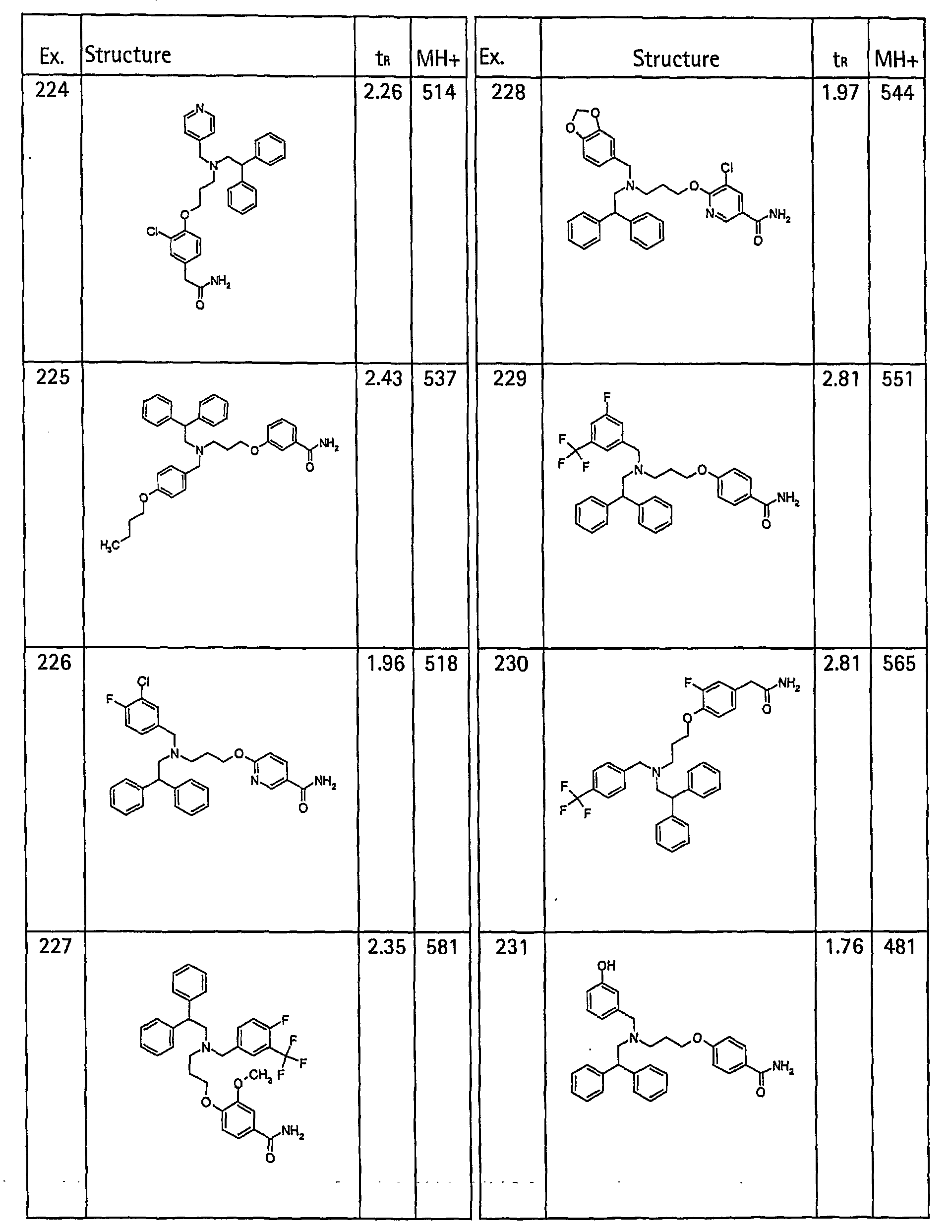

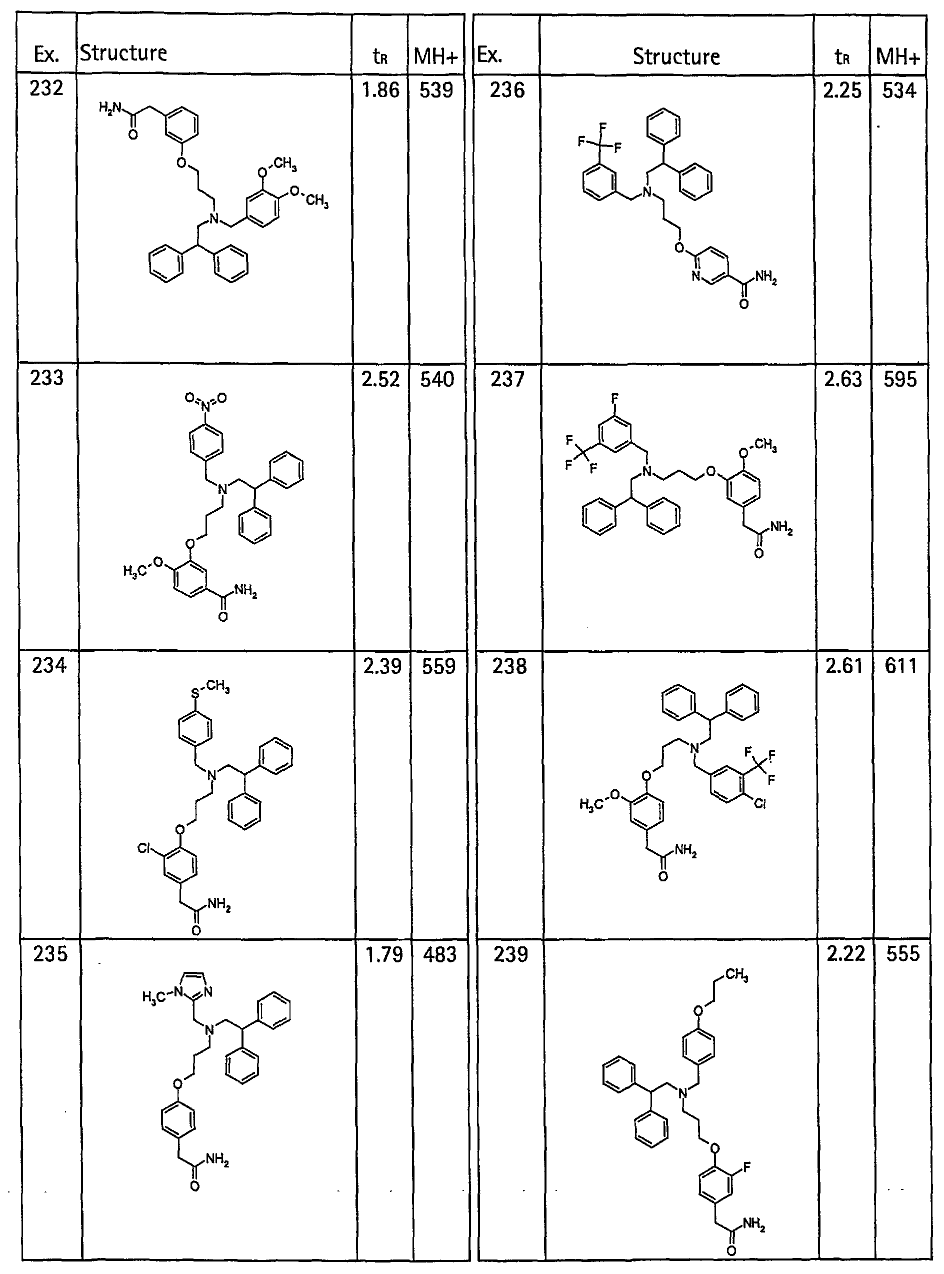

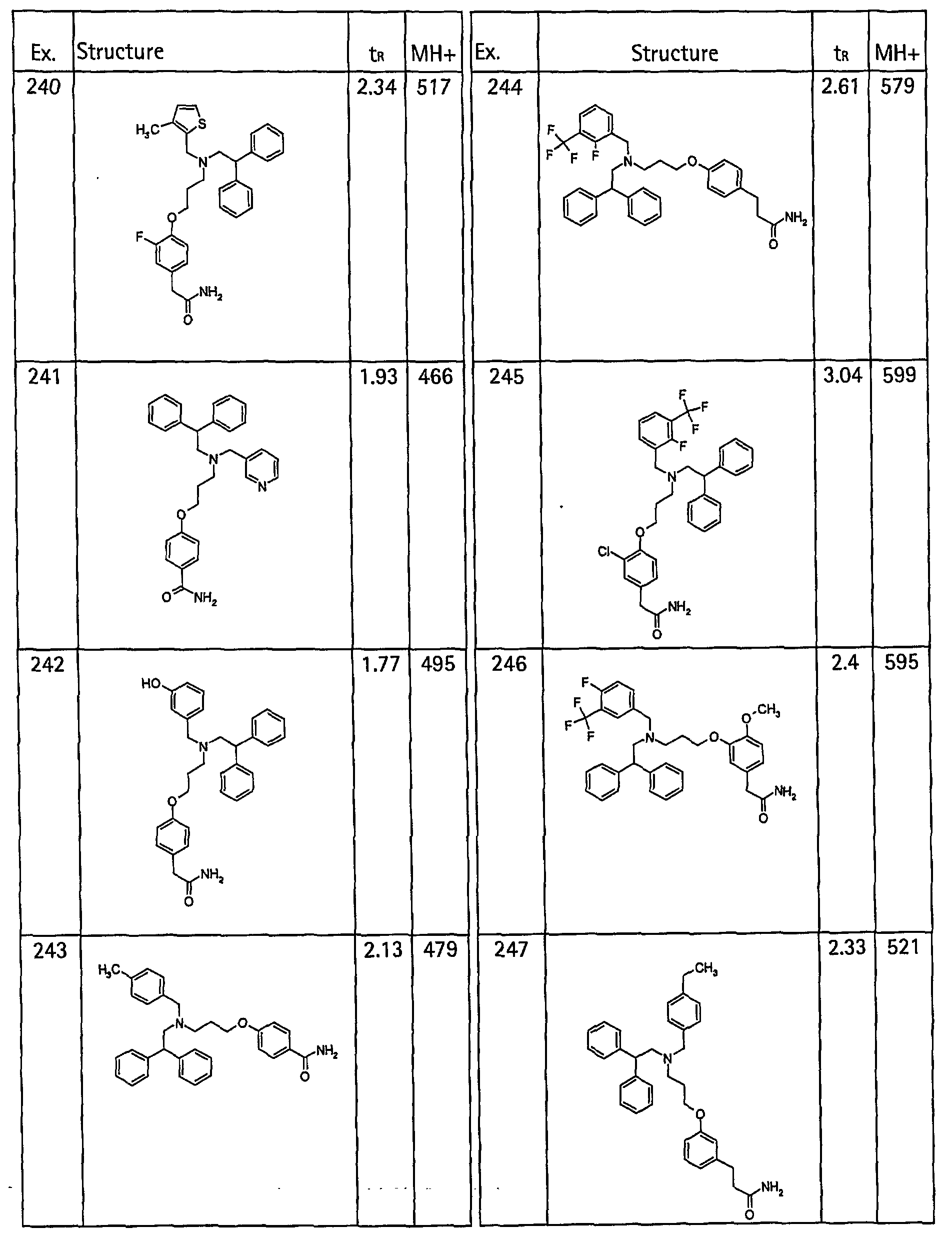

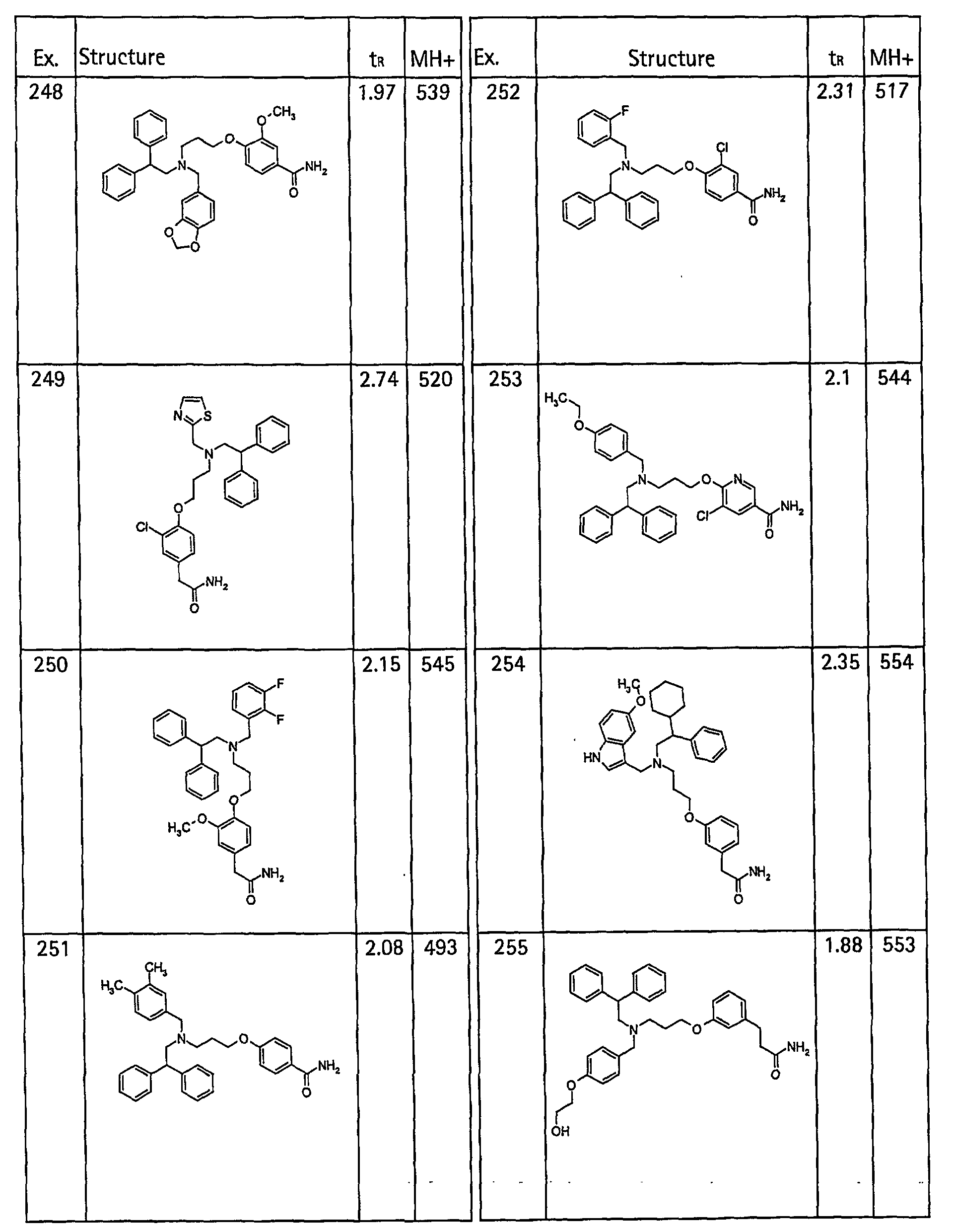

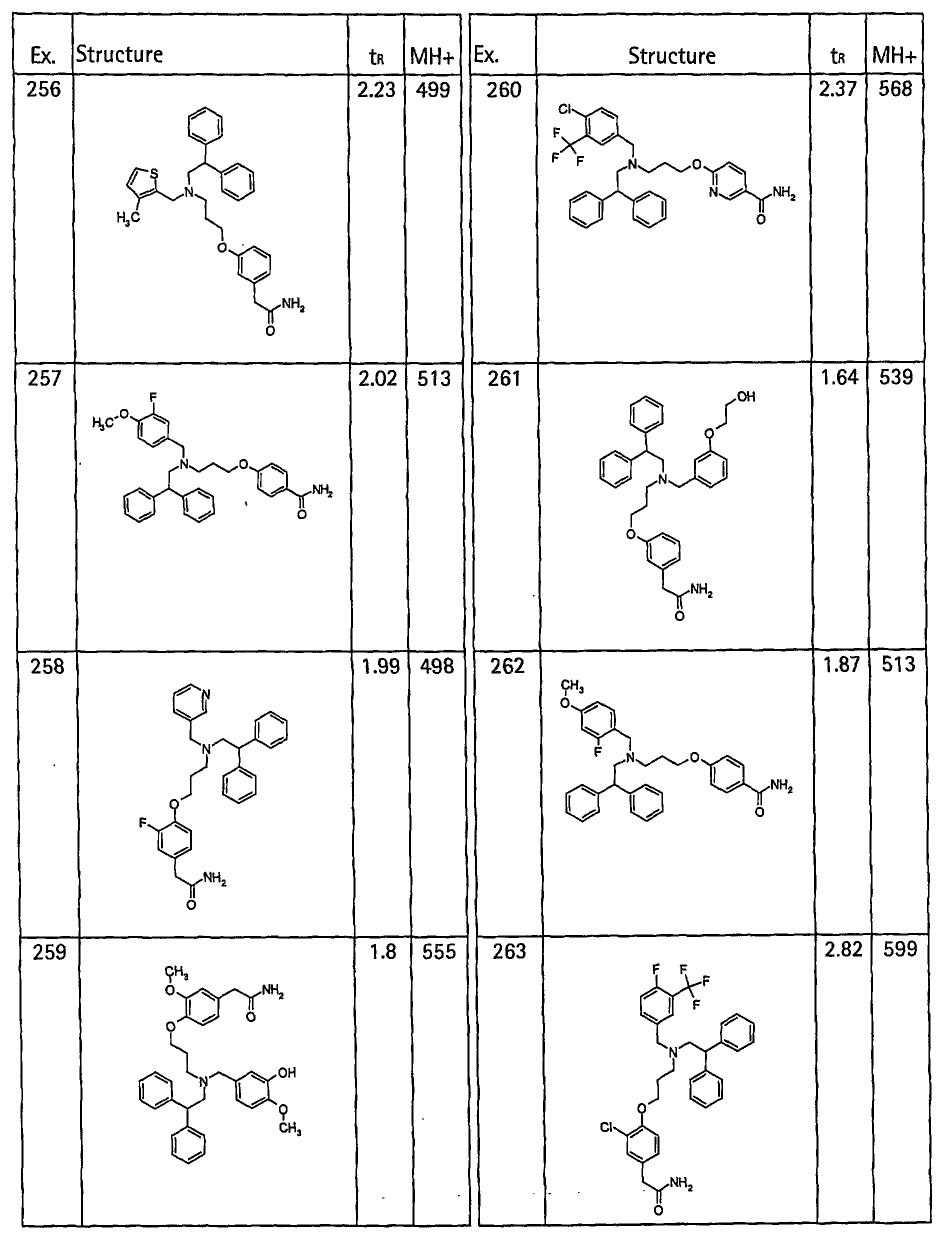

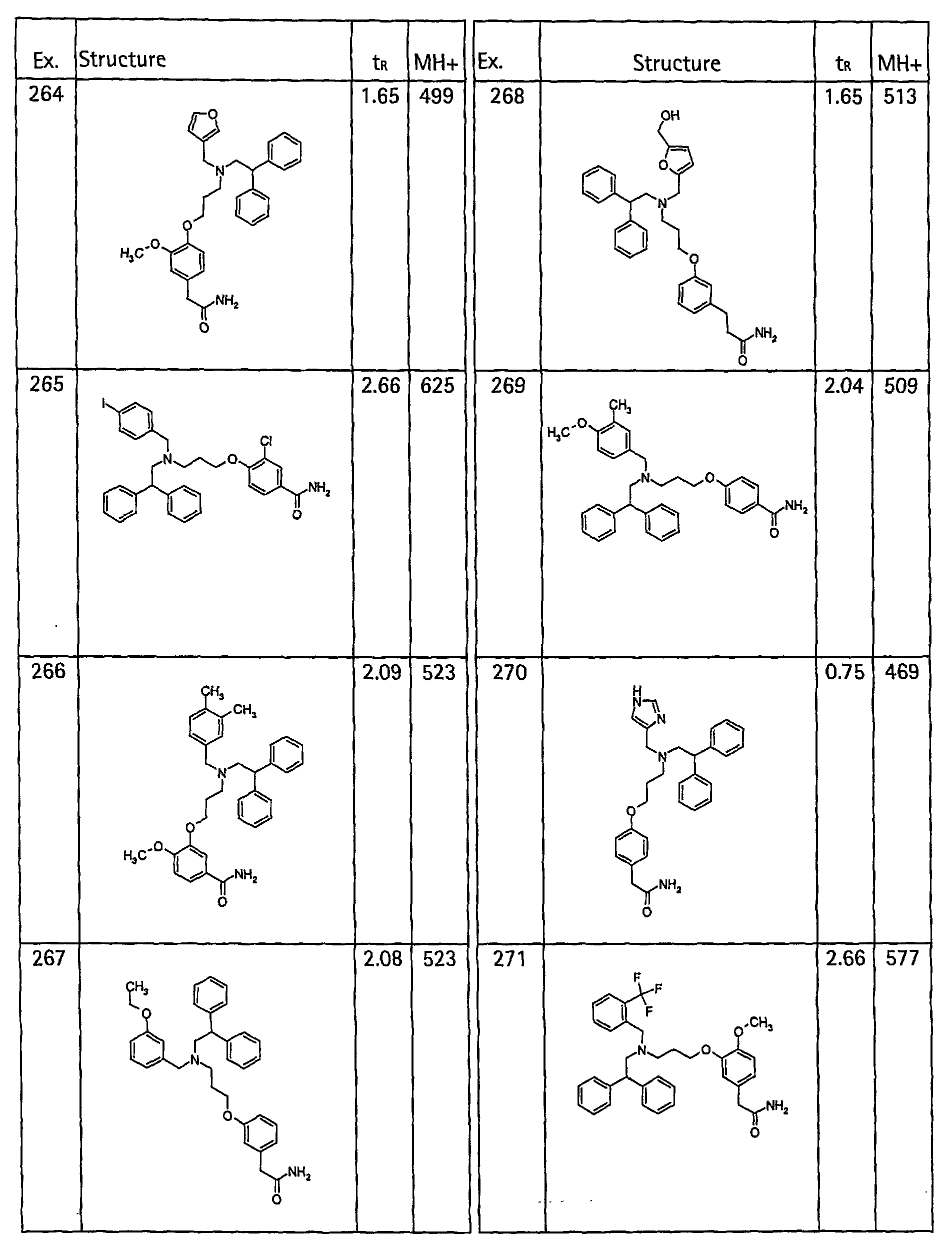

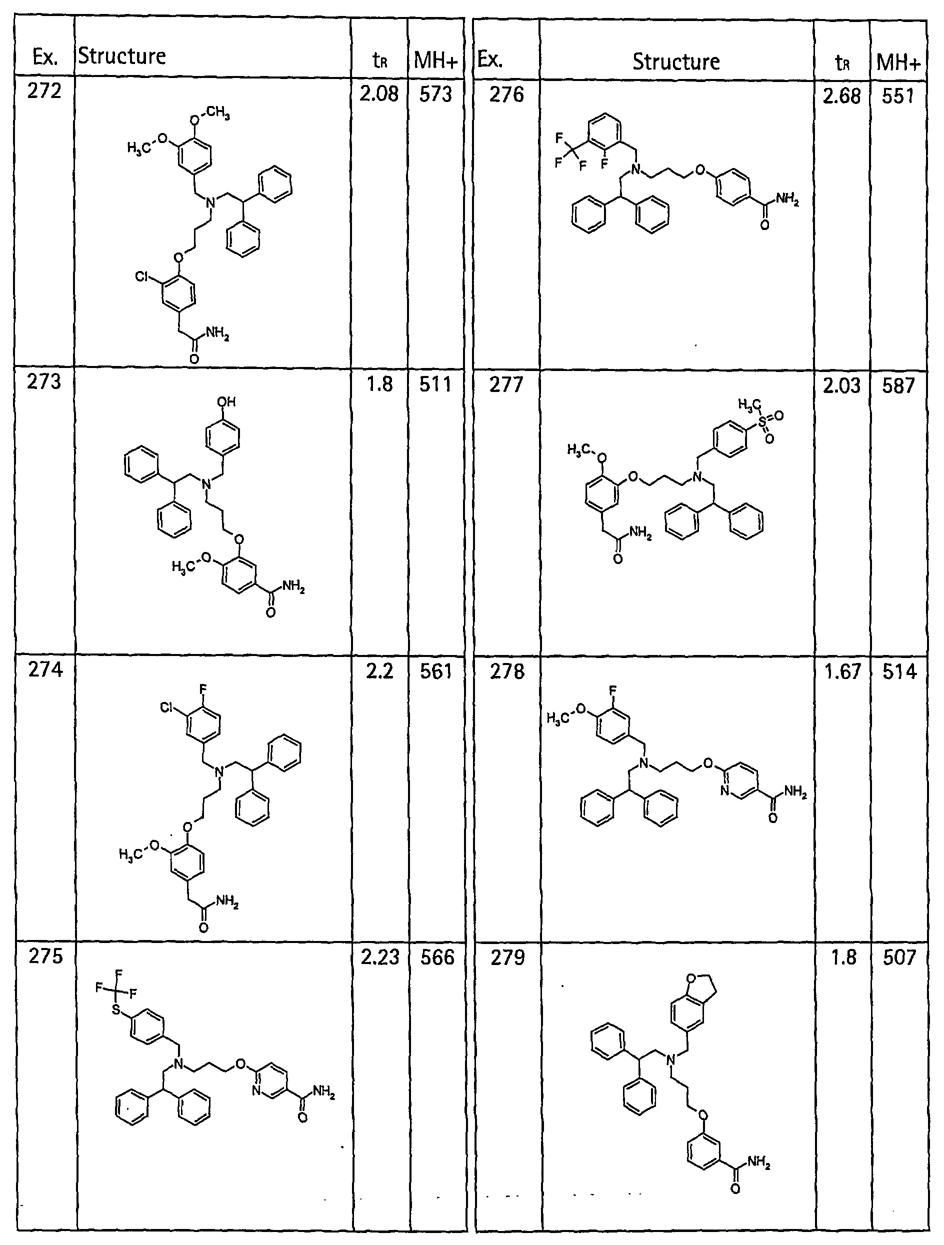

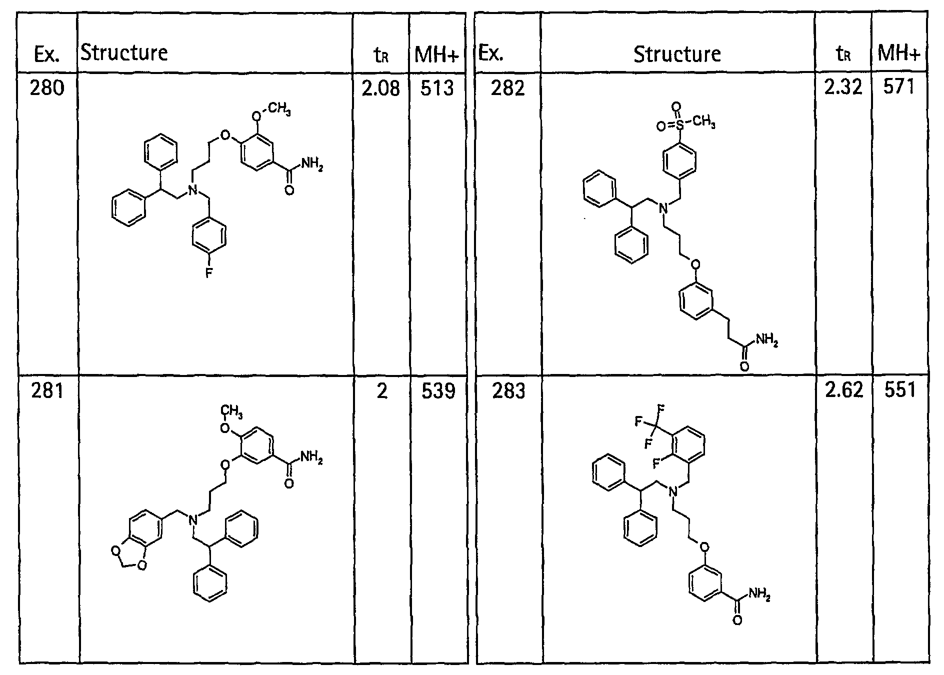

## Claims

1. A compound of formula (I): 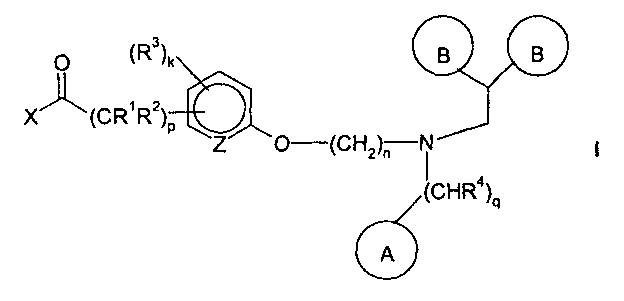 wherein:
X is OH or NH₂;
p is 0-6;
each R¹ and R² are the same or different and are each independently selected from the group consisting of H, C₁₋₈alkyl, C₁₋₈alkoxy and C₁₋₈thioalkyl;
Z is CH or N;
when Z is CH, k is 0-4;
when Z is N, k is 0-3;
each R³ is the same or different and is independently selected from the group consisting of halo and C₁₋₈alkoxy;
n is 3;
q is 0 or 1;
R⁴ is selected from the group consisting of H and C₁₋₈alkyl;
Ring A is selected from the group consisting of:
i) C₃₋₈cycloalkyl,
ii) aryl selected from the group consisting of: phenyl, 1-naphthyl or 2-naphthyl;
iii) 4-8 membered heterocycle selected from the group consisting of: a monocyclic saturated or unsaturated non-aromatic carbocyclic ring or fused bicyclic non-aromatic carbocyclic ring each of which may contain 1, 2 or 3 heteroatoms selected from N, O and S; and
iv) 5-6 membered heteroaryl selected from the group consisting of: an aromatic monocyclic heterocyclic ring or aromatic fused bicyclic ring each of which may contain 1, 2 or 3 heteroatoms selected from N, O and S;
wherein ring A may be independently substituted by one or more substituents selected from the group consisting of halo, -OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano, wherein a is 0, 1 or 2; R⁶ is selected from the group consisting of H, C₁₋₈alkyl, C₁₋₈alkoxy and C₂₋₈alkenyl; each R⁷ and R⁸ is the same or different and is independently selected from the group consisting of H, C₁₋₈alkyl, C₂₋₈alkenyl and C₃₋₈alkynyl; R⁹ is selected from the group consisting of H, C₁₋₈alkyl and -NR⁷R⁸; and R¹⁰ is C₁₋ ₈alkyl;
each ring B is the same or different and is independently selected from the group consisting of cyclohexyl and phenyl; and
pharmaceutically acceptable salts and solvates thereof.

2. The compound according to claim 1, wherein X is OH.

3. The compound according to any of claims 1-2, wherein p is 0 or 1.

4. The compound according to any of claims 1-2, wherein p is 1.

5. The compound according to any of claims 1-4, wherein R¹ and R² are each H.

6. The compound according to any of claims 1-5, wherein Z is CH.

7. The compound according to any of claims 1-6, wherein k is 0.

8. The compound according to any of claims 1-7, wherein q is 1.

9. The compound according to any of claims 1-8, wherein Ring A is phenyl optionally substituted from 1 to 5 times with a substituent selected from the group consisting of halo, C₁₋₈alkyl, C₁₋₈alkoxy, and S(O)ₐR⁶.

10. The compound according to any of claims 1-9, wherein Ring A is phenyl optionally substituted from 1 to 5 times with a substituent selected from the group consisting of F, Cl, -CF₃, -OCH₃, and -OCF₃.

11. The compound according to any of claims 1-10 wherein both Rings B are phenyl optionally substituted from 1 to 5 times with a substituent selected from the group consisting of halo, -OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, -R¹⁰COOR⁶, - OR¹⁰COOR⁶, -CONR⁷R⁸, -OC(O)R⁹, -R¹⁰NR⁷R⁸, -OR¹⁰NR⁷R⁸, nitro, and cyano.

12. The compound according to any of claims 1-10 wherein one Ring B is phenyl optionally substituted from 1 to 5 times with a substituent selected from the group consisting of halo, -OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano and the other Ring B is cyclohexyl optionally substituted from 1 to 10 times with a substituent selected from the group consisting of halo, -OH, C₁₋₈alkyl, C₂₋₈alkenyl, C₁₋₈alkoxy, C₂₋₈alkenyloxy, S(O)ₐR⁶, -NR⁷R⁸, -COR⁶, -COOR⁶, - R¹⁰COOR⁶, -OR¹⁰COOR⁶, -CONR⁷R⁸, -OC(O)R⁹, -R¹⁰NR⁷R⁸, -OR¹⁰NR⁷R⁸, nitro, and cyano.

13. A compound selected from the group consisting of:
2-(3-{3-[[2-chloro-3-(trifluoromethyl)benzyl](2,2-diphenylethyl)amino]propoxy} phenyl) acetamide,
2-(3-{3-[[2-chloro-3-(trifluoromethyl)benzyl](2,2-diphenylethyl)amino]propoxy}- phenyl)acetic acid,
(3-{2-[(2,2-diphenylethyl)-(4-methoxybenzyl)amino]propoxy}phenyl)acetamide,
(3-{2-[(2,2-diphenylethyl)-(4-methoxybenzyl)amino]propoxy}phenyl)acetic acid,
2-(3-{3-[(2,2-diphenylethyl)(2-fluoro-4-methoxybenzyl)amino]propoxy}phenyl) acetamide,
2-(3-{3-[(2,4-dimethoxybenzyl)(2,2-diphenylethyl)amino]propoxy}phenyl) acetamide,
2-[3-(3-{(2,2-diphenylethyl)[4-fluoro-2-(trifluoromethyl)benzyl]amino}propoxy) phenyl] acetamide,
2-(3-{3-[(2,3-dichlorobenzyl)(2,2-diphenylethyl)amino]propoxy}phenyl) acetamide,
2-[3-(3-{(2,2-diphenylethyl)[3-(trifluoromethoxy)benzyl]amino}propoxy)phenyl] acetamide,
2-(3-{3-[(2,2-diphenylethyl)(3-fluoro-4-methoxybenzyl)amino]propoxy}phenyl) acetamide,
2-(3-{3-[(2,5-dimethoxybenzyl)(2,2-diphenylethyl)amino]propoxylphenyl) acetamide,
2-[3-(3-{(2,2-diphenylethyl)[3-(trifluoromethyl)benzyl]amino}propoxy)phenyl] acetamide,
2-[3-(3-{(2,2-diphenylethyl)[2-fluoro-3-(trifluoromethyl)benzyl]amino}propoxy) phenyl] acetamide;
Ethyl 4-[{3-[3-(aminocarbonyl)phenoxy]propyl}(2,2-diphenylethyl)amino]-1-piperidinecarboxylate;
3-{3-[(1-Benzoyl-4-piperidinyl)-(2,2-diphenylethyl)amino]propoxy}benzamide;
3-{3-[(1-Acetyl-4-piperidinyl)(2,2-diphenylethyl)amino]propoxy}benzamide;
Benzyl 4-[{3-[3-(aminocarbonyl)phenoxy]propyl}(2,2-diphenylethyl)amino]-1-piperidinecarboxylate;
3-(3-{(2,2-Diphenylethyl)[1-(2-phenylethyl)-4-piperidinyl]amino}propoxy)benzamide;
Ethyl 4-[{3-[3-(aminocarbonyl)phenoxy]propyl}(2-cyclohexyl-2-phenylethyl)amino]-1-piperidinecarboxylate;
3-{3-[(1-Benzoyl-4-piperidinyl)(2-cyclohexyl-2-phenylethyl)amino]propoxy}benzamide;
3-{3-[(1-Acetyl-4-piperidinyl)(2-cyclohexyl-2-phenylethyl)amino]propoxy}benzamide;
*tert*-Butyl 4-[{3-[3-(aminocarbonyl)phenoxy]propyl}(2-cyclohexyl-2-phenylethyl)amino]-1-piperidinecarboxylate;
Benzyl 4-[{3-[3-(aminocarbonyl)phenoxy]propyl}(2-cyclohexyl-2-phenylethyl)amino]-1-piperidinecarboxylate;
3-{3-[(1-Benzyl-4-piperidinyl)(2-cyclohexyl-2-phenylethyl)amino]propoxy}benzamide;
Ethyl 4-[{3-[3-(2-amino-2-oxoethyl)phenoxy]propyl}(2,2-diphenylethyl)amino]-1-piperidinecarboxylate;
2-(3-{3-[(1-Benzoyl-4-piperidinyl)(2,2-diphenylethyl)amino]propoxy}phenyl)-acetamide;
2-(3-{3-[(1-Acetyl-4-piperidinyl)(2,2-diphenylethyl)amino]propoxy}phenyl)-acetamide;
*tert*-Butyl 4-({3-[3-(2-amino-2-oxoethyl)phenoxy]propyl}(2,2-diphenylethyl)amino]-1-piperidinecarboxylate;
Benzyl 4-[{3-[3-(2-amino-2-oxoethyl)phenoxy]propyl}(2,2-diphenylethyl)amino]-1-piperidinecarboxylate;
2-[3-(3-{(2,2-Diphenylethyl)[1-(2-phenylethyl)-4-piperidinyl]amino}propoxy)phenyl]-acetamide;
2-(3-{3-[(1-Benzoyl-4-piperidinyl)(2-cyclohexyl-2-phenylethyl)amino]propoxy}-phenyl)acetamide;
2-(3-{3-[(1-Acetyl-4-piperidinyl)(2-cyclohexyl-2-phenylethyl)amino]propoxy}phenyl)acetamide;
Benzyl-4-[{3-[3-(2-amino-2-oxoethyl)phenoxy]propyl}(2-cyclohexyl-2-phenylethyl)amino]-1-piperidinecarboxylate;
3-{3-[(3-Cyanobenzyl)(2,2-diphenylethyl)amino]propoxy}benzamide;
3-{3-[Cyclohexyl(2,2-diphenylethyl)amino]propoxy}benzamide;
4-[{3-[3-(Aminocarbonyl)phenoxy]propyl}(2,2-diphenylethyl)amino]-1-piperidinecarboxamide;
3-{3-[(1,3-Benzodioxol-4-ylmethyl)(2,2-diphenylethyl)amino]propoxy}benzamide;
3-{3-[(3,4-Dimethoxybenzyl)(2,2-diphenylethyl)amino]propoxy}benzamide;
3-{3-[(4-Cyanobenzyl)(2-cyclohexyl-2-phenylethyl)amino]propoxy}benzamide;
3-{3-[(4-Cyanobenzyl)(2-cyclohexyl-2-phenylethyl)amino]propoxy}benzamide;
2-(3-{3-[Cyclohexyl(2,2-diphenylethyl)amino]propoxy}phenyl)acetamide;
2-(3-{3-[(3,4-Dimethoxybenzyl)(2,2-diphenylethyl)amino]propoxy}phenyl)acetamide;
3-{3-[(2-Cyclohexyl-2-phenylethyl)(3,4-dimethoxybenzyl)amino]propoxy}benzamide;
3-{3-[(2,6-Dichlorobenzyl)(2,2-diphenylethyl)amino]propoxy}benzamide;
3-{[{3-[3-(Aminocarbonyl)phenoxy]propyl}(2,2-diphenylethyl)amino]methyl}benzoic acid;
4-{[{3-[3-(Aminocarbonyl)phenoxy]propyl}(2,2-diphenylethyl)amino]methyl}benzoic acid;
3-(3-{(2,2-Diphenylethyl)[(5-methoxy-1*H*-indol-3-yl)methyl]amino}propoxy)-benzamide;
3-{3-[(2,2-Diphenylethyl)(4-methoxybenzyl)amino]propoxy}benzamide;
3-{3-[[(1-Acetyl-1*H*-indol-3-yl)methyl](2,2-diphenylethyl)amino]propoxy}benzamide;
Methyl 4-{[{3-[3-(aminocarbonyl)phenoxy]propyl}(2,2-diphenylethyl)amino]methyl}-benzoate;
3-{3-[(2,3-Dihydro-1,4-benzodioxin-6-ylmethyl)(2,2-diphenylethyl)amino]propoxy}-benzamide;
3-{3-[(2,2-Diphenylethyl)(4-pyridinylmethyl)amino]propoxy}benzamide;
2-(3-{3-[(2-Cyclohexyl-2-phenylethyl)(3,4-difluorobenzyl)amino]propoxy}phenyl) acetamide;
2-(3-{3-(2,2-Diphenylethyl)[[(6-chloro-1,3-benzodioxol-5-yl)methyl]amino]propoxy}-phenyl)acetamide;
2-(3-{3-[(2,2-Diphenylethyl)(cyclohexylmethyl)amino]propoxy}phenyl) acetamide;
2-(3-{3-[(2,2-Diphenylethyl)(bicyclo[2.2.1]hept-5-en-2-ylmethyl)amino]propoxy}-phenyl) acetamide;
2-(3-{3-[(2,2-diphenylethyl)(2,4-dimethoxy-5-pyrimidinyl) methyl)amino]propoxy} phenyl) acetamide;
2-(3-{3-[(2,2-Diphenylethyl(5-isopropyl-3-methyl-4-isoxazolyl)methyl)amino]-propoxy}phenyl) acetamide;
2-(3-{3-[(2,2-Diphenylethyl)( 3,4-dihydro-2*H*-pyran-2-ylmethyl)amino]propoxy}-phenyl) acetamide;
2-(3-{3-[(2,2-Diphenylethyl)( 4-chloro-1*H*-pyrazol-3-yl)methyl)amino]propoxy}-phenyl) acetamide;
2-(3-{3-[(2,2-Diphenylethyl)( )[(7-methoxy-1,3-benzodioxol-5-yl)methyl)amino]-propoxy}phenyl) acetamide;
2-(3-{3-[(2,2-Diphenylethyl-(2,6,6-trimethyl-1-cyclohexen-1-yl)ethyl)amino]-propoxy}phenyl) acetamide;
2-(3-{3-[(2,2-Diphenylethyl)( 3-cyclohexen-1-ylmethyl)amino]propoxy}phenyl) acetamide;
2-[3-(3-{(2,2-Diphenylethyl)[(2*E*)-3-phenyl-2-propenyl]amino}propoxy) phenyl]acetamide;
Ethyl 2-{[{3-[3-(2-amino-2-oxoethyl)phenoxy]propyl}(2,2-diphenylethyl)amino]-methyl}cyclopropanecarboxylate;
2-(3-{3-[(2,2-diphenylethyl)( 1-cyclohexen-1-ylmethyl)amino]propoxy} phenyl) acetamide;
2-(3-{3-[(2,2-Diphenylethyl)( 1*H*-benzimidazol-2-ylmethyl)amino]propoxy}phenyl) acetamide;
2-(3-{3-[(2,2-Diphenylethyl)[(1,3-dimethyl-2-oxo-2,3-dihydro-1*H*-benzimidazol-5-yl)methyl]amino]propoxy}phenyl) acetamide; and
2-(3-{3-[(2,2-Diphenylethyl)(2-pyrrolidinylmethyl)amino]propoxy}phenyl) acetamide;
and pharmaceutically acceptable salts and solvates thereof.

14. 2-(3-{3-[[2-Chloro-3-(trifluoromethyl)benzyl](2,2-diphenylethyl)amino]propoxy}phenyl)acetic acid and pharmaceutically acceptable salts and solvates thereof.

15. A pharmaceutical composition comprising a compound according to any of claims 1-13 and pharmaceutically acceptable carrier or diluent.

16. Use of a compound according to any of claims 1-13 for the preparation of a medicament for the prevention or treatment of cardiovascular disease.

17. Use of a compound according to claim 16 for the preparation of a medicament for the prevention or treatment of atherosclerosis.

18. Use of a compound according to any of claims 1-13 for the preparation of a medicament for increasing reverse cholesterol transport.

19. Use of a compound according to any of claims 1-13 for the preparation of a medicament for inhibiting cholesterol absorption.

20. A compound according to any of claims 1-13 for use in therapy.

## Patentansprüche

1. Verbindungen der Formel (I): 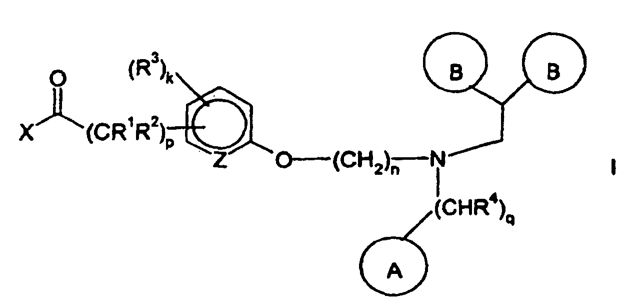 worin:
X OH oder NH₂ ist;
p 0-6 ist;
R¹ und R² jeweils gleich oder verschieden sind und jeweils unabhängig aus der Gruppe ausgewählt sind, die aus H, C₁₋₈-Alkyl, C₁₋₈-Alkoxy und C₁₋₈-Thioalkyl besteht;
Z CH oder N ist;
wenn Z CH ist, k 0-4 ist;
wenn Z N ist, k 0-3 ist;
R³ jeweils gleich oder verschieden ist und unabhängig aus der Gruppe ausgewählt ist, die aus Halogen und C₁₋₈-Alkoxy besteht;
n 3 ist;
q 0 oder 1 ist;
R⁴ aus der Gruppe ausgewählt ist, die H und C₁₋₈-Alkyl besteht;
Ring A aus der Gruppe ausgewählt ist, die aus:
i) C₃₋₈-Cycloalkyl,
ii) Aryl, das aus der Gruppe ausgewählt ist, die aus Phenyl, 1-Naphthyl oder 2-Naphthyl besteht;
iii) einem 4- bis 8-gliedrigen Heterocyclus, der aus der Gruppe ausgewählt ist, die aus einem monocyclischen gesättigten oder ungesättigten nicht-aromatischen carbocyclischen Ring oder einem kondensierten bicyclischen nicht-aromatischen carbocyclischen Ring besteht, von denen jeder 1, 2 oder 3 Heteroatome enthalten kann, die aus N, O und S ausgewählt sind; und
iv) 5- bis 6-gliedrigem Heteroaryl besteht, das aus der Gruppe ausgewählt ist, die aus einem aromatischen monocyclischen heterocyclischen Ring oder einem aromatischen kondensierten bicyclischen Ring besteht, von denen jeder 1, 2 oder 3 Heteroatome enthalten kann, die aus N, O und S ausgewählt sind;
worin Ring A unabhängig mit einem oder mehreren Substituenten substituiert sein kann, die aus der Gruppe ausgewählt sind, die aus Halogen, -OH, C₁₋₈-Alkyl, C₂₋₈-Alkenyl, C₁₋₈-Alkoxy, C₂₋₈-Alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, Nitro und Cyano besteht, worin a 0, 1 oder 2 ist; R⁶ aus der Gruppe ausgewählt ist, die aus H, C₁₋₈-Alkyl, C₁₋₈-Alkoxy und C₂₋₈-Alkenyl besteht; R⁷ und R⁸ jeweils gleich oder verschieden sind und unabhängig aus der Gruppe ausgewählt sind, die aus H, C₁₋₈-Alkyl, C₂₋₈-Alkenyl und C₃₋₈-Alkinyl besteht; R⁹ aus der Gruppe ausgewählt ist, die aus H, C₁₋₈-Alkyl und -NR⁷R⁸ besteht; und R¹⁰ C₁₋₈-Alkyl ist;
Ring B jeweils gleich oder verschieden ist und unabhängig aus der Gruppe ausgewählt ist, die aus Cyclohexyl und Phenyl besteht; und
pharmazeutisch akzeptable Salze und Solvate davon.

2. Verbindung gemäß Anspruch 1, worin X OH ist.

3. Verbindung gemäß einem der Ansprüche 1 bis 2, worin p 0 oder 1 ist.

4. Verbindung gemäß einem der Ansprüche 1 bis 2, worin p 1 ist.

5. Verbindung gemäß einem der Ansprüche 1 bis 4, worin R¹ und R² jeweils H sind.

6. Verbindung gemäß einem der Ansprüche 1 bis 5, worin Z CH ist.

7. Verbindung gemäß einem der Ansprüche 1 bis 6, worin k 0 ist.

8. Verbindung gemäß einem der Ansprüche 1 bis 7, worin q 1 ist.

9. Verbindung gemäß einem der Ansprüche 1 bis 8, worin Ring A Phenyl ist, das gegebenenfalls 1- bis 5-mal mit einem Substituenten substituiert ist, der aus der Gruppe ausgewählt ist, die aus Halogen, C₁₋₈-Alkyl, C₁₋₈-Alkoxy und S(O)ₐR⁶ besteht.

10. Verbindung gemäß einem der Ansprüche 1 bis 9, worin Ring A Phenyl ist, das gegebenenfalls 1- bis 5-mal mit einem Substituenten substituiert ist, der aus der Gruppe ausgewählt ist, die aus F, Cl, -CF₃, -OCH₃ und -OCF₃ besteht.

11. Verbindung gemäß einem der Ansprüche 1 bis 10, worin beide Ringe B Phenyl sind, das gegebenenfalls 1- bis 5-mal mit einem Substituenten substituiert ist, der aus der Gruppe ausgewählt ist, die aus Halogen, -OH, C₁₋₈-Alkyl, C₂₋₈-Alkenyl, C₁₋₈-Alkoxy, C₂₋₈-Alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, Nitro und Cyano besteht.

12. Verbindung gemäß einem der Ansprüche 1 bis 10, worin ein Ring B Phenyl ist, das gegebenenfalls 1- bis 5-mal mit einem Substituenten substituiert ist, der aus der Gruppe ausgewählt ist, die aus Halogen, -OH, C₁₋₈-Alkyl, C₂₋₈-Alkenyl, C₁₋₈-Alkoxy, C₂₋₈-Alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, Nitro und Cyano besteht, und der andere Ring B Cyclohexyl ist, das gegebenenfalls 1- bis 10-mal mit einem Substituenten substituiert ist, der aus der Gruppe ausgewählt ist, die aus Halogen, -OH, C₁₋₈-Alkyl, C₂₋₈-Alkenyl, C₁₋₈-Alkoxy, C₂₋₈-Alkenyloxy, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, Nitro und Cyano besteht.

13. Verbindung, die aus der Gruppe ausgewählt ist, die aus:
2-(3-{3-[[2-Chlor-3-(trifluormethyl)benzyl](2,2-diphenylethyl)amino]propoxy}phenyl)acetamid;
2-(3-{3-[[2-Chlor-3-(trifluormethyl)benzyl](2,2-diphenylethyl)amino]propoxy}phenyl)essigsäure;
(3-{2-[(2,2-Diphenylethyl)-(4-methoxybenzyl)amino]propoxy}phenyl)acetamid;
(3-{2-[(2,2-Diphenylethyl)-(4-methoxybenzyl)amino]propoxy}phenyl)essigsäure;
2-(3-{3-[(2,2-Diphenylethyl)-(2-fluor-4-methoxybenzyl)amino]propoxy}phenyl)acetamid;
2-(3-{3-[(2,4-Dimethoxybenzyl)-(2,2-diphenylethyl)amino]propoxy}phenyl)acetamid;
2-[3-(3-{(2,2-Diphenylethyl)-[4-fluor-2-(trifluormethyl)benzyl]amino}propoxy)phenyl]acetamid;
2-(3-{3-[(2,3-Dichlorbenzyl)-(2,2-diphenylethyl)amino]propoxy}phenyl)acetamid;
2-[3-(3-{(2,2-Diphenylethyl)-[3-(trifluormethoxy)benzyl]amino}propoxy)phenyl]acetamid;
2-(3-{3-[(2,2-Diphenylethyl)-3-fluor-4-methoxybenzyl)amino]propoxy}phenyl)acetamid;
2-(3-{3-[(2,5-Dimethoxybenzyl)-(2,2-diphenylethyl)amino]propoxy}phenyl)acetamid;
2-[3-(3-{(2,2-Diphenylethyl)-[3-(trifluormethyl)benzyl]amino}propoxy)phenyl]acetamid;
2-[3-(3-{(2,2-Diphenylethyl)-[2-fluor-3-(trifluormethyl)benzyl]amino}propoxy)phenyl]acetamid;
Ethyl-4-[{3-[3-(aminocarbonyl)phenoxy]propyl}-(2,2-diphenylethyl)amino]-1-piperidincarboxylat;
3-{3-[(1-Benzoyl-4-piperidinyl)-(2,2-diphenylethyl)amino]propoxy}benzamid;
3-{3-[(1-Acetyl-4-piperidinyl)-(2,2-diphenylethyl)amino]propoxy}benzamid;
Benzyl-4-[{3-[3-(aminocarbonyl)phenoxy]propyl}-(2,2-diphenylethyl)amino]-1-piperidincarboxylat;
3-(3-{(2,2-Diphenylethyl)-[1-(2-phenylethyl)-4-piperidinyl]amino}propoxy)benzamid;
Ethyl-4-[{3-[3-(aminocarbonyl)phenoxy]propyl}-(2-cyclhoexyl-2-phenylethyl)amino]-1-piperidincarboxylat;
3-{3-[(1-Benzoyl-4-piperidinyl)-(2-cyclohexyl-2-phenylethyl)amino]propoxy}benzamid;
3-{3-[(1-Acetyl-4-piperidinyl)-(2-cyclohexyl-2-phenylethyl)amino]propoxy}benzamid;
tert-Butyl-4-[{3-[3-(aminocarbonyl)phenoxy]propyl}-(2-cyclohexyl-2-phenylethyl)amino]-1-piperidincarboxylat;
Benzyl-4-[{3-[3-(aminocarbonyl)phenoxy]propyl}-(2-cyclohexyl-2-phenylethyl)amino]-1-piperidincarboxylat;
3-{3-[(1-Benzyl-4-piperidinyl)-(2-cyclohexyl-2-phenylethyl)amino]propoxy}benzamid;
Ethyl-4-[{3-[3-(2-amino-2-oxoethyl)phenoxy]propyl}-(2,2-diphenylethyl)amino]-1-piperidincarboxylat;
2-(3-{3-[(1-Benzoyl-4-piperidinyl)-(2,2-diphenylethyl)amino]propoxy}phenyl)acetamid;
2-(3-{3-[(1-Acetyl-4-piperidinyl)-(2,2-diphenylethyl)amino]propoxy}phenyl)acetamid;
tert-Butyl-4-[{3-[3-(2-amino-2-oxoethyl)phenoxy]propyl}-(2,2-diphenylethyl)amino]-1-piperidincarboxylat;
Benzyl-4-[{3-[3-(2-amino-2-oxoethyl)phenoxy]propyl}-(2,2-diphenylethyl)amino]-1-piperidincarboxylat;
2-[3-(3-{(2,2-Diphenylethyl)-[1-(2-phenylethyl)-4-piperidinyl]amino}propoxy)phenyl]acetamid;
2-(3-{3-[(1-Benzoyl-4-piperidinyl)-(2-cyclohexyl-2-phenylethyl)amino]propoxy}phenyl)acetamid;
2-(3-{3-[(1-Acetyl-4-piperidinyl)-(2-cyclhohexyl-2-phenylethyl)amino]propoxy}phenyl)acetamid;
Benzyl-4-[{3-[3-(2-amino-2-oxoethyl)phenoxy]propyl}-(2-cyclohexyl-2-phenylethyl)amino]-1-piperidincarboxylat;
3-{3-[(3-Cyanobenzyl)-(2,2-diphenylethyl)amino]propoxy}benzamid,
3-{3-[Cyclohexyl(2,2-diphenylethyl)amino]propoxy]-benzamid;
4-[{3-[3-(Aminocarbonyl)phenoxy]propyl}-(2,2-diphenylethyl)amino]-1-piperidincarboxamid;
3-{3-[(1,3-Benzodioxol-4-ylmethyl)-(2,2-diphenylethyl)amino]propoxy}benzamid;
3-{3-[(3,4-Dimethoxybenzyl)-(2,2-diphenylethyl)amino]propoxy}benzamid;
3-{3-[(4-Cyanobenzyl)-(2-cyclohexyl-2-phenylethyl)amino]propoxy}benzamid;
3-{3-[(4-Cyanobenzyl)-(2-cyclohexyl-2-phenylethyl)amino]propoxy}benzamid;
2-(3-{3-[Cyclohexyl(2,2-diphenylethyl)amino]-propoxy}phenyl)acetamid;
2-(3-{3-[(3,4-Dimethoxybenzyl)-(2,2-diphenylethyl)amino]propoxy}phenyl)acetamid;
3-{3-[(2-Cyclohexyl-2-phenylethyl)-(3,4-dimethoxybenzyl)amino]propoxy}benzamid;
3-{3-[(2,6-Dichlorbenzyl)-(2,2-diphenylethyl)amino]propoxy}benzamid;
3-{[{3-[3-(Aminocarbonyl)phenoxy]propyl}-(2,2-diphenylethyl)amino]methyl}benzoesäure;
4-{[{3-[3-(Aminocarbonyl)phenoxy]propyl}-(2,2-diphenylethyl)amino]methyl}benzoesäure;
3-(3-{(2,2-Diphenylethyl)-[(5-methoxy-1H-indol-3-yl)methyl]amino}propoxy)benzamid;
3-{3-[(2,2-Diphenylethyl)-(4-methoxybenzyl)amino]propoxy}benzamid;
3-{3-[[(1-Acetyl-1H-indol-3-yl)methyl]-(2,2-diphenylethyl)amino]propoxy}benzamid;
Methyl-4-{[{3-[3-(aminocarbonyl)phenoxy]propyl}-(2,2-diphenylethyl)amino]methyl}benzoat;
3-{3-[(2,3-Dihydro-1,4-benzodioxin-6-ylmethyl)-(2,2-diphenylethyl)amino]propoxy}benzamid;
3-{3-[(2,2-Diphenylethyl)-(4-pyridinylmethyl)amino]propoxy}benzamid;
2-(3-{3-[(2-Cyclohexyl-2-phenylethyl)-(3,4-difluorbenzyl)amino]propoxy}phenyl)acetamid;
2-(3-{3-(2,2-Diphenylethyl)-[[(6-chlor-1,3-benzodioxol-5-yl)methyl]amino]propoxy}phenyl]acetamid;
2-(3-{3-[(2,2-Diphenylethyl)-(cyclohexylmethyl)amino]propoxy}phenyl)acetamid;
2-(3-{3-[(2,2-Diphenylethyl)-(bicyclo[2.2.1]hept-5-en-2-ylmethyl)amino]propoxy}phenyl)acetamid;
2-(3-{3-[(2,2-Diphenylethyl)-(2,4-dimethoxy-5-pyrimidinyl)methyl)amino]propoxy}phenyl)acetamid;
2-(3-{3-[(2,2-Diphenylethyl(5-isopropyl-3-methyl-4-isoxazolyl)methyl)amino]propoxy}phenyl)acetamid;
2-(3-{3-[(2,2-Diphenylethyl)-(3,4-dihydro-2H-pyran-2-ylmethyl)amino]propoxy}phenyl)acetamid;
2-(3-{3-[(2,2-Diphenylethyl)-(4-chlor-1H-pyrazol-3-yl)methyl)amino]propoxy}phenyl)acetamid;
2-(3-{3-[(2,2-Diphenylethyl)()[(7-methoxy-1,3-benzodioxol-5-yl)methyl)amino]propoxy}phenyl)acetamid;
2-(3-{3-[(2,2-Diphenylethyl-(2,6,6-trimethyl-1-cyclohexen-1-yl)ethyl)amino]propoxy}phenyl)acetamid;
2-(3-{3-[(2,2-Diphenylethyl)-(3-cyclohexen-1-ylmethyl)amino]propoxy}phenyl)acetamid;
2-[3-(3-{(2,2-Diphenylethyl)[(2E)-3-phenyl-2-propenyl]amino}propoxy)phenyl]acetamid;
Ethyl-2-{[{3-[3-(2-amino-2-oxoethyl)phenoxy]propyl}-(2,2-diphenylethyl)amino]methyl}cyclopropancarboxylat;
2-(3-{3-[(2,2-Diphenylethyl)-(1-cyclohexen-1-ylmethyl)amino]propoxy}phenyl)acetamid;
2-(3-{3-[(2,2-Diphenylethyl)-(1H-benzimidazol-2-ylmethyl)amino]propoxy}phenyl)acetamid;
2-(3-{3-[(2,2-Diphenylethyl)-[(1,3-dimethyl-2-oxo-2,3-dihydro-1H-benzimidazol-5-yl)methyl]amino]propoxy}phenyl)acetamid und
2-(3-{3-[(2,2-Diphenylethyl)-(2-pyrrolidinylmethyl)amino]propoxy}phenyl)acetamid besteht;
und pharmazeutisch akzeptable Salze und Solvate davon.

14. 2-(3-{3-[[2-Chlor-3-(trifluormethyl)benzyl]-(2,2-diphenylethyl)amino]propoxy}phenyl)essigsäure und pharmazeutisch akzeptable Salze und Solvate davon.

15. Pharmazeutische Zusammensetzung, die eine Verbindung gemäß einem der Ansprüche 1 bis 13 und einen pharmazeutisch akzeptablen Träger oder Verdünnungsstoff umfaßt.

16. Verwendung einer Verbindung gemäß einem der Ansprüche 1 bis 13 zur Herstellung eines Medikaments zur Prävention oder Behandlung von kardiovaskulärer Krankheit.

17. Verwendung einer Verbindung gemäß Anspruch 16 zur Herstellung eines Medikaments zur Prävention oder Behandlung von Atherosklerose.

18. Verwendung einer Verbindung gemäß einem der Ansprüche 1 bis 13 zur Herstellung eines Medikaments zur Erhöhung des reversen Cholesterintransports.

19. Verwendung einer Verbindung gemäß einem der Ansprüche 1 bis 13 zur Herstellung eines Medikaments zur Hemmung der Cholesterinabsorption.

20. Verbindung gemäß einem der Ansprüche 1 bis 13 zur Verwendung in der Therapie.

## Revendications

1. Composé de formule (I) 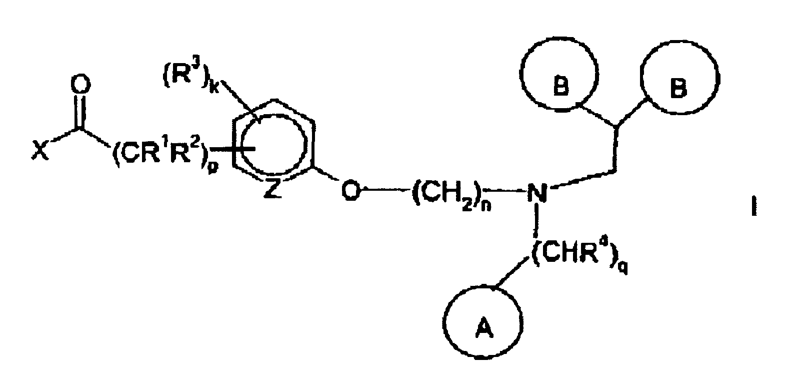 dans laquelle
X est OH ou NH² ;
p vaut de 0 à 6 ;
chaque R¹ et R² sont identiques ou différents et sont chacun indépendamment choisis à partir du groupe constitué de H, d'un groupe alkyle en C₁₋₈, alkoxy en C₁₋₈ et thioalkyle en C₁₋₈ ;
Z est CH ou N ;
lorsque Z est CH, k vaut de 0 à 4 ;
lorsque Z est N, k vaut de 0 à 3 ;
chaque R³ est identique ou différent et est choisi indépendamment à partir du groupe constitué d'un halogène et d'un groupe alkoxy en C_{1-8 ;}
n est 3 ;
q est 0 ou 1 ;
R⁴ est choisi à partir du groupe constitué de H et d'un groupe alkyle en C_{1-8 ;}
le cycle A est choisi à partir du groupe constitué de
i) un groupe cycloalkyle en C₃₋₈
ii) un groupe aryle choisi à partir du groupe constitué d'un groupe phényle, 1-naphtyle ou 2-naphtyle ;
iii) un hétérocycle de 4 à 8 éléments choisi à partir du groupe constitué d'un carbocycle monocyclique-non-aromatique saturé ou insaturé ou d'un carbocycle bicyclique fusionné non-aromatique, chacun d'eux pouvant contenir 1, 2 ou 3 hétéroatomes choisis à partir de N, O et S ; et
iv) un groupe hétéroaryle de 5 à 6 éléments choisi à partir du groupe constitué d'un hétérocycle monocyclique aromatique ou d'un cycle bicyclique aromatique fusionné chacun d'eux pouvant contenir 1, 2 ou 3 hétéroatomes choisis à partir de N, O et S ;
dans lequel le cycle A peut être substitué indépendamment par un ou plusieurs substituants choisis parmi le groupe constitué d'un halogène, d'un groupe -OH, alkyle en C₁₋₈, alcényle en C₂₋₈, alkoxy en C₁₋₈, alcényloxy en C₂₋₈, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro et cyano, dans lesquels a est 0, 1 ou 2 ; R⁶ est choisi à partir du groupe constitué de H, d'un groupe alkyle en C₁₋₈, alkoxy en C₁₋₈, alcényle en C₂₋₈ ; chaque R⁷ et R⁸ est identique ou différent et il est choisi indépendamment à partir du groupe constitué de H, d'un groupe alkyle en C₁₋₈, alcényle en C₂₋₈, alcynyle en C₃₋₈ ; R⁹ est choisi parmi le groupe constitué de H, d'un groupe alkyle en C₁₋₈ et de -NR⁷R⁸ ; et R¹⁰ est un groupe alkyle en C_{1-8 ;}
chaque cycle B est identique ou différent et il est choisi indépendamment à partir du groupe constitué d'un groupe cyclohexyle et d'un groupe phényle ; et des sels et des solvates pharmaceutiquement acceptables de celui-ci.

2. Composé selon la revendication 1, dans lequel X est OH.

3. Composé selon l'une quelconque des revendications 1 et 2, dans lequel p est 0 ou 1

4. Composé selon l'une quelconque des revendications 1 et 2, dans lequel p est 1.

5. Composé selon l'une quelconque des revendications 1 à 4, dans lequel R¹ et R² sont chacun H.

6. Composé selon l'une quelconque des revendications 1 à 5, dans lequel Z est CH.

7. Composé selon l'une quelconque des revendications 1 à 6, dans lequel k est 0.

8. Composé selon l'une quelconque des revendication 1 à 7, dans lequel q est 1.

9. Composé selon l'une quelconque des revendications 1 à 8, dans lequel le cycle A est un groupe phényle éventuellement substitué de 1 à 5 fois par un substituant choisi à partir du groupe constitué d'un halogène, d'un groupe alkyle en C₁₋₈, alkoxy en C₁₋₈, et S(O)ₐR⁶.

10. Composé selon l'une quelconque des revendications 1 à 9, dans lequel le cycle A est un groupe phényle éventuellement substitué de 1 à 5 fois par un substituant choisi à partir du groupe constitué de F, Cl, -CF³, -OCH³, et -OCF³.

11. Composé selon l'une quelconque des revendications 1 à 10, dans lequel les deux cycles B sont des groupes phényles éventuellement substitués de 1 à 5 fois par un substituant choisi à partir du groupe constitué d'un halogène, d'un groupe -OH, alkyle en C₁₋₈, alcényle en C₂₋₈, alkoxy en C₁₋₈, alcényloxy en C₂₋₈, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, -R¹⁰NR⁷R⁸, -OR¹⁰NR⁷R⁸, nitro et cyano.

12. Composé selon l'une quelconque des revendications 1 à 10, dans lequel un cycle B est un groupe phényle éventuellement substitué de 1 à 5 fois par un substituant choisi à partir du groupe constitué d'un halogène, d'un groupe -OH, alkyle en C₁₋₈, alcényle en C₂₋₈, alkoxy en C₁₋₈, alcényloxy en C₂₋₈, S(O)ₐR⁶, -NR⁷R⁸, -COR⁶, -COOR⁶, -R¹⁰COOR⁶ , -OR¹⁰COOR⁶, -CONR⁷R⁸, -OC(O)R⁹, -R¹⁰NR⁷R⁸, -OR¹⁰NR⁷R⁸, nitro et cyano et l'autre cycle B est un groupe cyclohexyle éventuellement substitué de 1 à 10 fois par un substituant choisi à partir du groupe constitué d'un halogène, d'un groupe -OH, alkyle en C₁₋₈, alcényle en C₂₋₈, alkoxy en C₁₋₈, alcényloxy en C₂₋₈, S(O)ₐR⁶, -NR⁷R⁸, -COR⁶, -COOR⁶, -R¹⁰COOR⁶, -OR¹⁰COOR⁶, -CONR⁷R⁸, -OC(O)R⁹, - R¹⁰NR⁷R⁸, -OR¹⁰NR⁷R⁸, nitro et cyano.

13. Composé choisi à partir du groupe constitué de :
2-(3-{3-[[2-chloro-3-(trifluorométhyl)benzyl]-(2,2-diphényléthyl) amino]propoxy}phényl)acétamide,
acide 2-(3-{3-[[2-chloro-3-(trifluorométhyl)benzyl]- (2,2-diphényléthyl amino]propoxy}-phényl)acétique,
(3-{2-[(2,2-diphényléthyl)-(4-méthoxybenzyl)amino]-propoxy}phényl)acétamide,
acide (3-{2-[(2,2-diphényléthyl}(4-méthoxybenzyl)-amino]propoxy}phényl)acétique,
2-(3-{3-((2,2-diphényléthyl)(2-fluoro-4-méthoxybenzyl)- amino]propoxy}phényl)acétamide,
2-(3-{3-[(2,4-diméthoxybenzyl)(2,2-diphényléthyl)-amino]propoxy}phényl)acétamide,
2-(3-(3-{(2,2-diphényléthyl)[4-fluoro-2-(trifluorométhyl)benzyl]amino}propoxy)phényl] acétamide,
2-(3-{3-[(2,3-dichlorobenzyl)(2,2-diphényléthyl)amino]-propoxy}phényl)acétamide,
2-[3-(3-{(2,2-diphényléthyl)[3-(trifluorométhoxy)-benzyl]amino}propoxy)phényl]acétamide,
2-(3-{3-((2,2-diphényléthyl)(3-fluoro-4-méthoxybenzyl)amino]propoxy}phényl)acétamide,
2-(3-{3-[(2,5-diméthoxybenzyl)(2,2-diphényléthyl)-amino]propoxy}phényl)acétamide,
2-[3-(3-{(2,2-diphényléthyl)[3-trifluorométhyl)benzyl]-amino}propoxy)phényl]acétamide,
2-[3-(3-{(2,2-diphényléthyl)[2-fluoro-3-(trifluorométhyl)benzyl]amino}propoxy)phényl]-acétamide,
éthyle 4-[{3-[3-(aminocarbonyl)phénoxy]propyl}(2,2-diphényléthyl)amino]-1-pipéridinecarboxylate,
3-{3-[(1-benzoyl-4-pipéridinyl)-(2,2-diphényléthyl)- amino]propoxy}benzamide;
3-{3-[(1-acétyl-4-pipéridinyl)(2,2-diphényléthyl)-amino}propoxy}benzamide,
benzyle 4-[{3-[3-(aminocarbonyl)phénoxy]propyl}(2,2-diphényléthyl)amino]-1-pipéridinecarboxylate,
3-(3-{(2,2-diphényléthyl)[1-(2-phényléthyl)-4-pipéridinyl]amino}propoxy)-benzamide,
éthyle 4-[{3-[3-(aminocarbonyl)phénoxy]propyl}(2-cyclohexyl-2-phényléthyl)amino]-1-pipéridine-carboxylate,
3-{3-[(1-benzoyl-4-pipéridinyl)(2-cyclohexyl-2-phényléthyl)amino]propoxy}-benzamide,
3-{3-((1-acétyl-4-pipéridinyl)(2-cyclohexyl-2-phényléthyl)amino] propoxy}benzamide,
tert-butyl 4-[{3-[3-(aminocarbonyl)phénoxy)propyl}-(2-cyclohexyl-2-phényléthyl)amino]-1-pipéridine-carboxylate,
benzyle 4-[{3-[3-(aminocarbonyl)phénoxy]propyl}(2-cyclohexyl-2-phénytéthyl)amino]-1-pipéridine-carboxytate,
3-{3-[(1-benzyl-4-pipéridinyl)(2-cyclohexyl-2-phényléthyl)amino] propoxy}-benzamide,
éthyle 4-[{3-{3-(2-amino-2-oxoéthyl)phénoxy]propyl}-{2,2-diphényléthyl)amino]-1-pipéridinecarboxylate,
2-(3-{3-[(1-Benzoyl-4-pipéridinyl)(2,2-diphényléthyl}amino]propoxy}phényl)acétamide,
2-(3-{3-((1-acétyl-4-pipéridinyl)(2,2-diphényléthyl)-amino]propoxy}phényl)-acétamide,
tert-butyl 4[{3-[3-(2-amino-2-oxoéthyl)phénoxy]propyl}-(2,2-diphényléthyl)amino]-1-pipéridinecarboxylate,
benzyle 4-[{3-[3-(2-amino-2-oxoéthyl)phénoxy]propyl}-(2,2-diphényléthyl)amino]-1-péridinecarboxytate,
2-(3-(3-{(2,2-diphényléthyl)[1-(2-phényléthyl)-4-pipéridinyl]amino}propoxy)phényl]-acétamide,
2-(3-{3-[(1-benzoyl-4-pipéridinyl)(2-cyclohexyl-2-phényléthyl)amino]propoxy}-phényl}acétamide,
2-(3-{3-[(1-acétyl-4-pipéridinyl)(2-cyclohexyl-2-phényléthyl)amino]propoxy}phényl)acétamide,
benzyl-4-[{3-[3-(2-amino-2-oxoéthyl)phénoxy]propyl}- (2-cyclohexyl-2-phényléthyl)amino]-1-pipéridine-carboxylate,
3-{3-[{3-cyanobenzyl)(2,2-diphényléthyl)amino]propoxy} benzamide,
3-{3-[cyclohexyl(2,2-diphényléthyl)amino]-propoxy}benzamide,
4-[{3-[3-(aminocarbonyl)phénoxy]propyl}(2,2-diphényléthyl)amino]-1-pipéridinecarboxamide,
3-{3-[(1,3-benzodioxol-4-ylméthyl)(2,2-- diphényléthyl) amino]propoxy}benzamide,
3-{3-[(3,4-diméthoxybenzyl)(2,2-diphényléthyl)amino]-propoxy}benzamide,
3-{3-[(4-cyanobenzyl)(2-cyclohexyl-2-phényléthyl)-amino]propoxy}benzamide,
3-{3-[(4-cyanobenzyl)(2-cyclohexyl-2-phényléthyl)-amino]propoxy}benzamide,
2-(3-{3-[cyclohexyl(2,2-diphényléthyl)amino]propoxy}-phényl)acétamide,
2-(3-{3-[(3,4-diméthoxybenzyl)(2,2-diphényléthyl)-amino]propoxy}phényl)acétamide,
3-{3-[(2-cyclohexyl-2-phényléthyl)(3,4-diméthoxybenzyl)amino]propoxy}benzamide,
3-{3-[(2,6-dichlorobenzyl) (2,2-diphényléthyl)amino]-propoxy}benzamide,
acide 3-{[{3-[3-(aminocarbonyl)phénoxy]propyl}(2,2-diphényl-éthyl)amino]méthyl}benzoïque,
acide 4-{[{3-[3-(aminocarbonyl)phénoxy]propyl}(2,2-diphényléthyl)amino]méthyl}benzoïque,
3-(3-{(2,2-diphényléthyl)[(5-méthoxy-1H-indol-3-yl)-méthyl]amino}propoxy)-benzamide,
3-{3-[(2,2-diphényléthyl)(4-méthoxybenzyl)amino]-propoxy}benzamide,
3-{3-[[(1-acétyl-1H-indol-3-yl)méthyl](2,2-diphényl-éthyl)amino]propoxy}benzamide,
méthyle 4-{[{3-[3-(aminocarbonyl)phénoxy]propyl}(2,2-diphényléthyl)amino]méthyl}benzoate,
3-{3-[(2,3-dihydro-1,4-benzodioxin-6-ylméthyl)-(2,2-diphényléthyl)amino]propoxy}benzamide,
3-{3-[(2,2-diphényléthyl)(4-pyridinylméthyl)amino]-propoxy}benzamide,
2-(3-{3-[(2-cyclohexyl-2-phényléthyl)(3,4-difluorobenzyl)amino]propoxy}phényl)acétamide;
2-(3{3-(2,2-diphényléthyl)[[(6-chloro-1,3-benzodioxol-5-yl)méthyl]amino]propoxy}phényl)acétamide,
2-(3-{3-[(2,2-diphényléthyl)(cyclohexylméthyl)-amino]propoxy}phényl)acétamide,
2-(3-{3-[(2,2-diphényléthyl)(bicyclo[2.2.1]hept-5-èn-2-ylméthyl)amino]propoxy}-phényl)acétamide,
2-(3-{3-[(2,2-diphényléthyl)(2,4-diméthoxy-5-pyrimidinyl)méthyl)amino]propoxy}phényl)acétamide,
2-(3-{3-[(2,2-diphényléthyl(5-isopropyl-3-méthyl-4-isoxazolyl)méthyl)amino]propoxy}phényl) acétamide,
2-(3-{3-[(2,2-diphényléthyl)(3,4-dihydro-2H-pyran-2-ylméthyl)amino]propoxy}phényl)acétamide,
2-(3-{3-[(2,2-diphényléthyl)(4-chloro-1H-pyrazol-3-yl)méthyl)amino]propoxy}-phényl)acétamide,
2-(3-{3-[(2,2-diphényléthyl)( )[(7-méthoxy-1,3-benzo-dioxol-5-yl)méthyl)amino]-propoxy}phényl)acétamide,
2-(3-{3-[(2,2-diphényléthyl-(2,6,6-triméthyl-1-cyclohexèn-1-yl)éthyl)amino]propoxy}phényl)acétamide,
2-(3-{3-[(2,2-diphényléthyl)(3-cyclohexèn-1-ylméthyl)-amino]propoxy}phényl)acétamide,
2-[3-(3-{(2,2-diphényléthyl)[(2E)-3-phényl-2-propényl]-amino}propoxy)phényl]acétamide,
éthyle 2-{[{3-[3-(2-amino-2-oxoéthyl)phénoxy]propyl}(2,2-diphényléthyl)amino}méthyl]cyclopropanecarboxylate,
2{3-{3-[(2,2-diphényléthyl)(1-cyclohexèn-1-ylméthyl)-amino)propoxy}phényl)acétamide,
2-(3-{3-[(2,2-diphényléthyl)(1H-benzimidazol-2-ylméthyl)amino]propoxy}phényl)acétamide,
2-(3-{3-[(2,2-diphényléthyl)[(1,3-diméthyl-2-oxo-2,3-dihydro-1H-benzimidazol-5-yl)méthyl]amino]-propoxy}phényl)acétamide, et
2-(3-{3-[(2,2-diphényléthyl)(2-pyrrolidinylméthyl)-amino]propoxy}phényl)acétamide,
et les sels et solvates pharmaceutiquement acceptables de ceux-ci.

14. Acide 2-(3-{3-[[2-chloro-3-(trifluorométhyl)-benzyl](2,2diphényléthyl)amino]propoxy}phényl)acétique et les sels et solvates pharmaceutiquement acceptables de celui-ci.

15. Composition pharmaceutique comprenant un composé selon l'une quelconque des revendications 1 à 13 et un vecteur ou diluant pharmaceutiquement acceptable.

16. Utilisation d'un composé selon l'une quelconque des revendications 1 à 13 pour la préparation d'un médicament pour la prévention ou le traitement d'une maladie cardiovasculaire.

17. Utilisation d'un composé selon la revendication 16 pour la préparation d'un médicament pour la prévention ou le traitement de l'artériosclérose.

18. Utilisation d'un composé selon l'une quelconque des revendications 1 à 13 pour la préparation d'un médicament pour augmenter le transport inverse du cholestérol.

19. Utilisation d'un composé selon l'une quelconque des revendications 1 à 13 pour la préparation d'un médicament inhibant l'absorption du cholestérol.

20. Composé selon l'une quelconque des revendications 1 à 13 pour une utilisation en thérapie.


### SEQUENCE LISTING

<110> Glaxo Group Limited
<120> Chemical Compounds
<130> PU4191
<140> to be assigned
   <141> 2001-09-06
<150> 60/233,144
   <151> 2000-09-18
<160> 5
<170> FastSEQ for Windows Version 4.0
<210> 1
   <211> 11
   <212> PRT
   <213> Artificial Sequence
<220>
   <223> modified polyhistidine tag
<400> 1
   Met Lys Lys Gly His His His His His His Gly
   1 5 10
<210> 2
   <211> 25
   <212> PRT
   <213> Artificial Sequence
<220>
   <223> biotinylated peptide comprising amino acids 675-699 of SRC-1
<400> 2
   Cys Pro Ser Ser His Ser Ser Leu Thr Glu Arg His Lys Ile Leu His
   1 5 10 15
   Arg Leu Leu Gln Glu Gly Ser Pro Ser
   20 25
<210> 3
   <211> 24
   <212> DNA
   <213> Artificial Sequence
<220>
   <223> mouse ABC1 (X75926) forward primer
<400> 3
   aagggtttct ttgctcagat tgtc 24
<210> 4
   <211> 17
   <212> DNA
   <213> Artificial Sequence
<220>
   <223> mouse ABC1 (X75926) reverse primer
<400> 4
   tgccaaaggg tggcaca 17
<210> 5
   <211> 25
   <212> DNA
   <213> Artificial Sequence
<220>
   <223> mouse ABC1 (X75926) probe oligo
<400> 5
   ccagctgtct ttgtttgcat tgccc 25